(19) Europäisches Patentamt
European Patent Office
Office européen des brevets

(11) EP 4 379 036 A1

(12) # EUROPEAN PATENT APPLICATION
published in accordance with Art. 153(4) EPC

(43) Date of publication:
**05.06.2024 Bulletin 2024/23**

(21) Application number: **22849127.0**

(22) Date of filing: **28.06.2022**

(51) International Patent Classification (IPC):
***C12M 1/00*** *(2006.01)* ***C12M 1/10*** *(2006.01)*
***C12M 1/26*** *(2006.01)* ***C12N 1/02*** *(2006.01)*
***C12N 11/02*** *(2006.01)*

(52) Cooperative Patent Classification (CPC):
**C12M 1/00; C12M 1/10; C12M 1/26; C12N 1/02; C12N 11/02**

(86) International application number:
**PCT/JP2022/025814**

(87) International publication number:
**WO 2023/008057 (02.02.2023 Gazette 2023/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.07.2021 JP 2021123678**
**07.03.2022 JP 2022034382**
**09.05.2022 JP 2022076878**

(71) Applicant: **PHC Holdings Corporation**
**Tokyo 105-8433 (JP)**

(72) Inventors:
- **NISHIO, Akira**
  **Toon-shi, Ehime 791-0395 (JP)**
- **MATSUBARA, Fumiya**
  **Toon-shi, Ehime 791-0395 (JP)**
- **TANIMOTO, Yasushi**
  **Toon-shi, Ehime 791-0395 (JP)**
- **JOHNO, Masahiro**
  **Toon-shi, Ehime 791-0395 (JP)**

(74) Representative: **Novagraaf International SA**
**Chemin de l'Echo 3**
**1213 Onex, Geneva (CH)**

(54) **CELL CAPTURE DEVICE, CENTRIFUGAL CONTAINER, CELL CAPTURE APPARATUS, AND CELL CAPTURE METHOD**

(57) A cell capture apparatus (101-105) comprises a centrifugal container (110, 110', 120) that can be mounted in a centrifuge (201, 202), and that has a space (110s) in which a test liquid containing target cells is held, and an inner surface (113) that defines the space (110s); and a cell adhesion layer (111) that is disposed on the inner surface (113) and adsorbs the target cells (150, 150A, 150B) in the test liquid.

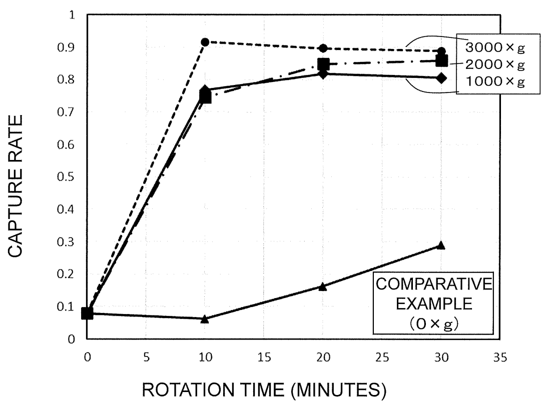


FIG. 17

EP 4 379 036 A1

## Description

### TECHNICAL FIELD

The present application relates to a cell capture device, a centrifugal container, a cell capture apparatus, and a cell capture method.

### BACKGROUND ART

There have been studies in recent years in which various kinds of cell other than blood cell components and plasma components contained in intravascular fluid such as blood or lymphatic fluid are detected and separated, and these are used for the prevention and treatment of disease.

Among various cells, tumor cells, for example, are released into the blood from primary tumor tissue or metastatic tumor tissue via EMT (epithelial-mesenchymal transformation), and circulate in the bloodstream as circulating tumor cells (CTC). Therefore, the progression and metastasis of cancer can be predicted by measuring the amount of circulating tumor cells in the blood.

Here, circulating tumor cells or other such cells that are to be measured can be separated and detected without being damaged, which allows the cells to proliferate and information about those cells to be analyzed.

For instance, Patent Literature 1 discloses a cell separation method in which cells in a test liquid are adsorbed and separated using a wettable composition containing intermediate water.

### CITATION LIST

### PATENT LITERATURE

Patent Literature 1: Japanese Patent No. 6,474,540

### SUMMARY

However, the following problem is encountered with the conventional cell separation method described above.

With the cell separation method disclosed in the above document, it was difficult to efficiently capture target cells, such as circulating tumor cells, which are present in only trace amounts in the blood.

### (TECHNICAL PROBLEM)

It is an object of the present invention to provide a cell capture device, a centrifugal container, a cell capture apparatus, and a cell capture method with which target cells can be captured efficiently.

### (SOLUTION TO PROBLEM)

The cell capture device according to an embodiment of the present disclosure comprises a centrifugal container that can be mounted in a centrifuge, and that has a space in which a test liquid containing target cells is held, and an inner surface that defines the space; and a cell adhesion layer that is disposed on the inner surface and includes a first biocompatible polymeric material capable of containing intermediate water.

The centrifugal container according to the present invention is a centrifugal container that can be mounted in a centrifuge, and that comprises an inner surface and a cell adhesion layer. The inner surface defines a space for holding a test liquid containing the target cells. The cell adhesion layer is disposed in a capture region of the inner surface that is substantially parallel to the centrifugal direction in which centrifugal force is applied by the centrifuge, and adsorbs the target cells in the test liquid.

The centrifugal container according to the present invention is a centrifugal container that is mounted in a centrifuge and is rotationally driven while the orientation is changed as necessary in each step, and comprises a main body, an inner surface, and a cell adhesion layer. The inner surface is provided inside the main body and forms a holding space for holding a test liquid containing target cells and contaminants other than the target cells. The cell adhesion layer is provided to a part of the inner surface in order to adsorb the target cells in the test liquid, and is disposed substantially vertically with respect to the direction of the centrifugal force applied by the rotation of the centrifuge when capturing the target cells contained in the test liquid poured into the holding space, and is disposed substantially horizontally with respect to the centrifugal force when removing the contaminants.

(EFFECTS)

The present invention allows target cells to be captured efficiently.

BRIEF DESCRIPTION OF THE DRAWINGS

Fig. 1 is an oblique view of the appearance of the cell capture apparatus according to a first embodiment;
Fig. 2 is an oblique view of a cell capture device of the cell capture apparatus shown in Fig. 1;
Fig. 3 is a cross-sectional view of the cell capture device of the cell capture apparatus shown in Fig. 1;
Fig. 4 is an oblique view showing a state in which the cell device is rotated and centrifugal force is applied in the cell capture apparatus according to the first embodiment;
Fig. 5 is a schematic diagram showing the hydration structure of a biocompatible polymeric material and the hydration shell of a target cell;
Fig. 6 is a schematic diagram illustrating how centrifugal force acts on target cells in a test liquid;
Fig. 7 is a schematic diagram illustrating the effect of centrifugal force on the adsorption of target cells to the cell adhesion layer;
Fig. 8 is a schematic diagram illustrating the effect of centrifugal force on the adsorption of target cells to the cell adhesion layer.
Fig. 9 is a schematic diagram illustrating the effect of centrifugal force on the adsorption of target cells to the cell adhesion layer;
Fig. 10 is a cross-sectional view of another mode of the cell capture device;
Fig. 11 is a cross-sectional view showing another mode of the cell capture device;
Fig. 12 is a cross-sectional view showing another mode of the cell capture device;
Fig. 13 is an oblique view of the appearance of the cell capture apparatus according to a second embodiment;
Fig. 14 is an exploded oblique view of the cell capture device of the cell capture apparatus shown in Fig. 13;
Fig. 15 is a cross-sectional view of the cell capture device of the cell capture apparatus shown in Fig. 13;
Fig. 16 is a flowchart showing an embodiment of a cell capture method;
Fig. 17 is a graph of the results of an experimental example, and shows the relation between how long the centrifugal force is applied and the capture rate;
Fig. 18 is an overall oblique view of the cell capture apparatus, and shows the state before the centrifugal container according to an embodiment of the present invention is mounted in a centrifuge;
Fig. 19 is an overall oblique view of the cell capture apparatus, and shows a state in which the centrifugal container of Fig. 18 is has been mounted in the centrifuge;
Fig. 20 is a schematic diagram of the hydration structure of a target cell and a biocompatible polymeric material;
Fig. 21A is a schematic diagram of target cells and contaminants contained in a test liquid placed in a centrifugal container, and Fig. 21B is a schematic diagram of a state in which the target cells and contaminants are subjected to centrifugal force when the centrifugal container is rotated by the centrifuge.
Fig. 22 is an exploded oblique view showing the configuration of the centrifugal container in Fig. 18, etc.;
Fig. 23 is a cross-sectional view of the centrifugal container in Fig. 22;
Fig. 24 is a plan view of the centrifugal container in Fig. 22;
Fig. 25A is a plan view of a state in which a test liquid containing target cells, etc., has been placed in the centrifugal container in Fig. 24, and Fig. 25B is a cross-sectional view along the A-A line in Fig. 25A;
Figs. 26A and 26B are diagrams of a state in which the target cells, etc., in Fig. 25A have sunken in the test liquid, and Figs. 26C and 26D are diagrams of the movement of the target cells, etc., when the centrifugal container is rotated around the rotation axis;
Fig. 27A is a plan view of the movement of contaminants other than the target cells when the centrifugal container is rotated around the rotation axis, and Fig. 27B is a cross-sectional view along the B-B line in Fig. 27A;
Fig. 28A is a plan view of the movement of contaminants, etc., other than the target cells when the centrifugal container is further rotated around the rotation axis, and Fig. 28B is a cross-sectional view along the C-C line in Fig. 28A;
Fig. 29 is a flowchart showing the flow of processing in a cell capture method performed by the cell capture apparatus in Fig. 18;
Fig. 30 is an exploded oblique view of the configuration of the centrifugal container according to another embodiment of the present invention;
Fig. 31A is a plan view of the centrifugal container in Fig. 30, and Fig. 31B is a cross-sectional view along the D-D line in Fig. 31A;

Fig. 32 is an exploded oblique view of the configuration of the centrifugal container according to yet another embodiment of the present invention;

Fig. 33A is a plan view of the centrifugal container in Fig. 32, and Fig. 33B is a cross-sectional view along the E-E line in Fig. 33A;

Fig. 34 is a cross-sectional view of the force exerted on contaminants, etc., that move along the curved surface when the centrifugal container shown in Fig. 33B is rotated around the rotation axis;

Fig. 35 is an exploded oblique view of the configuration of the centrifugal container according to yet another embodiment of the present invention;

Fig. 36A is a plan view of the centrifugal container in Fig. 35, and Fig. 36B is a cross-sectional view along the F-F line in Fig. 36A;

Fig. 37 is an exploded oblique view of the configuration of the centrifugal container according to yet another embodiment of the present invention;

Fig. 38A is a plan view of the centrifugal container in Fig. 37, and Fig. 38B is a cross-sectional view along the G-G line in Fig. 38A;

Fig.39 is an exploded oblique view of the configuration of the centrifugal container according to yet another embodiment of the present invention;

Fig. 40A is a plan view of the centrifugal container in Fig. 39, and Fig. 40B is a cross-sectional view along the H-H line in Fig. 40A;

Figs. 41A and 41B are cross-sectional views of the configuration of the centrifugal container according to yet another embodiment of the present invention;

Fig. 42 is an oblique view of a state in which the centrifugal container according to yet another embodiment of the present invention is mounted in a centrifuge;

Fig. 43 is an oblique view of the configuration of the centrifugal container in Fig. 42;

Fig. 44A is a plan view of the centrifugal container in Fig. 43, and Fig. 44B is a cross-sectional view along the I-I line in Fig. 44A;

Fig. 45 is an oblique view of the configuration of the cell capture apparatus when the centrifugal container according to an embodiment of the present invention is mounted in a centrifuge;

Fig. 46 is an oblique view of the configuration of the cell capture apparatus in which the centrifugal container in Fig. 45 has been mounted in a centrifuge;

Fig. 47 is a schematic diagram of the hydration structure of a target cell and a biocompatible polymeric material;

Fig. 48 is an oblique view of the configuration of the centrifugal container in Fig. 45;

Fig. 49A is a plan view of the centrifugal container in Fig. 48, Fig. 49B is a cross-sectional view along the A-A line in Fig. 49A, and Fig. 49C is a cross-sectional view along B-B line in Fig. 49A;

Fig. 50A is a cross-sectional view of the state before rotation of the centrifuge shown in Fig. 45, or a state in which the centrifuge is spinning at a rotation speed at which a centrifugal force less than a specific value is being applied, and Fig. 50B is a cross-sectional view of a state in which the orientation of the centrifugal container has changed when the rotation speed is increased from the state in Fig. 50A and centrifugal force of a specific value or more is applied;

Fig. 51A is a cross-sectional view of a state in which a sample has been poured into the pouring chamber of the centrifugal container in Fig. 47, and Fig. 51B is a cross-sectional view of a state in which the centrifuge is rotated at a rotation speed V1 from the state in Fig. 51A, centrifugal force is applied to the sample in the centrifugal container, and the liquid is transferred from the pouring chamber into the capture chamber;

Fig. 52A is a cross-sectional view of a state in which the liquid transfer step in Fig. 51B has been completed, and Fig. 52B is a cross-sectional view of the step of rotating the centrifuge at the rotation speed V2 from the state in Fig. 52A to apply centrifugal force to the target cells in the capture chamber and press them against the cell adhesion layer for capture;

Figs. 53A and 53B are cross-sectional views of a state in which the rotation speed is further increased from the state in Fig. 52B to V3 to increase the centrifugal force, and the orientation of the centrifugal container is changed to be substantially horizontal;

Fig. 54 is a cross-sectional view of a rinsing step in which the centrifuge is rotated at a rotation speed of V4 from the state in Fig. 53B to leave only the target cells in the cell adhesion layer and move contaminants outward in the centrifugal direction;

Fig. 55 is a graph of the relation between elapsed time and rotation speed in each of the steps of pumping, capturing, changing direction, and rinsing;

Fig. 56A is a cross-sectional view of the target cells and contaminants contained in a sample pumped into the capture chamber, and Fig. 56B is a cross-sectional view of processing in which the centrifuge is rotated at a rotation speed V2 from the state in Fig. 56A to apply centrifugal force to the target cells, etc., and press the target cells against the cell adhesion layer for capture;

Fig. 57A is a cross-sectional view of processing in which the centrifuge is rotated from the state in Fig. 56B at a rotation speed V3 and the orientation of the centrifugal container is changed from vertical to horizontal, and Fig. 57B is a cross-sectional view of a rinsing step in which the centrifuge is further rotated from the state in Fig. 57A at a rotation speed V4 to leave only the target cells in the cell adhesion layer and move contaminants outward in the centrifugal direction;

Fig. 58A is a cross-sectional view of a state in which air is mixed with the sample in the capture chamber of the centrifugal container, and Fig. 58B is a cross-sectional view of a state in which the centrifuge is rotated from the state in Fig. 58A to apply centrifugal force, and as a result, when the air moves inward in the centrifugal direction, the target cells are pulled away from the cell adhesion layer and move outward in the centrifugal direction;

Fig. 59 is a flowchart showing the flow of processing in a cell capture method performed by the cell capture apparatus including the centrifugal container and the centrifuge in Fig. 45;

Figure 60 is an oblique view of the configuration of the centrifugal container according to yet another embodiment of this invention;

Fig. 61A is a plan view of the centrifugal container in Fig. 60, Fig. 61B is a cross-sectional view along the C-C line in Fig. 61A, and Fig. 61C is a cross-sectional view along the D-D line in Fig. 61A;

Fig. 62 is an oblique view of the configuration of the centrifugal container according to yet another embodiment of the present invention;

Fig. 63A is a plan view of the centrifugal container in Fig. 62, Fig. 63B is a cross-sectional view along the E-E line in Fig. 63A, and Fig. 63C is a cross-sectional view along the F-F line in Fig. 63A;

Fig. 64 is an oblique view of the configuration of the centrifugal container according to yet another embodiment of the present invention;

Fig. 65A is a plan view of the centrifugal container in Fig. 64, Fig. 65B is a cross-sectional view along the G-G line in Fig. 65A, and Fig. 65C is a cross-sectional view along the H-H line in Fig. 65A; and

Figs. 66A and 66B are oblique views of the configuration of a rotor of a centrifuge according to yet another embodiment of the present invention.

DESCRIPTION OF THE EMBODIMENTS

Biological cells are believed to be surrounded by hydration shells composed of water molecules in various states, and it is believed to be preferable for a structure to be captured also to have a similar hydration structure in order to capture biological cells in a state in which there is no damage or major structural change. Nevertheless, the interaction between the hydration structure of such structures and the hydration structure of biological cells may be weak, making it difficult to capture cells efficiently. The inventors of the present application have come up with a cell capture device, a cell capture apparatus, and a cell capture method with which cells can be efficiently captured by utilizing centrifugal force to capture cells. The cell capture device, cell capture apparatus, and cell capture method in this embodiment are as follows.

[Item 1]

A cell capture device, comprising:

a centrifugal container that can be mounted in a centrifuge, and that has a space in which a test liquid containing target cells is held, and an inner surface that defines the space; and

a cell adhesion layer that is disposed on the inner surface and adsorbs the target cells in the test liquid.

[Item 2]

The cell capture device according to item 1, wherein the cell adhesion layer is disposed in a capture region of the inner surface that is not parallel to the centrifugal direction of the centrifugal container.

[Item 3]

The cell capture device according to item 2, further comprising a cell adhesion suppression layer that is disposed in a region of the inner surface other than the capture region, and that suppresses adsorption of the target cells in the test liquid.

[Item 4]

The cell capture device according to any of items 1 to 3, wherein the target cells include at least one type selected from the group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells, and the cell adhesion layer adsorbs the target cells.

[Item 5]

The cell capture device according to any of claims 1 to 4, wherein the cell adhesion layer includes a first biocompatible polymeric material, and
the first biocompatible polymeric material includes one or more of the polymers expressed by the following formulas 1 to 4, or a copolymer of a monomer constituting the polymer expressed by formula 1 and a monomer constituting one or more types of polymer selected from the group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

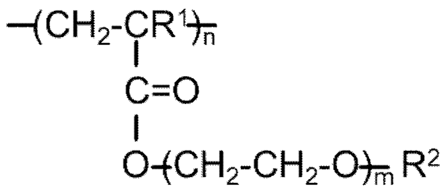

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

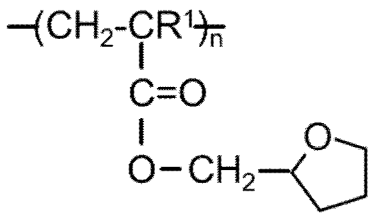

(2)

(In formula 2, $R^1$ is a hydrogen atom or a methyl group, and n is a repeating unit.)

Chemical Formula 3

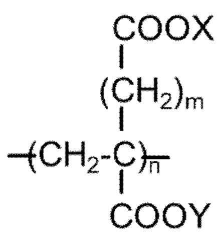

(3)

(In formula 3, X and Y are each independently a hydrogen atom, an alkali metal atom, or an ammonium group, m is an integer of 0, 1, or 2, and n is a repeating unit.)

Chemical Formula 4

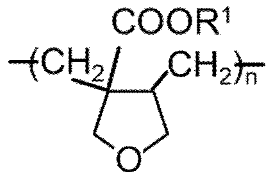

(4)

(In formula 4, $R^1$ is a hydrogen atom or an organic group having 1 to 30 carbon atoms, and n is a repeating unit.)

[Item 6]

The cell capture device according to item 3, wherein the cell adhesion suppression layer includes a second biocompatible polymeric material, and
the second biocompatible polymeric material includes a polymer having phospholipid polar groups.

[Item 7]

The cell capture device according to item 3, wherein the centrifugal container has a cylindrical shape having the space,

the space has a columnar shape,
the inner surface includes a side surface portion that defines the side portion of the columnar shape, and a bottom surface portion that defines the bottom portion of the columnar shape, and
the cell adhesion layer covers the bottom surface portion of the columnar inner surface.

[Item 8]

The cell capture device according to item 7, wherein the bottom surface portion is partially spherical.

[Item 9]

The cell capture device according to item 8, wherein the cell adhesion layer is disposed on an arc with respect to the center of rotation.

[Item 10]

The cell capture device according to any of items 7 to 9, wherein the cell adhesion suppression layer covers the side surface portion of the columnar inner surface.

[Item 11]

The cell capture device according to item 3, wherein the centrifugal container has a disk shape having an upper surface, a lower surface, and a rotary shaft,

the space is formed at a position that is away from the rotary shaft of the disk shape in the radial direction,
the inner surface includes an outer side surface portion that is farthest away from the rotary shaft in the radial direction, and
the cell adhesion layer is disposed on the outer side surface portion.

[Item 12]

The cell capture device according to item 11, wherein the cell adhesion suppression layer covers the surface of the inner surface other than the outer side surface portion.

[Item 13]

A cell capture apparatus, comprising:

the cell capture device according to any of items 1 to 12; and
a centrifuge in which the cell capture device can be mounted, and that applies centrifugal force to the cell capture device by rotating the cell capture device.

[Item 14]

A cell capture method, comprising:

a step of introducing a test liquid containing target cells into a space of a cell capture device comprising a space for holding a test liquid containing target cells, a centrifugal container having an inner surface that defines the space, and a cell adhesion layer including a first biocompatible polymeric material that is disposed on the inner surface and adsorbs the target cells in the test liquid; and
a step of mounting the cell capture device in a centrifuge and rotating the cell capture device to adsorb the target cells to the cell adhesion layer.

[Item 15]

The cell capture method according to item 14, wherein, in the adsorption step, a centrifugal force of at least 500 G and no more than 10,000 G is applied to the test liquid held in the cell capture device.

[Item 16]

The cell capture method according to item 14 or 15, further comprising a step of bringing a liquid containing water into contact with the cell adhesion layer prior to the introduction step.

[Item 17]

The cell capture method according to any of items 14 to 16, wherein the cell adhesion layer is disposed in a capture region of the inner surface that is not parallel to the centrifugal direction of the centrifugal container.

[Item 18]

The cell capture method according to any of items 14 to 17, wherein the target cells include at least one type selected from the group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells, and the cell adhesion layer adsorbs the target cells.

[Item 19]

The cell capture method according to any of items 14 to 18, wherein the first biocompatible polymeric material includes one or more of the polymers expressed by the following formulas 1 to 4, or a copolymer of a monomer constituting the polymer expressed by formula 1 and a monomer constituting one or more types of polymer selected from the group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

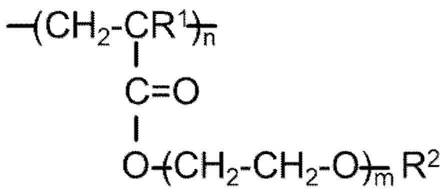

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

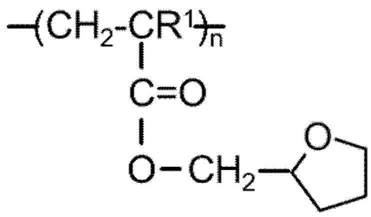

(2)

(In formula 2, $R^1$ is a hydrogen atom or a methyl group, and n is a repeating unit.)

Chemical Formula 3

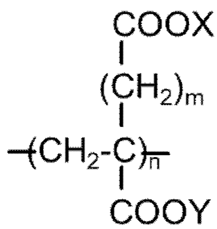

(3)

(In formula 3, X and Y are each independently a hydrogen atom, an alkali metal atom, or an ammonium group, m is an integer of 0, 1, or 2, and n is a repeating unit.)

Chemical Formula 4

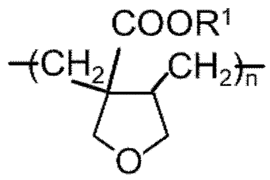

(4)

(In formula 4, $R^1$ is a hydrogen atom or an organic group having 1 to 30 carbon atoms, and n is a repeating unit.)

The cell capture device, cell capture apparatus, and cell capture method of this embodiment capture target cells in a test liquid, such as blood or lymphatic fluid. The target cells are at least one type selected from the group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells contained in intravascular fluids such as blood and lymphatic fluid. The cell capture device, cell capture apparatus, and cell capture method of this embodiment will now be described in specific terms with reference to the drawings.

First Embodiment

Fig. 1 shows the appearance of a cell capture apparatus 301 of this embodiment. The cell capture apparatus 301 comprises a cell capture apparatus 101 and a centrifuge 201.

Figs. 2 and 3 are an oblique view and a cross-sectional view of the cell capture apparatus 101. The cell capture apparatus 101 comprises a centrifugal container 110 and a cell adhesion layer 111. The centrifugal container 110 can be mounted in the centrifuge 201 as mentioned above. The centrifugal container 110 has a space 110s for holding a test liquid containing target cells, and an inner surface 113 that defines the space 110s. In this embodiment, the space 110s has a cylindrical shape. The shape of the space 110s is not limited to that of a cylinder, and may instead have some other shape. The centrifugal container 110 has a cylindrical shape having the space 110s. More specifically, the centrifugal container 110 has a tubular shape.

In this embodiment, the inner surface 113 that defines the space 110s includes a side surface portion 113s and a bottom surface portion 113b. When the cell capture apparatus 101 is mounted in the centrifuge 201 and centrifugal force is exerted on the cell capture apparatus 101, the centrifugal direction in which the centrifugal force acts on the cell capture apparatus 101 is indicated by the arrow G. Here, of the inner surface 113, the side surface portion 113s is substantially parallel to the centrifugal direction G. On the other hand, the bottom surface portion 113b is not parallel to the centrifugal direction G. Therefore, when centrifugal force acts on the cell capture apparatus 101 in a state in which the test liquid has been introduced into the space 110s, the test liquid and the target cells in the test liquid are pressed against the bottom surface portion 113b. The bottom surface portion 113b is a capture region against which target cells are pressed by centrifugal force. That is, the capture region is not parallel to the centrifugal direction.

In this embodiment, the bottom surface portion 113b is a single circular flat surface, and the side surface portion 113s is a single cylindrical curved surface. However, the bottom surface portion 113b and the side surface portion 113s may each be composed of a plurality of surfaces. Alternatively, the bottom surface portion 113b and the side surface portion 113s may be connected by a smooth curved surface, without any clear boundary between them.

The volume of the space 110s of the centrifugal container 110 can be determined according to the test liquid. For instance, the volume of the space 110s is at least 20 mL and no more than 50 mL. Also, the centrifugal container 110 can be made from various materials, such as glass, resin, or metal.

The cell adhesion layer 111 is disposed on the inner surface 113. More specifically, the cell adhesion layer 111 is disposed on at least on the bottom surface portion 113b of the inner surface 113, which is the capture region. The cell adhesion layer 111 is a layer that adsorbs target cells in the test liquid. The cell adhesion layer 111 includes a first biocompatible polymeric material.

The first biocompatible polymeric material may contain intermediate water. In a hydrated state, the first biocompatible polymeric material preferably contains intermediate water in a proportion of at least 1 wt% and no more than 30 wt% with respect to the whole. Also, the first biocompatible polymeric material preferably contains more intermediate water than free water in a hydrated state.

More specifically, the first biocompatible polymeric material includes one or more of the polymers expressed by the following formulas 1 to 4.

Chemical Formula 1

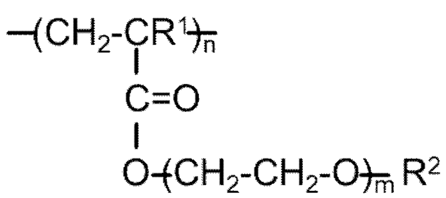

(1)

In formula 1, $R^1$ is a hydrogen atom or a methyl group, $R^2$ is a methyl group or an ethyl group, m is an integer of 1, 2, or 3, and n is a repeating unit.

Chemical Formula 2

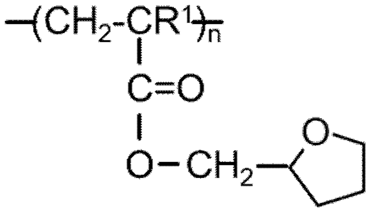

(2)

In formula 2, $R^1$ is a hydrogen atom or a methyl group, and n is a repeating unit.

Chemical Formula 3

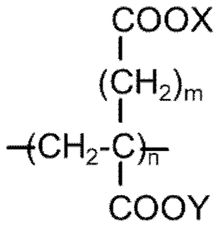

(3)

In formula 3, X and Y are each independently a hydrogen atom, an alkali metal atom, or an ammonium group, m is an integer of 0, 1, or 2, and n is a repeating unit.

Chemical Formula 4

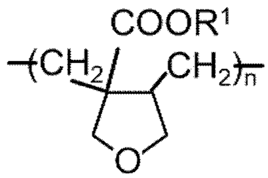

(4)

In formula 4, $R^1$ is a hydrogen atom or an organic group having 1 to 30 carbon atoms, and n is a repeating unit.
Specific examples of the polymer expressed by formula 1 include:

poly(2-methoxyethyl acrylate) (PMEA),
poly(2-ethoxyethyl acrylate) (PEEA or PEtEA),
poly[2-(2-methoxyethoxy)ethyl methacrylate] (PMe2A),
poly[2-(2-ethoxyethoxy)ethyl acrylate] (PEt2A),
poly(6-methoxyhexyl acrylate) (PMC6A),
poly(butoxyethyl acrylate) (PBuEA),
poly[2-(2-ethoxyethoxy)ethyl acrylate] (PEt2A),
poly[2-(2-(2-methoxyethoxy)ethoxy)ethyl acrylate], and
poly[2-(2-ethoxyethoxy)ethyl methacrylate].

A specific example of the polymeric material expressed by formula 2 is poly(tetrahydrofurfuryl acrylate).
Specific examples of the polymeric material expressed by formula 3 include polymethylene glutaric acid and polyitaconic acid.
A specific example of the polymeric material expressed by formula 4 is poly- α-allyloxymethyl methoxyethyl acrylate.
The first biocompatible polymeric material may include a copolymer of a monomer constituting the polymer expressed by formula 1 and a monomer constituting at least one polymer selected from the group consisting of polyethylene glycol, polyvinylpyrrolidone, polyvinyl methyl ether, polymethoxyethyl acrylamide, polymethoxyethyl methacrylamide, and polymethoxyethyl vinyl ether, polytetrahydrofurfuryl acrylate, and polytetrahydrofurfuryl methacrylate. According to Patent Literature 1, the size of the target cells that can be adsorbed varies with the amount of intermediate water that can be contained in the first biocompatible polymeric material. The amount of intermediate water that can be contained can be adjusted by varying the proportions of the two or more monomers in the copolymer.

The cell capture apparatus 101 may further comprise a cell adhesion suppression layer 112 that is disposed on the inner surface 113 in a region other than the capture region. In this embodiment, the side surface portion 113s, which is a region other than the bottom surface portion 113b, is a region other than the capture region, and the cell adhesion suppression layer 112 is located on the side surface portion 113s.

The cell adhesion suppression layer 112 is a layer that suppresses the adsorption of target cells in the test liquid. The cell adhesion suppression layer 112 includes a second biocompatible polymeric material. The second biocompatible polymeric material may contain intermediate water in a proportion of at least 30 wt% with respect to the whole in a hydrated state. The second biocompatible polymeric material may contain more free water than intermediate water in a hydrated state. Alternatively, the second biocompatible polymeric material may contain more intermediate water than free water in a hydrated state. The second biocompatible polymeric material is a polymer having phospholipid polar groups. More specifically, the second biocompatible polymeric material includes poly(2-methacryloyloxyethylphosphorylcholine ) and the like.

The first biocompatible polymeric material and the second biocompatible polymeric material may contain intermediate water and/or free water when capturing target cells, and the first biocompatible polymeric material and the second biocompatible polymeric material need not contain intermediate water, free water, etc., when the cell capture apparatus 101 is being fabricated, procured, stored, etc.

The cell adhesion layer 111 containing the first biocompatible polymeric material and the cell adhesion suppression layer 112 containing the second biocompatible polymeric material can be formed by spray dipping or another such coating method, by graft polymerization with ultraviolet rays, electron beams, radiation, or the like, by chemical reaction with a functional group of the material constituting the centrifugal container 110, or the like. Also, the thickness of the cell adhesion layer 111 and the cell adhesion suppression layer 112 is, for example, 0.01 to 0.4 $\mu$m.

Next, the centrifuge 201 will be described. The centrifuge 201 is, for example, an ordinary centrifuge of the kind used in medicine, life sciences, biotechnology, and other such fields. The cell capture apparatus 101 can be mounted in the centrifuge 201, and the centrifuge 201 applies centrifugal force to the cell capture apparatus 101 by the spinning cell capture apparatus 101. More specifically, the centrifuge 201 comprises a plurality of buckets 211 that hold a cell capture apparatus 101. Each of the buckets 211 is rotatably supported around one end of an arm 213 and a shaft 213a. The other end of the arm 213 is connected to a rotary shaft 212.

When the centrifuge 201 is started, the arms 213 rotate around the rotary shaft 212, causing the cell capture apparatuses 101 held in the buckets 211 to rotate around the rotary shaft 212. When the centrifugal force produced by this rotation acts on the cell capture apparatus 101, the buckets 211 change their orientation so that the bottom surface portions 211b of the buckets 211 face the outer peripheral side of a circle centered on the rotary shaft 212, as shown in Fig. 4. Consequently, the cell capture apparatuses 101 held in the buckets 211 spin in a state in which the bottom surface portions 113b are horizontal and facing toward the outer peripheral side of the circle centered on the rotary shaft 212, and are subjected to centrifugal force. As a result, the held test liquid and the target cells in the test liquid are pressed against the bottom surface portion 113b by this centrifugal force.

The centrifuge 201 is capable of rotating the cell capture apparatus 101 around the rotary shaft 212 in the range of 100 to 10,000 rpm, for example, and can exert a centrifugal force of from 100 $\times$ g (G) to 10,000 $\times$ g (G) on the test liquid held in the cell capture apparatuses 101 (discussed below).

Next, the capture of target cells by the cell capture apparatus 101 will be described.

Fig. 5 is a schematic diagram of the hydration structure of a target cell 150 and a biocompatible polymeric material 160. The biocompatible polymeric material 160 consists of a first biocompatible polymeric material and a second biocompatible polymeric material. The target cell 150 is surrounded by a hydration shell 151 under conditions in which a large amount of water is present, such as an intravascular fluid. The hydration shell 151 is made up of water molecules in various states. More specifically, this includes nonfreezing water 152 that is bound by strong interaction with the target cell 150, free water 154 that interacts weakly with the target cell 150, and intermediate water 153 that interacts with the target cell 150 with an intermediate strength that is in between nonfreezing water and free water.

Similarly, a hydration structure 161 is formed on the surface of the biocompatible polymeric material 160 in a hydrated state. The hydration structure 161 includes nonfreezing water 162 that is bound by strong interaction with the target cell 150, free water 164 that interacts weakly with the target cell 150, and intermediate water 163 that interacts with the target cell 150 with an intermediate strength that is in between nonfreezing water and free water.

In Fig. 5, for the sake of explanation, the nonfreezing water, the intermediate water, and the free water are shown as layers disposed in that order, starting from the target cell 150 and the biocompatible polymeric material 160, and the actual structure is not accurately reflected. The nonfreezing water is located close to atoms with which hydrogen bonding can occur, such as oxygen or nitrogen in the molecules that make up the target cell 150 and the biocompatible polymeric material 160.

Nonfreezing water does not freeze (solidify) even at -150° C or below because of its strong interaction with the target cells 150 or biocompatible polymeric material 160. Free water freezes or melts at 0° C. Intermediate water freezes or melts at a temperature below 0 ° C, such as at several tens of degrees below zero (Celsius). When water freezes or melts, heat is released or absorbed, so the proportion of water in these different states in the hydration shell 151 and hydration structure 161 can be found by using a differential scanning calorimeter to measure the amounts of heat released and absorbed, for example.

In general, a layer of intermediate water 163 and/or free water 164 is formed on the surface of biocompatible polymeric material 160 in a hydrated state. Therefore, when a target cell 150 surrounded by the hydration shell 151 approaches the surface of the biocompatible polymeric material 160, the hydration shell 151 of the target cell 150 is in contact with the intermediate water 163 or the free water 164 and interacts weakly. Since this interaction is not enough to pull out the water molecules of the hydration shell 151, there is no major change to the hydration shell 151, and the structure of the target cell is also maintained, which is believed to result in biocompatibility, or in other words, the target cell exhibits no foreign body reaction or rejection reaction.

On the other hand, with a bioincompatible polymeric material, it is possible that almost no layers of intermediate water and free water will be formed. Therefore, when the target cell 150 surrounded by the hydration shell 151 approaches the surface of a bioincompatible polymeric material, the water molecules in the hydration shell 151 of the target cell 150 may interact strongly with the bioincompatible polymeric material itself or with the nonfreezing water captured on the surface of the bioincompatible polymeric material, causing the water molecules to be desorbed from the hydration shell 151, or the structure of the hydration shell 151 may be greatly changed, resulting in changes in the three-dimensional structure of cells, damage, etc., with the upshot being that the cell ceases to function.

The first biocompatible polymeric material contains intermediate water in its hydrated state. As described in Patent Literature 1, a biocompatible polymeric material containing intermediate water selectively adsorbs the above-mentioned target cells in the intravascular fluid. More specifically, a biocompatible polymeric material containing intermediate water selectively captures the above-mentioned target cells without capturing blood cell components or protein components in the blood.

However, investigation by the inventors of the present application has revealed that a biocompatible polymeric material containing intermediate water will not capture a sufficient number of target cells because the adsorption of the target cells is not strong. Either that, or the capture takes a long time. Investigation performed by the inventors of the present application has found that, as described in the working examples given below, using centrifugal force is an effective means of increasing the number of captured cells without significantly affecting the activity of the target cells.

As shown in Fig. 6, the test liquid 170 containing the target cells 150 is introduced into the cell capture apparatus 101, the cell capture apparatus 101 is mounted in the centrifuge 201, and the cell capture apparatus 101 is spun as discussed above. As a result, centrifugal force is exerted on the test liquid 170 and the target cells 150 in the test liquid 170 along the centrifugal direction G as shown in Fig. 6. Since the cell adhesion layer 111 containing the first biocompatible polymeric material is disposed on the bottom surface portion 113b, which is a capture region not parallel to the centrifugal direction G, the target cells 150 subjected to centrifugal force are pressed against the cell adhesion layer 111.

The mechanism by which the target cells 150 are efficiently captured by the cell adhesion layer 111 by the application of centrifugal force is assumed to be as follows.

(1) Expansion of Contact Surface Area

As shown in the upper part of Fig. 7, the target cells 150 surrounded by hydration shells are believed to have an overall spherical shape. Therefore, when the target cells 150 approach or come into contact with the hydration structure 161 formed on the surface of the cell adhesion layer 111 due to natural settling caused by gravity, for example, the surface area where interaction with the cell adhesion layer 111 occurs is small. By contrast, when centrifugal force is exerted on the target cells 150, it is believed that the target cells 150 deform into a flattened shape under the centrifugal force. It is thought that this enlarges the surface area over which there is interaction with the hydration structure 161, and that the target cells 150 are securely adsorbed by the cell adhesion layer 111.

(2) Fixing of Target Cells

As shown in the upper part of Fig. 8, when the target cells 150 approach or come into contact with the hydration structure 161 formed on the surface of the cell adhesion layer 111, if the interaction is weak, the water molecules in the target cells 150 will move of near the surface of the hydration structure 161, making stable interaction difficult. By contrast, it is believed that when centrifugal force is exerted on the target cells 150, the target cells 150 move closer to the hydration structure 161, making it possible for stable interaction with the water molecules in the hydration structure 161 to interact earlier, and the target cells 150 are strongly adsorbed by the cell adhesion layer 111.

(3) Elimination of Stacked Structure

As shown in the upper part of Fig. 9, when a large number of target cells 150 are contained in the test liquid, they will sometimes stack up on the surface of the cell adhesion layer 111 by natural settling. The target cells 150A in the lower layer closer to the cell adhesion layer 111 have stable interaction due to their proximity to the hydration structure 161, but the target cells 150B in the upper layer interact more weakly because they are father away from the hydration structure 161. By contrast, when centrifugal force is exerted on the target cells 150, the target cells 150B in the upper layer are subjected to the centrifugal force before the target cells 150A in the lower layer of the stacked target cells 150 undergo stable interaction with the hydration structure 161, and push away the underlying target cells 150A in the lower layer, thereby moving closer to the cell adhesion layer 111. Consequently, it is believed that the target cells 150A and 150B are both in close proximity to the hydration structure 161 and undergo stable interaction, and are thereby more securely adsorbed to the cell adhesion layer 111.

(4) Reduction of Adsorption Time

When the target cells are in a dispersed state in the test liquid, those target cells located farther away from the cell adhesion layer will take longer to approach the cell adhesion layer. By contrast, when centrifugal force is applied to the test liquid, it is possible to bring any target cells that are farther away from the cell adhesion layer closer to the cell adhesion layer, so that many target cells can be adsorbed to the cell adhesion layer in a short time.

In addition to the effect produced by centrifugal force, when some target cells, such as circulating tumor cells in the blood, are adsorbed to the cell adhesion layer, this is believed to be related to the strong interaction of the cell adhesion molecules possessed by the cells with the cell adhesion layer.

Also, the amount of target cells is extremely small compared to blood cell components in the blood. Therefore, when centrifugal force is exerted on the test liquid, there will be cases in which a large amount of blood cell component settles on the cell adhesion layer 111 and interfere with the adsorption of the target cells 150 by the above-mentioned action. In such a case, it is preferable to remove the blood cell component from the test liquid in advance, or reduce the blood cell component to the extent that capture of the target cells 150 will not be significantly affected. For example, the test liquid may be subjected to pretreatment such as red blood cell separation using a specific gravity adjusting agent, or red blood cell removal using a hemolyzing agent.

In this embodiment, the cell capture apparatus 101 comprises the cell adhesion suppression layer 112 disposed in the region of the inner surface 113 other than the capture region, that is, the side surface portion 113s. Since the side surface portion 113s is parallel to the centrifugal direction, the target cells located near the side surface portion 113s are subjected to centrifugal force without making contact with the side surface portion 113s, and move toward the cell adhesion layer 111 located on the bottom surface portion 113b. However, if the centrifugal container 110 is formed from a material that is not biocompatible, there is the possibility that the target cells near the surface of the side surface portion 113s will make contact with or be adsorbed to the side surface portion 113s and be damaged. Nevertheless, if the cell adhesion suppression layer 112 is disposed on the side surface portion 113s, the target cells are less likely to be damaged in this way. Since it is possible for the cell adhesion suppression layer 112 to contain more free water than intermediate water, nearby target cells are not strongly bound. Therefore, disposing the cell adhesion suppression layer 112 makes it possible to efficiently capture the target cells in the capture region without damaging the target cells in the test liquid.

Thus, with the cell capture device and the cell capture apparatus of this embodiment, it is possible to efficiently capture target cells such as circulating tumor cells in the test liquid by using centrifugal force. Also, since the target cells are captured using a biocompatible polymeric material, it is possible to capture target cells while they are alive, with less damage to the target cells.

Various modifications can be made to the cell capture device and cell capture apparatus of this embodiment. The side surface portion 113s of the cell capture apparatus 101 may be configured differently. For example, the cell capture apparatus 102 shown in Fig. 10 differs from the cell capture apparatus 101 in that it comprises a cell adhesion layer 114 on the side surface portion 113s. A cell adhesion layer 114 is similar to the cell adhesion layer 111 in including a first biocompatible polymeric material. Materials that can be used as the first biocompatible polymeric material are as discussed above. The first biocompatible polymeric material of the cell adhesion layer 111 and the first biocompatible polymeric material of the cell adhesion layer 114 may be the same or different.

When the side surface portion 113s is covered by the cell adhesion layer 114, a single layer can be formed on the surface of the inner surface 113, so the cost of manufacturing the cell adhesion layer 114 can be reduced, and fewer manufacturing steps will be entailed. On the other hand, when the side surface portion 113s is covered by the cell adhesion layer 114, it is possible that the target cells will also be adsorbed to the side surface portion 113s. However, the centrifugal force acts in the direction of moving the target cells adsorbed to the cell adhesion layer 114 of the side surface portion 113s parallel to the surface of the cell adhesion layer 111. Accordingly, the target cells are substantially less likely to be adsorbed to the side surface portion 113s.

The cell capture apparatus 103 shown in Fig. 11 is different from the cell capture apparatus 101 in that a centrifugal container 110' is formed from a second biocompatible polymeric material, and no cell adhesion suppression layer is provided. Because the centrifugal container 110' is formed from the second biocompatible polymeric material, the side surface portion 113's is biocompatible and is unlikely to adsorb target cells even though no cell adhesion suppression layer is provided. Consequently, the same effect as that with the cell capture apparatus 101 can be exhibited.

The cell capture apparatus 104 shown in Fig. 12 differs from the cell capture apparatus 101 in that the bottom surface portion 113b' of the inner surface 113' of the centrifugal container 110" has a partially spherical shape. The bottom surface portion 113b' is preferably part of a spherical surface whose center is a point 212c at which the rotary shaft 212 intersects with a plane parallel to the centrifugal direction and passing through the center of the cell capture apparatus 103 when the cell capture apparatus 103 is mounted in the centrifuge 201 the cell capture apparatus 103 is spun so that the cell capture apparatus 103 faces in the horizontal direction. Accordingly, in a plane perpendicular to the rotary shaft 212, the cell adhesion layer 111 is disposed on an arc with respect to the center of rotation of the cell capture apparatus 103.

Because the bottom surface portion 113b' has this shape, the centrifugal force acting on the bottom surface portion 113b' is uniform. Accordingly, centrifugal force of the same magnitude acts on the target cells regardless of the position on the bottom surface portion 113b', and the target cells are uniformly distributed and pressed against the bottom surface portion 113b'. This makes it possible to capture target cells more efficiently.

Second Embodiment

Fig. 13 shows the appearance of a cell capture apparatus 302 in this embodiment. The cell capture apparatus 302 comprises a cell capture apparatus 105 and a centrifuge 202. Figs. 14 and 15 are an exploded oblique view and a cross-sectional view of the cell capture apparatus 105.

With the cell capture apparatus 105 in this embodiment, centrifugal force is exerted on the held test liquid by rotating around a rotary shaft located inside the cell capture apparatus 105. As shown in Figs. 14 and 15, the cell capture apparatus 105 comprises a centrifugal container 120 and the cell adhesion layer 111 disposed inside the centrifugal container 120. The centrifugal container 120 has a disk shape with an upper surface 120a, a lower surface 120b, and a rotary shaft 120c.

In this embodiment, the centrifugal container 120 includes a base 121' and a cover 122. The base 121' includes the rotary shaft 120c and a space 120s. The base 121' has a disk shape with a specific thickness, and the space 120s is formed in the base 121'. In this embodiment, the space 120s is formed at a position that is away from the rotary shaft 120c in the radial direction. The space 120s has a truncated-cone side shape in a plan view parallel to the upper surface 120a.

The inner surface 113 defining the space 120s has an inner side surface portion 113a that is the closest to the rotary shaft 120c in the radial direction, an outer side surface portion 113b' that is the farthest from the rotary shaft 120c, side portions 113c that are parallel to the radial direction, a bottom surface portion 113d, and an top surface portion 113e. The top surface portion 113e is located on the cover 122.

The disc shape of the centrifugal container 120 has, for example, a diameter of 50 to 200 mm and a thickness of 10 to 30 mm. Also, the volume of the space 120s is 20 to 50 mL, for example.

With the centrifugal container 120, when the centrifugal container 120 rotates around the rotary shaft 120c, the radial direction centered on the rotary shaft 120c is the centrifugal direction G. The outer side surface portion 113b' is not parallel to the centrifugal direction, and the test liquid introduced into the space 120s is pressed against the outer side surface portion 113b'. Therefore, the outer side surface portion 113b' is the capture region, and the cell adhesion layer 111 is disposed on the outer side surface portion 113b'. The cell adhesion suppression layer 112 is disposed on the inner side surface portion 113a, the side surface portions 113c, the bottom surface portion 113d, and the top surface portion 113e.

The cover 122 has a disk shape and is disposed so as to cover the upper surface of the base 121'. The cover 122 is provided with an opening 122h that communicates with the space 120s. The cover 122 may be detachably attached to the base 121', or may be permanently joined. A detachable configuration makes it easier to separate the target cells that have been adsorbed to the cell adhesion layer 111.

The centrifuge 202 comprises a turntable 220' on which the cell capture apparatus 105 can be mounted. Mounting the cell capture apparatus 105 into which the test liquid has been introduced on the turntable 220' and rotating the turntable 220' allows the target cells to be adsorbed to the cell adhesion layer 111 as described in the first embodiment.

The centrifuge 202 may, for example, make use of a sample analyzer that is used to detect and quantify a specific component in blood by using a disk-shaped sample analysis substrate, or may be a dedicated centrifuge for the cell capture apparatus 105 having a structure that rotates the disc-shaped substrate of this sample analyzer.

When the cell capture apparatus 302 is used, a test liquid containing the target cells is introduced into the space 120s of the cell capture apparatus 105 through the opening 122h, and the cell capture apparatus 105 is mounted on the

turntable 220' of the centrifuge 202. When the centrifuge 202 is switched on and the cell capture apparatus 105 is rotated, centrifugal force acts on the target cells, and the target cells are efficiently adsorbed to the cell adhesion layer 111 as described in the first embodiment.

Third Embodiment

An embodiment of a cell capture method will now be described. Fig. 16 is a flowchart of the cell capture method in this embodiment. The cell capture method of this embodiment comprises a step of bringing a liquid containing water into contact with the cell adhesion layer (S 1), a step of introducing a test liquid (S2), a step of adsorbing target cells to the cell adhesion layer (S3), a step of washing the cell adhesion layer (S4), and a step of separating the target cells from the cell adhesion layer (S5). These steps will be described in detail below.

(1) Step of bringing liquid containing water into contact with cell adhesion layer (S1)

A cell capture apparatus equipped with a cell capture device and a centrifuge is readied. The cell capture apparatus may be the one from either the first or second embodiment. The following description will refer to the cell capture apparatus 301 shown in Fig. 1.

A liquid containing water is brought into contact with the cell adhesion layer 111 of the cell capture apparatus 101 of the readied cell capture apparatus.

Any liquid containing water, pure or otherwise, can be brought into contact with the cell adhesion layer 111 so long as a hydration structure containing intermediate water is formed on the surface of the cell adhesion layer 111. The cell capture apparatus 101 has the cell adhesion suppression layer 112, and since it is preferable to form a hydration structure also on the surface of the cell adhesion suppression layer 112, the liquid containing water is also brought into contact with the cell adhesion suppression layer 112. The water-containing liquid may be the mother liquor component of the test liquid. For example, it may be serum or a component containing serum. Using the mother liquor component of the test liquid protects or maintains the hydration structure formed on the surfaces of the cell adhesion layer 111 and the cell adhesion suppression layer 112, so even if the test liquid is introduced while excess mother liquor component of the test liquid remains on these surfaces, the target cells and other components in the test liquid will make contact with the liquid in different environments, making modification less likely.

Also, this step (S1) may be omitted in the event that a hydration structure is quickly formed on the surfaces of the cell adhesion layer 111 and the cell adhesion suppression layer 112 by the water contained in the mother liquor of the test liquid upon introduction of the test liquid. Also, this step (S1) can be omitted in the event that the cell capture apparatus 101 is stored in a state in which a hydration structure is formed on the surfaces of the cell adhesion layer 111 and the cell adhesion suppression layer 112.

(2) Step of introducing test liquid (S2)

A test liquid containing target cells is introduced into the space 110s of the cell capture apparatus 101. If the test liquid is blood, then blood cell components may be removed in advance by centrifugation or the like.

(3) Step of adsorbing the target cells to the cell adhesion layer (S3)

The cell capture apparatus 101 is mounted in the centrifuge 201, the centrifuge 201 is switched on, and the cell capture apparatus 101 is spun to adsorb the target cells to the cell adhesion layer 111. The rotation duration is 1 to 30 minutes, for example, and the applied centrifugal force is from 500 $\times$ g (G) to 10,000 $\times$ g (G). The rotation duration and the magnitude of the centrifugal force can be determined on the basis of the concentration of the target cells in the test liquid, the size (molecular weight) of the target cells, and so forth.

(4) Step of washing the cell adhesion layer (S4)

The centrifuge 201 is stopped and the cell capture apparatus 101 is removed from the centrifuge 201. In order to remove any cells or the like not adsorbed to the cell adhesion layer 111, the test liquid is taken out and the surface of the cell adhesion layer 111 is rinsed with physiological saline, cell culture medium, or the like.

(5) Step of separating the target cells from the cell adhesion layer (S5)

When the target cells are to be cultured, they can be cultured in a medium together with the cell adhesion layer to which the target cells have been adsorbed, for example. When the target cells are separated from the cell adhesion

layer 111 to be analyzed, the target cells can be separated from the cell adhesion layer 111 by enzymatic treatment using trypsin or the like, for example.

With the cell capture method of this embodiment, as described in the first embodiment, centrifugal force can be used to efficiently capture target cells such as circulating tumor cells in the test liquid. Also, since a biocompatible polymeric material is used to capture the target cells, it is possible to capture the target cells while they are alive, with less damage to the target cells.

Experimental Example

An experiment was conducted to confirm the effect that centrifugal force has on adsorption to the cell adhesion layer. The details and results of the experiment will be described below.

1. Preparation of Sample

(1) Readying Cell Capture Apparatus

The cell capture device of the first embodiment was readied. A centrifugal container having a bottom surface portion 113b with a diameter of 30 mm and a depth of 50 mm was readied as the cell capture apparatus 101. As the cell adhesion layer 111, poly(2-methoxyethyl acrylate) having a number average molecular weight (Mn) of 100,000 was formed on the bottom surface portion 113b by a coating method. The thickness was 0.1 μm. No cell adhesion suppression layer 112 was formed.

(2) Preparation of Test Liquid

Cultured cancer cells (A549) were suspended in a serum-containing medium (DMEM) at 5000 cells/mL.

(3) Formation of Hydration Structure

The serum-containing medium (DMEM) was applied dropwise to the cell adhesion layer 111 of the cell capture apparatus 101 and allowed to stand for 1 hour.

(4) Adsorption of Test Liquid

The medium on the cell adhesion layer 111 was removed, and the test liquid was introduced into the cell capture apparatus 101. A plurality of samples were produced by mounting the cell capture apparatus 101 in the centrifuge 201 and varying the centrifugal force and duration. The cell capture apparatus 101 was rotated so that centrifugal forces of 1000 × g, 2000 × g and 3000 × g were applied. Samples were produced by spinning for 10, 20, and 30 minutes at each centrifugal force.

(5) In order to remove any cancer cells not adsorbed to the cell adhesion layer 111, the test liquid was stirred using a Pipetman, and the test liquid was removed. The number of cancer cells adsorbed to the cell adhesion layer 111 was found using a hemocytometer, and the capture rate was calculated from the number thus obtained.

(6) As a comparative example, samples were produced in the same manner by allowing them to stand for 0, 10, 20, and 30 minutes, but without applying any centrifugal force.

2. Results and Discussion

Fig. 17 shows the experiment results. The horizontal axis is the rotation duration, that is, how long the centrifugal force was applied, and the vertical axis is the capture rate. First, the results of the comparative example in which no centrifugal force was applied will be described. When no centrifugal force was applied, cancer cells were captured at a rate of 0.1 or less when the test liquid is brought into contact with the cell adhesion layer 111, but the capture rate barely changes in the first 10 minutes or so. After that, the capture rate gradually increases over time. However, even after 30 minutes have elapsed, the capture rate is still only about 0.3.

On the other hand, when centrifugal force was applied, regardless of whether the centrifugal force was 1000 × g, 2000 × g, or 3000 × g, the capture rate was increased sharply by the application of centrifugal force for 10 minutes, reaching 0.7 or more. After that, the capture rate barely changed over time. There is no major difference in the capture rate between centrifugal forces of 1000 × g and 2000 × g, but when a centrifugal force of 3000 × g is applied, a capture rate of about 0.9 is obtained.

Based on these results, it is believed that the effect that centrifugal force has on improving the capture rate

appears in a short period of time. Also, since a high capture rate was obtained, it is believed that the cancer cells adsorbed to the cell adhesion layer 111 by centrifugal force are strong enough not to dissociate.

Since the cancer cells can thus be adsorbed to the cell adhesion layer 111 in a short time, it can be seen that the damage to the adsorbed cancer cells is kept to a minimum, that is, the cancer cells can be adsorbed in a live state.

Fourth Embodiment

A centrifugal container 20 and a cell capture apparatus 10 equipped with the same according to an embodiment of the present invention will now be described with reference to Figs. 18 to 29.

In this embodiment, unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

(1) Configuration of Cell Capture Apparatus 10

The cell capture apparatus 10 according to this embodiment is an apparatus for capturing target cells C1 contained in a test liquid such as blood or lymphatic fluid, and comprises a centrifuge 11 and a centrifugal container 20 as shown in Figs. 18 and 19.

Here, the target cells C1 captured by the cell capture apparatus 10 are, for example, at least one type selected from among circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells, which are contained in intravascular fluid (test liquid), such as blood or lymphatic fluid.

The centrifuge 11 is, for example, an ordinary centrifugation device used in fields such as medicine, life sciences, and biotechnology, and is spun in a state in which the centrifugal container 20 has been mounted, which applies centrifugal force to the test liquid containing the target cells that has been put in the centrifugal container 20. As shown in Figs. 18 and 19, the centrifuge 11 comprises a main body 12, a lid 13, a mounting portion 14 in which the centrifugal container 20 is mounted, and a display unit 15.

The main body 12 is a portion that constitutes the housing of the centrifuge 11, and the mounting portion 14 is provided in the interior space thereof.

The lid 13 is attached to the main body 12 so as to be openable and closable, and is opened or closed when the centrifugal container 20 mounted in the internal space is put in or taken out.

The mounting portion 14 is provided in the internal space of the main body 12 and rotates the mounted centrifugal container 20.

The display unit 15 is provided on the outer side surface on the front side of the main body 12, and displays various information, such as the setting of the centrifuge 11 and the usage state.

When the centrifuge 11 is switched on to spin the centrifugal container 20 mounted on the mounting portion 14, centrifugal force is applied to the test liquid contained in the centrifugal container 20. The centrifuge 11 spins the centrifugal container 20 around the rotation axis 23 in the range of 100 to 10,000 rpm, for example. Consequently, a centrifugal force of 100 to 10,000 $\times$ g (G), for example, is applied to the test liquid held in the centrifugal container 20 (discussed below).

The rotation axis 23 serving as the center of rotation when the centrifugal container 20 is spun by the centrifuge 11 may be a rotary shaft physically provided to the centrifugal container 20, or may be a virtual rotation axis generated on the centrifugal container 20 side during rotational drive by the centrifuge 11.

The centrifugal container 20 is rotationally driven while mounted to the mounting portion 14 of the centrifuge 11. This allows centrifugal force to be applied to the test liquid contained in the centrifugal container 20.

The detailed configuration of the centrifugal container 20 will be described below.

Here, the principle of using the centrifugal container 20 according to this embodiment to capture the target cells C1 contained in the test liquid will be described with reference to Fig. 20.

The centrifugal container 20 has a bottom surface (inner surface, bottom surface portion) 24a that is included in the inner surface that forms a holding space in which a test liquid (discussed below) is held, and a cell adhesion layer 27 that is provided in a capture region such as the bottom surface 24a and adhesively captures the target cell C1 contained in the cell (see Fig. 21A, etc.).

The cell adhesion layer 27 is a layer that adsorbs the target cells C1 contained in the test liquid and does not adsorb contaminants C2 other than the target cells C1, and contains a first biocompatible polymeric material.

The first biocompatible polymeric material includes one or more of the polymers expressed by the following

formulas 1 to 4, or a copolymer of a monomer constituting the polymer expressed by formula 1 and a monomer constituting one or more types of polymer selected from the group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

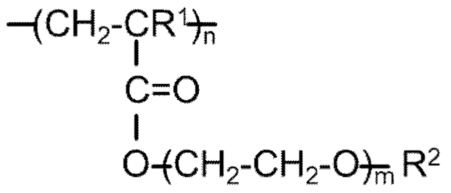

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

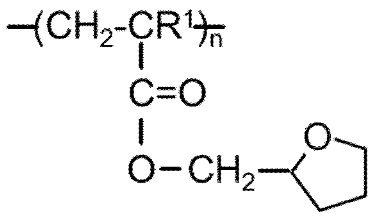

(2)

(In formula 2, $R^1$ is a hydrogen atom or a methyl group, and n is a repeating unit.)

Chemical Formula 3

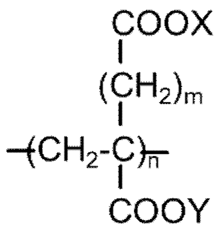

(3)

(In formula 3, X and Y are each independently a hydrogen atom, an alkali metal atom, or an ammonium group, m is an integer of 0, 1, or 2, and n is a repeating unit.)

Chemical Formula 4

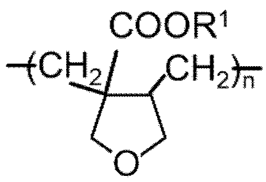

(4)

(In formula 4, $R^1$ is a hydrogen atom or an organic group having 1 to 30 carbon atoms, and n is a repeating unit.)

Fig. 20 is a schematic diagram illustrating the hydration structure of the target cell C1 and the biocompatible polymeric material 40.

The biocompatible polymeric material 40 represents a first biocompatible polymeric material and a second biocompatible polymeric material.

In a state in which a large quantity of water such as intravascular fluid (test liquid) is present, the target cell C1 is surrounded by the hydration shell 31 as shown in Fig. 20.

The hydration shell 31 is made up of water molecules in various states. More specifically, the hydration shell 31 includes nonfreezing water 32 that strongly interacts with the target cell C1, free water 34 that weakly interacts with the target cell C1, and intermediate water 33 that interacts with the target cell C1 at a strength that is in between that of the nonfreezing water 32 and the free water 34.

Similarly, the hydration structure 41 is formed, in a hydrated state, on the surface of the biocompatible polymeric material 40 included in the cell adhesion layer 27.

The hydration structure 41 includes nonfreezing water 42 bound by strong interaction with the biocompatible polymeric material 40, free water 44 that weakly interacts with the target cell C1, and intermediate water 43 that interacts with the target cell C1 at a strength that is in between that of the nonfreezing water 42 and the free water 44.

In Fig. 20, for the sake of description, the nonfreezing water 32 and 42, the intermediate water 33 and 43, and the free water 34 and 44 are shown as layers disposed in that order, on the target cell C1 and the biocompatible polymeric material 40, but this does not accurately reflect the actual structure.

The nonfreezing water 32 and 42 are disposed near atoms with which hydrogen bonding can occur, such as oxygen and nitrogen, in the molecules constituting the target cell C1 and the biocompatible polymeric material 40.

The nonfreezing water 32 and 42 do not freeze (solidify) even at -150° C or lower because of their strong interaction with the target cell C1 or the biocompatible polymeric material 40.

The free water 34 and 44 freeze or melt at 0° C.

The intermediate water 33 and 43 freeze or melt at a temperature of 0° C or below, such as at several tens of degrees Celsius below zero. When water freezes or melts, heat is released or absorbed, so the proportion of water in these different states in the hydration shell 31 and hydration structure 41 can be found by measuring the amount of heat absorbed or released using a differential scanning calorimeter, for example.

In general, a layer of intermediate water 43 and/or free water 44 is formed on the surface of the biocompatible polymeric material 40 in the hydrated state. Therefore, when the target cell C1 surrounded by the hydration shell 31 approaches the surface of the biocompatible polymeric material 40, the hydration shell 31 of the target cell C1 makes contact with the intermediate water 43 or the free water 44, and the interaction is weak. It is believed that since this interaction is not enough to pull out the water molecules from the hydration shell 31, there is little change in the structure of the hydration shell 31, and the structure of the target cell C1 is also maintained, so there is no biocompatibility, that is, the target cell C1 does not exhibit foreign body reaction or rejection.

On the other hand, with a bioincompatible molecular material, it is believed that almost no layers of intermediate water 33 and 43 or free water 34 and 44 are formed. Accordingly, when the target cell C1 surrounded by the hydration shell 31 approaches the surface of the bioincompatible polymeric material, the water molecules in the hydration shell 31 of the target cell C1 interact strongly with the bioincompatible polymeric material itself or with the nonfreezing water captured on the surface of the bioincompatible polymeric material, causing the water molecules to detach from the hydration shell 31, or there is a major change in the structure of the hydration shell 31, causing the three-dimensional structure of the target cell to change, or damage occurs, etc., all of which results in cells ceasing to function.

Also, in the centrifugal container 20, cell adhesion suppression layers 28a and 28b are provided to the inner surface forming the holding space 24, at locations (the outer peripheral surface 24b and the inner peripheral surface 24c) other than the bottom surface 24a serving as the capture region (see Fig. 23).

The cell adhesion suppression layers 28a and 28b are layers that suppress the adsorption of the target cells C1 in the test liquid, and contain a second biocompatible polymeric material.

The second biocompatible polymeric material contains intermediate water in a proportion of at least 30 wt% with respect to the whole in a hydrated state. In a hydrated state, the second biocompatible polymeric material may contain more free water than intermediate water, or may contain more intermediate water than free water. The second biocompatible polymeric material is a polymer having phospholipid polar groups, such as poly(2-methacryloyloxyethylphosphorylcholine).

The first biocompatible polymeric material and the second biocompatible polymeric material should contain intermediate water and/or free water when capturing target cells, but when the centrifugal container 20 is being produced, procured, stored, or the like, the first biocompatible polymeric material and the second biocompatible polymeric material need not contain intermediate water, free water, etc.

The cell adhesion layer 27 containing the first biocompatible polymeric material and the cell adhesion suppressing layers 28a and 28b containing the second biocompatible polymeric material can be formed by a coating method such as spray dipping, by graft polymerization with ultraviolet rays, electron beams, radiation, or the like, a chemical

reaction with the functional groups had by the material forming the centrifugal container 20, or the like. Also, the thickness of the cell adhesion layer 27 and the cell adhesion suppression layers 28a and 28b is 0.01 to 0.4 μm, for example.

As discussed above, since the first biocompatible polymeric material contains the intermediate water 43 in a hydrated state, which means that the above-mentioned target cells C1 in the test liquid can be selectively adsorbed and captured.

More specifically, the biocompatible polymeric material containing intermediate water can selectively capture the target cells C1 without capturing any contaminants C2, such as blood cell components and protein components in blood (the test liquid).

With the centrifugal container 20 of this embodiment, the centrifugal container 20 into which the test liquid containing the target cells C1 has been introduced is mounted in the centrifuge 11, and the centrifugal container 20 is spun around the rotation axis 23. As a result, as shown in Figs. 21A and 21B, centrifugal force G is applied to the test liquid, the target cells C1 in the test liquid, and the contaminants C2 in the test liquid in the direction of the arrow in Fig. 21B.

At this point, the cell adhesion layer 27 containing the first biocompatible polymeric material is disposed on the bottom surface (inner surface) 24a, which is substantially parallel to the centrifugal direction in which the centrifugal force G is applied. Therefore, the target cells C1 adhere to the cell adhesion layer 27 upon sinking to the bottom surface 24a in the test liquid before the centrifugal force G is applied.

Consequently, the target cells C1 adhered to the cell adhesion layer 27 remain held on the bottom surface 24a by the cell adhesion layer 27 even when subjected to the centrifugal force G produced by the rotation of the centrifuge 11. On the other hand, the contaminants C2 other than the target cells C1 receive the centrifugal force G and move radially outward around the rotation axis 23.

As a result, when the centrifugal container 20 is spun around the rotation axis 23, the contaminants C2 other than the target cells contained in the test liquid are moved radially outward by the centrifugal force, and the target cells C1 can be held on the bottom surface 24a by the cell adhesion layer 27, which means that the target cells C1 can be selectively captured.

(2) Configuration of Centrifugal Container 20

As shown in Fig. 22, the centrifugal container 20 of this embodiment comprises a main body 21, a cover 22, a rotation axis 23, a holding space 24, a discharge port 25, and a collection chamber 26 (see Fig. 23).

As shown in Fig. 22, the main body 21 is a substantially cylindrical member, and has the rotation axis 23 at its center. Therefore, the centrifugal container 20 mounted in the centrifuge 11 is rotationally driven around the rotation axis 23.

As shown in Fig. 22, the cover 22 is a substantially disc-shaped member and is attached so as to cover the upper surface of the main body 21, and particularly the test liquid holding space 24 provided to the main body 21. The cover 22 has a vent hole 22a that allows the holding space 24 containing the test liquid to communicate with the outside air.

As shown in Fig. 22, the rotation axis 23 is provided at the center of the substantially cylindrical main body 21 and serves as the center of rotation when the centrifuge 11 spins the centrifugal container 20.

The holding space 24 is a space for holding the test liquid containing the target cells C1 and contaminants C2 other than the target cells C1, and as shown in Fig. 22, is provided as a substantially arc-shaped depression at a position that is away from the rotation axis 23 in the radial direction, on the upper surface side of the main body 21. The holding space 24 is formed by the bottom surface (inner surface, bottom surface portion) 24a, the outer peripheral surface (inner surface, outer side surface portion, first inclined portion) 24b, and the inner peripheral surface (inner surface, second inclined portion) 24c.

As shown in Fig. 23, the bottom surface (inner surface) 24a is a plane that is substantially parallel to the centrifugal direction of the centrifugal force G applied when the centrifugal container 20 spins, and forms the bottom of the holding space 24. The front of the bottom surface 24a is provided with the cell adhesion layer 27 that adsorbs and captures the target cells C1 as discussed above.

As shown in Figs. 22 and 23, the outer peripheral surface (inner surface, outer side surface portion, first inclined portion) 24b is a substantially arc-shaped surface formed so as to incline downward from the outside to the inside in the radial direction with respect to the bottom surface 24a, and is connected to the bottom surface 24a at the end on the inside in the radial direction. Also, the outer peripheral surface 24b is provided with the discharge port 25 at the end on the outside in the radial direction.

Furthermore, as shown in Fig. 23, the front of the outer peripheral surface 24b is provided with the cell adhesion suppression layer 28a that suppresses adsorption of the target cells C1 contained in the test liquid. Therefore, even if the target cells C1 come into contact with the outer peripheral surface 24b when the test liquid is put into the holding space 24 and the target cells C1 sink in the test liquid, it is less likely that the target cells C1 will be adsorbed to the outer peripheral surface 24b.

As shown in Figs. 22 and 23, the inner peripheral surface (inner surface, second inclined portion) 24c is a

substantially arc-shaped surface (in plan view) that is formed so as to incline downward from the inside to the outside in the radial direction with respect to the bottom surface 24a, and is connected to the bottom surface 24a at the end on the outside in the radial direction.

Furthermore, as shown in Fig. 23, as is the outer peripheral surface 24b, the front of the inner peripheral surface 24c is provided with the cell adhesion suppression layer 28a that suppresses adsorption of the target cells C1 contained in the test liquid. Therefore, even if the target cells C1 come into contact with the outer peripheral surface 24b when the test liquid is put into the holding space 24 and the target cells C1 sink in the test liquid, it is less likely that the target cells C1 will be adsorbed to the outer peripheral surface 24b.

Here, in this embodiment, as shown in Fig. 24, the inner peripheral surface 24c is disposed in an arc centered on the rotation axis 23, whereas the outer peripheral surface 24b is disposed in an eccentric shape in which a first distance d1 from the rotation axis 23 on the left side in Fig. 24 is different from a second distance d2 from the rotation axis 23 on the right side (d1 < d2).

That is, the outer peripheral surface 24b is formed such that the inside diameter (second distance d2) near the clockwise end of the substantially arc-shaped holding space 24 is greater than the inside diameter (first distance d2) near the counterclockwise end.

Also, as shown in Fig. 24, the discharge port 25 for discharging contaminants C2 moved by the centrifugal force G out of the holding space 24 is provided near the second distance d2 where the inside diameter is larger on the outer peripheral surface 24b.

As shown in Figs. 23 and 24, the discharge port 25 is a substantially circular through-hole in plan view, communicates with the collection chamber 26, and any contaminants C2 that have been moved radially outward by the centrifugal force G are made to sink and collected in collection chamber 26.

As shown in Fig. 23, the collection chamber 26 is provided directly below the discharge port 25, and collects and holds the contaminants C2 that drop out of the discharge port 25 under the centrifugal force G.

The way in which the target cells C1 and the contaminants C2 contained in the test liquid placed in the holding space 24 of the centrifugal container 20 move when the centrifugal container 20 of this embodiment is spun by the centrifuge 11 will now be described with reference to Figs. 25A to 28B.

In the centrifugal container 20 of this embodiment, as shown in Fig. 25A, when the test liquid is put into the holding space 24, the target cells C1 and the contaminants C2 are dispersed in the test liquid.

At this point, the target cells C1 and the contaminants C2 are in a state of floating in the test liquid as shown in Fig. 25B.

Next, the centrifugal container 20 is allowed to stand for a specific length of time (such as 1 hour). At this point, as shown in Fig. 26A, the target cells C1, the contaminants C2, etc., naturally sink in the test liquid.

As the target cells C1 and contaminants C2 further sink, the target cells C1 and contaminants C2 come into contact with the bottom surface 24a as shown in Fig. 26B.

At this point, the outer peripheral surface 24b and the inner peripheral surface 24c that constitute the holding space 24 are each inclined downward toward the bottom surface 24a. The cell adhesion suppression layers 28a and 28b are formed on the outer peripheral surface 24b and the inner peripheral surface 24c, respectively.

Therefore, the target cells C1 and contaminants C2 that have sunk in the test liquid continue sinking without being held on the outer peripheral surface 24b or the inner peripheral surface 24c, roll down the inclined surfaces, and move to the bottom surface 24a, and their position in the holding space 24 before the start of rotation is set.

Consequently, as shown in Fig. 26B, the target cells C1 and contaminants C2 contained in the test liquid can be collected on the bottom surface 24a on which the cell adhesion layer 27 is provided.

The waiting time (such as 1 hour) mentioned above is set to be long enough for the target cells C1 and the contaminants C2 in the test liquid placed in the holding space 24 of the centrifugal container 20 to settle down to the bottom surface 24a. Therefore, if the holding space 24 is deep, and if a large amount of test liquid is put in the holding space 24, a waiting time of a few hours (longer than 1 hour) may be set. On the other hand, if the holding space 24 is shallow, and only a small amount of test liquid is put in the holding space 24, a waiting time of about 30 minutes (shorter than 1 hour) may be set.

Once the specified waiting time has elapsed, the centrifuge 11 is switched on to start spinning the centrifugal container 20. At this point, a centrifugal force is applied to the test liquid in the centrifugal container 20 toward the outside in the radial direction around the rotation axis 23.

Consequently, a centrifugal force G is applied to the target cells C1 and the contaminants C2 that have settled on the bottom surface 24a in the test liquid. At this point, the target cells C1 are adsorbed by the cell adhesion layer 27 provided on the front of the bottom surface 24a, whereas the contaminants C2 are hardly adsorbed at all by the cell adhesion layer 27.

Consequently, as shown in Fig. 26C, when the target cells C1 and the contaminants C2 are subjected to the centrifugal force G, the target cells C1 are held on the bottom surface 24a, and only the contaminants C2 move outward in the radial direction centered on the rotation axis 23, and are driven up the inclined surface of the outer peripheral

surface 24b.

Then, as shown in Fig. 26D, the contaminants C2 drop into the collection chamber 26 from the discharge port 25 provided at the outermost peripheral portion in the radial direction of the outer peripheral surface 24b.

With the centrifugal container 20 of this embodiment, as discussed above, the outer peripheral surface 24b has a shape that is eccentric with respect to the inner peripheral surface 24c that lies on an arc centered on the rotation axis 23.

Therefore, the contaminants C2 that have moved outward in the radial direction under the centrifugal force applied by the rotation of the centrifugal container 20 move away from the rotation axis 23 along the outermost peripheral portion of the outer peripheral surface 24b as shown in Figs. 27A and 27B.

As the centrifugal container 20 continues to rotate, as shown in Figs. 28A and 28B, the contaminants C2 move away from the rotation axis 23 along the outermost peripheral portion of the outer peripheral surface 24b, drop out of the discharge port 25, and are collected in the collection chamber 26.

In the step of collecting the contaminants C2, the surface Z of the test liquid is formed on the inner peripheral surface 24c side as shown in Fig. 28B, and the target cells C1, etc., that are further to the right side than the liquid surface Z in the drawing can be maintained in a state of being immersed in the test liquid.

Consequently, even when the centrifugal container 20 is rotated to apply the centrifugal force G to the test liquid in order to collect the contaminants C2, the target cells C1 adsorbed to the cell adhesion layer 27 can be prevented from coming out of the test liquid and dying off.

In this embodiment, as described above, because the centrifugal container 20 is rotated to apply centrifugal force to the target cells C1 and the contaminants C2 contained in the test liquid contained in the holding space 24, just the contaminants C2 can be moved to the collection chamber 26 and washed, leaving the target cells C1 adsorbed to the cell adhesion layer 27 still held on the bottom surface 24a, which allows the target cells C1 to be easily removed from the test liquid.

Cell Capture Method

The cell capture method in this embodiment is carried out according to the flowchart shown in Fig. 29 by the cell capture apparatus 10, using the centrifugal container 20 described above.

That is, in step S11, a liquid containing water is brought into contact with the cell adhesion layer 27 provided on the bottom surface 24a of the holding space 24 of the centrifugal container 20. As long as a hydration structure containing intermediate water is formed on the surface of the cell adhesion layer 27, pure water or any liquid containing water can be brought into contact with the cell adhesion layer 27. Also, a liquid containing water is brought into contact with the cell adhesion suppression layers 28a and 28b on the outer peripheral surface 24b and the inner peripheral surface 24c of the holding space 24 of the centrifugal container 20.

The liquid containing water may be a mother liquor component of the test liquid. For example, it may be serum or a component containing serum. If a mother liquor component of the test liquid is used, then even if the test liquid is introduced while an excess of the mother liquor component of the test liquid remains on the surfaces of the cell adhesion layer 27 and the cell adhesion suppressing layers 28a and 28b in order to protect or maintain the hydration structure formed on these surfaces, the target cells and other components in the test liquid will still be less likely to come into contact with liquids in different environments and from being modified.

This step (S1 1) may be omitted if the water contained in the mother liquor of the test liquid upon introduction of the test liquid quickly forms a hydration structure on the surfaces of the cell adhesion layer 27 and the cell adhesion suppression layers 28a and 28b. Also, this step (S11) may be omitted if the centrifugal container 20 is stored in a state in which hydration structures are formed on the surfaces of the cell adhesion layer 27 and the cell adhesion suppression layers 28a and 28b.

Next, in step S12, a test liquid containing the target cells C1 is introduced into the holding space 24 of the centrifugal container 20. If the test liquid is blood, the blood cell components may be removed ahead of time by centrifugation or the like.

Next, in step S13, the system waits until a specific length of time (such as 1 hour) has elapsed since the test liquid was introduced in step S12.

As discussed above, the waiting time in step S13 may be set longer than 1 hour when the holding space 24 of the centrifugal container 20 is deep, or when a large amount of the test liquid put in the holding space 24, and may be set to less than 1 hour when the holding space 24 is shallow, or when a small amount of test liquid is put in the holding space 24.

Next, in step S14, when the waiting time in step S13 has elapsed, the target cells C1 and contaminants C2 contained in the test liquid settle through the test liquid and come into contact with the cell adhesion layer 27 on the bottom surface 24a.

At this point, the target cells C1 are adsorbed by the cell adhesion layer 27.

Next, in step S15, the centrifuge 11 is switched on and the centrifugal container 20 starts spinning.

The centrifugal container 20 is spun by the centrifuge 11 around the rotation axis 23 in the range of 100 to 10,000 rpm, for example. The rotation speed during the centrifugation may be controlled so as to be maintained at a substantially constant speed, or may be controlled so as to change in steps.

Next, in step S16, the contaminants C2 move outward in the radial direction inside the holding space 24 under the centrifugal force G imparted by the rotation of the centrifugal container 20, and are collected from the discharge port 25 into the collection chamber 26.

Next, in step S17, the system waits until the collection of the contaminants C2 in step S16 is nearly complete.

Next, in step S18, the rotation of the centrifugal container 20 by the centrifuge 11 is halted.

Next, in step S19, the centrifugal container 20 is taken out of the centrifuge 11, and the target cells C1 adsorbed by the cell adhesion layer 27 on the bottom surface 24a are separated and taken out.

Main Features

As shown in Fig. 18, etc., the centrifugal container 20 of this embodiment is a container that can be mounted in the centrifuge 11, and as shown in Fig. 23, etc., comprises the bottom surface 24a, the outer peripheral surface 24b, the inner peripheral surface 24c, and the cell adhesion layer 27. The bottom surface 24a, the outer peripheral surface 24b, and the inner peripheral surface 24c define the space in which the test liquid containing the target cells is held. The cell adhesion layer 27 is disposed in a capture region of the bottom surface 24a that is substantially parallel to the centrifugal direction in which the centrifugal force is applied by the centrifuge 11, and adsorbs the target cells in the test liquid.

Consequently, when the centrifugal container 20 is rotated by the centrifuge 11 and the centrifugal force G is applied to the target cells C1 and contaminants C2 contained in the test liquid placed in the holding space 24 of the centrifugal container 20, just the target cells C1 are adsorbed by the cell adhesion layer 27 provided on the bottom surface 24a of the holding space 24, so just the contaminants C2 other than the target cells C1 receive the centrifugal force G and move radially outward.

As a result, the contaminants C2 other than the target cells C1 (the detection target) are discharged, and the test liquid is washed, allowing just the target cells C1 to be efficiently captured.

Fifth Embodiment

The centrifugal container 120 according to another embodiment of the present invention will now be described with reference to Figs. 30 to 31B.

The centrifugal container 120 in this embodiment differs from the centrifugal container 20 of the fourth embodiment in that the outer peripheral surface 124b of the inner surface constituting the holding space 124 is not formed in an eccentric shape around the rotation axis 23, and is instead formed concentrically with the inner peripheral surface 124c, and that a plurality of discharge ports 125 are provided on the outer peripheral surface 124b.

The rest of the components are substantially the same as those of the centrifugal container 20 of the fourth embodiment, so members having the same function and shape will be numbered the same and will not be described in detail again.

As shown in Fig. 30, the centrifugal container 120 of this embodiment comprises a main body 121 that is rotated by the centrifuge 11 around the rotation axis 23, a holding space 124 that is formed in a substantially arc shape, and a plurality of discharge ports 125.

As shown in Fig. 30, the main body 121 is a substantially cylindrical member, and has a rotation axis 23 at its center.

The holding space 124 is a space for holding the test liquid containing the target cells C1 and the contaminants C2, and as shown in Fig. 30, is provided as a substantially arc-shaped recess at a position that is away from the rotation axis 23 in the radial direction, on the upper surface side of the main body 121. The holding space 124 is formed by a bottom surface (inner surface, bottom surface portion) 124a, an outer peripheral surface (inner surface, outer side surface portion, first inclined portion) 124b, and an inner peripheral surface (inner surface, second inclined portion) 124c.

As shown in Figs. 31A and 31B, the bottom surface (inner surface) 124a is a plane substantially parallel to the centrifugal direction of the centrifugal force G applied when the centrifugal container 120 spins, and forms the bottom of the space 124. The cell adhesion layer 27 that adsorbs and captures the target cells C1 is provided on the front of the bottom surface 124a.

As shown in Figs. 31A and 31B, the outer peripheral surface (inner surface, outer side surface portion, first inclined portion) 124b is a substantially arc-shaped surface formed so as to incline downward from the outside to the inside in the radial direction with respect to the bottom surface 124a, and is connected to the bottom surface 124a at the end on the inside in the radial direction. Also, the outer peripheral surface 124b is provided with the discharge ports

25 at the end on the outside in the radial direction.

Furthermore, as shown in Fig. 31B, the front of the outer peripheral surface 124b is provided with the cell adhesion suppression layer 28a that suppresses adsorption of the target cells C1 contained in the test liquid. Accordingly, even if the target cells C1 come into contact with the outer peripheral surface 124b when the test liquid is put into the holding space 24 and the target cells C1 sink in the test liquid, it is less likely that the target cells C1 will be adsorbed to the outer peripheral surface 124b.

As shown in Figs. 31A and 31B, the inner peripheral surface (inner surface, second inclined portion) 124c is a substantially arc-shaped surface (in plan view) that is formed so as to incline downward from the inside to the outside in the radial direction with respect to the bottom surface 124a, and is connected to the bottom surface 124a at the end on the outside in the radial direction.

Furthermore, as shown in Fig. 31B, as is the outer peripheral surface 124b, the front of the inner peripheral surface 124c is provided with the cell adhesion suppression layer 28a that suppresses adsorption of the target cells C1 contained in the test liquid. Therefore, even if the target cells C1 come into contact with the inner peripheral surface 124c when the test liquid is put into the holding space 124 and the target cells C1 sink in the test liquid, it is less likely that the target cells C1 will be adsorbed to the inner peripheral surface 124c.

Four discharge ports 125 are provided on the outermost peripheral side of the outer peripheral surface 124b. Consequently, even though the outer peripheral surface 124b does not have an eccentric shape as in the fourth embodiment, the contaminants C2 that have moved to the outermost peripheral side due to the application of the centrifugal force G can be moved from the four discharge ports 125 to the collection chamber 26.

Because the centrifugal container 120 of this embodiment can is configured as above, the same effect as in the fourth embodiment above can be obtained.

Sixth Embodiment

The centrifugal container 220 according to another embodiment of the present invention will now be described with reference to Figs. 32 to 34.

The centrifugal container 220 of this embodiment differs from the centrifugal container 20 of the fourth embodiment in that an outer peripheral surface 224b is formed in a curve in cross-sectional view and inclines downward to the inside in the radial direction, and that an inner peripheral surface 224c is formed as a plane that is substantially parallel with the rotation axis 23, and that the cell adhesion layer 27 is provided not only on the bottom surface 224a but also on the outer peripheral surface 224b.

The rest of the components are substantially the same as those of the centrifugal container 20 of the fourth embodiment, so members having the same function and shape will be numbered the same and will not be described in detail again.

As shown in Fig. 32, the centrifugal container 220 of this embodiment comprises a main body 221 that is rotated around the rotation axis 23 by the centrifuge 11, and a holding space 224 that is formed in a substantially arc shape.

As shown in Fig. 32, the main body 221 is a substantially cylindrical member, and has the rotation axis 23 at its center.

The holding space 224 is a space for holding the test liquid containing the target cells C1 and the contaminants C2, and as shown in Fig. 32, is provided as a substantially arc-shaped recess at a position that is away from the rotation axis 23 in the radial direction, on the upper surface side of the main body 221. The holding space 224 is formed by the bottom surface (inner surface, bottom surface portion) 224a, the outer peripheral surface (inner surface, outer side surface portion, first inclined portion) 224b, and the inner peripheral surface (inner surface) 224c.

As shown in Figs. 33A and 33B, the bottom surface (inner surface) 224a is a plane substantially parallel to the centrifugal direction of the centrifugal force G applied when the centrifugal container 220 spins, and forms the bottom of the holding space 224. The front of the bottom surface 224a is provided with the cell adhesion layer 27 that adsorbs and captures the target cells C1 as discussed above.

As shown in Figs. 33A and 33B, the outer peripheral surface (inner surface, outer side surface portion, first inclined portion) 224b is a substantially arc-shaped surface formed so as to incline downward from the outside to the inside in the radial direction with respect to the bottom surface 224a, and is connected to the bottom surface 224a at the end on the inside in the radial direction.

In this embodiment, the outer peripheral surface 224b is formed to be a curved inclined surface in the cross-sectional view shown in Fig. 33B. A cell adhesion layer 27 is provided to the front of the outer peripheral surface 224b.

Consequently, when the target cells C1 come into contact with the outer peripheral surface 224b after the test liquid is put into the holding space 224 and the target cells C1 sink in the test liquid, the target cells C1 can be adsorbed at that position.

Here, since the outer peripheral surface 224b is formed in a curved shape in cross-sectional view, when the centrifugal container 220 is rotated and centrifugal force is applied, the force F that attempts to move the target cells C1

adsorbed on the outer peripheral surface 224b radially outward is expressed by the following formula.

That is, as shown in Fig. 34, if we let r1 be the distance of the target cells C1 from the rotation axis 23 near the connection portion between the bottom surface 224a and the outer peripheral surface 224b, r2 be the distance of the target cells C1 from the rotation axis 23 halfway up the outer peripheral surface 224b, and r3 be the distance of the target cells C1 from the rotation axis 23 at the top of the outer peripheral surface 224b, the respective forces F1, F2, and F3 related to the target cells C1 are expressed by the following relational formulas 1 to 3.

$$F1 = r1\,\omega^2 \ldots (1)$$

$$F2 = r2\,\omega^2 \cos\theta 2 \ldots (2)$$

$$F3 = r3\,\omega^2 \cos\theta 3 \ldots (3)$$

Here, since the outer peripheral surface 224b is formed in a curved shape in cross-sectional view, $F1 \approx F2 \approx F3$.

If there are target cells C1 on the bottom surface, for example, the force when the target cells C1 are pulled away from the cell adhesion layer 27 varies with their position in the radial direction (the distance from the rotation axis). That is, the greater is the distance from the rotation axis, the greater is the force exerted on the target cells, and the greater is the variance.

Consequently, with the configuration of this embodiment, it is possible to reduce variance in the force at which the target cells C1 are pulled away from the cell adhesion layer 27 in the holding space 224 of the centrifugal container 20.

As shown in Figs. 33A and 33B, the inner peripheral surface (inner surface, second inclined portion) 224c is a cylindrical surface formed at an angle of approximately 90 degrees with respect to the bottom surface 224a, and is connected at its lower end to the bottom surface 224a.

Furthermore, as shown in Fig. 33B, the surface of the inner peripheral surface 224c is provided with a cell adhesion suppression layer 228 that suppresses the adsorption of the target cells C1 contained in the test liquid. Accordingly, even if the target cells C1 come into contact with the inner peripheral surface 224c after the test liquid is put in the holding space 224 and the target cells C1 sinks in the test liquid, the adsorption of the target cells C1 to the inner peripheral surface 224c can be suppressed.

Because of the above configuration, the centrifugal container 220 of this embodiment exhibits the same effect as that of the fourth embodiment described above.

## Seventh Embodiment

The centrifugal container 320 according to another embodiment of the present invention will now be described with reference to Figs. 35 to 36B.

The centrifugal container 320 in this embodiment differs from the centrifugal container 20 of the fourth embodiment in that an outer peripheral surface 324b is formed in a concentric arc shape rather than being eccentric with respect to the inner peripheral surface 324c, and that an inner peripheral surface 324c is formed as a cylindrical surface that is substantially perpendicular to the bottom surface 324a.

The rest of the components are substantially the same as those of the centrifugal container 20 of the fourth embodiment, so members having the same function and shape will be numbered the same and will not be described in detail again.

As shown in Fig. 35, the centrifugal container 320 of this embodiment comprises a main body 321 that is rotated around a rotation axis 23 by the centrifuge 11, and a holding space 324 that is formed in a substantially arc shape.

As shown in Fig. 35, the main body 321 is a substantially cylindrical member, and has the rotation axis 23 at its center.

The holding space 324 is a space for holding a test liquid containing the target cells C1 and the contaminants C2, and as shown in Fig. 35, is provided as a substantially arc-shaped recess at a position that is away from the rotation axis 23 in the radial direction, on the upper surface side of the main body 321. The holding space 324 is formed by a bottom surface (inner surface, bottom surface portion) 324a, an outer peripheral surface (inner surface, outer side surface portion, first inclined portion) 324b, and an inner peripheral surface (inner surface, second inclined portion) 324c.

As shown in Figs. 36A and 36B, the bottom surface (inner surface) 324a is a plane substantially parallel to the centrifugal direction of the centrifugal force G applied when the centrifugal container 320 spins, and forms the bottom of the holding space 324. The cell adhesion layer 27 that adsorbs and captures the target cells C1 is provided on the front of the bottom surface 324a.

As shown in Figs. 36A and 36B, the outer peripheral surface (inner surface, outer side surface portion, first inclined portion) 324b is a substantially arc-shaped surface formed so as to incline downward from the outside to the inside in the radial direction with respect to the bottom surface 324a, and is connected to the bottom surface 324a at the end on the inside in the radial direction.

In this embodiment, the outer peripheral surface 324b is provided with the cell adhesion layer 27 as shown in Fig. 36B.

Consequently, when the target cells C1 come into contact with the outer peripheral surface 324b after the test liquid is put into the holding space 324 and the target cells C1 sink in the test liquid, the target cells C1 can be adsorbed at that position.

Also, since the outer peripheral surface 324b inclines downward from the outside to the inside in the radial direction, in a state in which the centrifugal force G is applied, if we let r4 be the distance of the target cells C1 from the rotation axis near the connection portion between the bottom surface 324a and the outer peripheral surface 324b, and r5 be the distance of the target cells C1 from the rotation axis 23 on the outer peripheral surface 324b, the forces F4 and F5 related to the target cells C1 are expressed by the following relational formulas 4 and 5.

$$F4 = r4\,\omega^2 \ldots (4)$$

$$F5 = r5\,\omega^2 \cos\theta 5 \ldots (5)$$

Since the outer peripheral surface 324b is thus an inclined surface that inclines downward at an angle $\theta 5$ inward in the radial direction, the force that attempts to pull the target cells C1 near the connection portion between the bottom surface 324a and the outer peripheral surface 324b away from the cell adhesion layer 27, and the force that attempts to pull away the target cells C1 on the inclined surface of the outer peripheral surface 324b that intersects the bottom surface 324a at the angle $\theta 5$ are substantially the same ($F4 \approx F5$).

Because of the above configuration, the centrifugal container 320 of this embodiment exhibits the same effect as that of the fourth embodiment described above.

Eighth Embodiment

The centrifugal container 420 according to another embodiment of the present invention will now be described with reference to Figs. 37 to 38B.

That is, the centrifugal container 420 of this embodiment differs from the centrifugal container 20 of the fourth embodiment in that an outer peripheral surface 424b and an inner peripheral surface 424c are both connected at approximately 90 degrees, rather than being inclined with respect to the bottom surface 424a.

The rest of the components are substantially the same as those of the centrifugal container 20 of the fourth embodiment, so members having the same function and shape will be numbered the same and will not be described in detail again.

As shown in Fig. 37, the centrifugal container 420 of this embodiment comprises a main body 421 that is rotated around the rotation axis 23 by the centrifuge 11, and a holding space 424 that is formed in a substantially arc shape.

As shown in Fig. 37, the main body 421 is a substantially cylindrical member, and has a rotation axis 23 at its center.

The holding space 424 is a space for holding a test liquid containing the target cells C1 and the contaminants C2, and as shown in Fig. 37, is provided as a substantially arc-shaped recess at a position that is away from the rotation axis 23 in the radial direction, on the upper surface side of the main body 421. The holding space 424 is formed by a bottom surface (inner surface, bottom surface portion) 424a, an outer peripheral surface (inner surface, outer side surface portion) 424b, and an inner peripheral surface (inner surface) 424c.

As shown in Figs. 38A and 38B, the bottom surface (inner surface) 424a is a plane substantially parallel to the centrifugal direction of the centrifugal force G applied when the centrifugal container 320 spins, and forms the bottom of the holding space 424. The cell adhesion layer 27 that adsorbs and captures the target cells C1 is provided on the front of the bottom surface 424a.

As shown in Figs. 38A and 38B, the outer peripheral surface (inner surface, outer side surface portion) 424b is a substantially arc-shaped surface connected so as to intersect the bottom surface 424a at approximately 90 degrees, and is connected to the bottom surface 424a at the lower end.

In this embodiment, as shown in Fig. 38A, the inner peripheral surface 424c is disposed in an arc shape centered on the rotation axis 23, while the outer peripheral surface 424b is disposed in an eccentric shape with respect to the inner peripheral surface 424c.

Because of the above configuration, the centrifugal container 420 of this embodiment exhibits the same effect as that of the fourth embodiment described above.

Ninth Embodiment

The centrifugal container 520 according to another embodiment of the present invention will now be described with reference to Figs. 39 to 40B.

The centrifugal container 520 of this embodiment differs from the centrifugal container 20 of the fourth embodiment in that an outer peripheral surface 524b is formed in a concentric arc shape, rather than being eccentric with respect to an inner peripheral surface 524c, and that the inner peripheral surface 524c is formed as a cylindrical surface that is substantially perpendicular to a bottom surface 524a, and that a plurality of discharge ports 525 are provided on the outermost peripheral portion of the outer peripheral surface 524b.

The rest of the components are substantially the same as those of the centrifugal container 20 of the fourth embodiment, so members having the same function and shape will be numbered the same and will not be described in detail again.

As shown in Fig. 39, the centrifugal container 520 of this embodiment comprises a main body 521 that is rotated around the rotation axis 23 by the centrifuge 11, a holding space 524 that is formed in a substantially arc shape, and the plurality of discharge ports 525.

As shown in Fig. 39, the main body 521 is a substantially cylindrical member, and has a rotation axis 23 at its center.

The holding space 524 is a space for holding a test liquid containing the target cells C1 and the contaminants C2, and as shown in Fig. 39, is provided as a substantially arc-shaped recess at a position that is away from the rotation axis 23 in the radial direction, on the upper surface side of the main body 521. The holding space 424 is formed by the bottom surface (inner surface, bottom surface portion) 524a, the outer peripheral surface (inner surface, outer side surface portion) 524b, and the inner peripheral surface (inner surface) 524c.

As shown in Figs. 40A and 40B, the bottom surface (inner surface) 524a is a plane substantially parallel to the centrifugal direction of the centrifugal force G applied when the centrifugal container 520 spins, and forms the bottom of the holding space 524. The cell adhesion layer 27 that adsorbs and captures the target cells C1 is provided on the front of the bottom surface 524a.

As shown in Figs. 40A and 40B, the outer peripheral surface (inner surface, outer side surface portion, first inclined portion) 524b is a substantially arc-shaped surface formed so as to incline downward from the outside to the inside in the radial direction with respect to the bottom surface 524a, and is connected to the bottom surface 524a at the end on the inside in the radial direction.

As shown in Fig. 40A, four discharge ports 525 are provided on the outermost peripheral side of the outer peripheral surface 524b. Consequently, even though the outer peripheral surface 524b does not have an eccentric shape as in the fourth embodiment, the contaminants C2 that have moved to the outermost peripheral side under the application of the centrifugal force G can be moved through the four discharge ports 525 to collection chamber 26.

Because of the above configuration, the centrifugal container 520 of this embodiment exhibits the same effect as that of the fourth embodiment described above.

Other Embodiments

Embodiments of the present invention were described above, but the present invention is not limited to the above embodiments, and various modifications are possible without departing from the gist of the invention.

(A)
In the fourth embodiment above, an example was given in which the cell adhesion layer 27 was provided on the bottom surface 24a, and the cell adhesion suppression layers 28a and 28b were provided on the inner surface (the outer peripheral surface 24b and the inner peripheral surface 24c) other than the bottom surface 24a. However, the present invention is not limited to this.

For example, as shown in Fig. 41A, the centrifugal container 620 may be such that the cell adhesion layer 27 is provided to only the bottom surface 624a, and neither the cell adhesion layer nor the cell adhesion suppression layer is provided to the inner surface other than the bottom surface 624a (the outer peripheral surface 624b and the inner peripheral surface 624c).

Also, as shown in Fig. 41A, the configuration may be such that the outer peripheral surface 624b and the inner peripheral surface 624c are both arc-shaped surfaces that intersect the bottom surface 624a at approximately 90 degrees, that is, are not inclined surfaces.

Here again, because of the above configuration, the centrifugal container 620 of this embodiment exhibits the same effect as that of the fourth embodiment described above, which is that the target cells C1 can be adsorbed on the bottom surface 624a and can be easily taken out.

(B) In the above embodiment, an example of the centrifugal container 20 was given in which the cell adhesion layer 27 was provided on the bottom surface 24a, and the cell adhesion suppression layers 28a and 28b were provided on inclined surfaces (the outer peripheral surface 24b and the inner peripheral surface 24c) other than the bottom surface 24a. However, the present invention is not limited to this.

For example, as shown in Fig. 41B, a centrifugal container 720 may be such that the cell adhesion suppression layers 28a and 28b are provided on inner surfaces (outer peripheral surface 724b, inner peripheral surface 724c) that intersect substantially perpendicularly to a bottom surface 724a.

Here again, because of the above configuration, the centrifugal container 720 of this embodiment exhibits the same effect as that of the fourth embodiment described above, which is that the target cells C1 can be adsorbed on the bottom surface 724a and easily taken out.

(C) In the fourth embodiment, an example was given in which the outermost peripheral portion of the outer peripheral surface 24b was provided with one discharge port 25 for discharging, to the outside of the holding space 24, contaminants C2 other than the target cells C1 contained in the test liquid. However, the present invention is not limited to this.

For example, the number of discharge ports is not limited to one, and four discharge ports may be provided as in the fifth and ninth embodiments described above.

Also, the number of discharge ports is not limited to four, and two, three, or five or more discharge ports may be provided.

(D) In the fourth, fifth, and ninth embodiments above, an example was given in which the discharge ports 25, 125, and 525 were substantially circular in plan view. However, the present invention is not limited to this.

For example, the shape of the discharge port may be something other than circular, such as a through-hole formed in an arc shape along the outermost periphery of the outer peripheral surface.

(E) In the above embodiment, an example was given in which the centrifugal container 20, etc., having a substantially cylindrical shape was used. However, the present invention is not limited to this.

For example, as shown in Fig. 42, a cell capture apparatus 810 may be used that includes a centrifuge 811 equipped with a mounting portion 814 to which two fan-shaped centrifugal containers 820 can be mounted.

In this case, as shown in Fig. 43, the centrifugal container 820 comprises a substantially fan-shaped main body 821 and a substantially fan-shaped cover 822. The centrifugal container 820 is rotationally driven by the centrifuge 811 around a virtual rotary shaft 823 indicated by the one-dot chain line in the drawing.

As shown in Figs. 43, 44A, and 44B, the centrifugal container 820 has a substantially fan-shaped holding space 824. Just as with the holding space 24 described above, the holding space 824 is filled with a test liquid containing target cells, and is formed by a bottom surface (inner surface, bottom surface portion) 824a, an outer peripheral surface (inner surface, outer side surface portion) 824b, and an inner peripheral surface (inner surface) 824c.

The bottom surface 824a is provided with the cell adhesion layer 827 that adsorbs target cells, as is the bottom surface 24a and the like. A cell adhesion suppression layer 828 is provided on the inner surface (the outer peripheral surface 824b, the inner peripheral surface 824c, and the top surface) other than the bottom surface 824a.

Consequently, this configuration exhibits the same effect as in the above embodiments.

## Tenth Embodiment

The centrifugal container according to an embodiment of the present invention will now be described with reference to Figs. 45 to 59.

In this embodiment, unnecessarily detailed description may be omitted. For example, detailed description of already known facts or redundant description of components that are substantially the same may be omitted. This is to avoid unnecessary repetition in the following description, and facilitate an understanding on the part of a person skilled in the art.

The applicant has provided the appended drawings and the following description so that a person skilled in the art might fully understand this disclosure, but does not intend for these to limit what is discussed in the patent claims.

### (1) Configuration of Cell Capture Apparatus 950

The cell capture apparatus 950 according to this embodiment applies centrifugal force by spinning the centrifuge 930 in which the centrifugal container 910 has been placed, and captures the target cells C1 contained a test liquid S, such as blood or lymphatic fluid, poured into the centrifugal container 910. As shown in Fig. 45, this apparatus comprises a centrifugal container 910, a centrifuge 930, and a control unit 951 for controlling the rotation of the centrifuge 930 (see

Fig. 50A, etc.).

Here, the target cells C1 to be captured by the cell capture apparatus 950 are, for example, at least one type selected from the group consisting of circulating tumor cells, stem cells, vascular endothelial cells, nerve cells, dendritic cells, smooth muscle cells, fibroblasts, cardiomyocytes, skeletal muscle cells, liver parenchymal cells, liver non-parenchymal cells, and pancreatic islet cells, contained in intravascular fluid (test liquid) such as blood or lymphatic fluid.

The centrifugal container 910 is held by a container holder 934 of the centrifuge 930 as shown in Figs. 45 and 46 in a state in which the test liquid S containing the target cells C1 has been poured. The centrifugal container 910 is rotationally driven around a rotary shaft 933c in a state of being held by the container holder 934 of the centrifuge 930, which applies centrifugal force to the centrifugal container 910 toward the outside in the radial direction of a circle centered on the rotary shaft 933c.

The detailed configuration of the centrifugal container 910 will be described below.

The centrifuge 930 is, for example, a general centrifugal separation apparatus used fields such as medicine, life sciences, and biotechnology, and is rotated in a state in which the centrifugal container 910 has been mounted, which applies centrifugal force to the test liquid S containing the target cells C1 in the centrifugal container 910. As shown in Figs. 45 and 46, the centrifuge 930 comprises a base 931, a motor 932, a rotor 933, and a container holder 934.

As shown in Figs. 45 and 46, the base 931 is a plate-like member that constitutes the foundation of the centrifuge 930, and supports the motor 932, the rotor 933, etc., on its upper surface.

As shown in Fig. 45, the motor 932 is provided on the top side of the base 931 as a drive source that rotationally drives the rotor 933 around the rotary shaft 933c, with the rotor 933 supported in a state in which the container holder 934 holding the centrifugal container 910 is able to pivot. The motor 932 is controlled by a control unit 951 (see Fig. 50A, etc.) to start or stop rotation, adjust the rotation speed, and so forth.

The rotor 933 is a flat rotating body that is rotationally driven by the motor 932, and as shown in Fig. 46, has swing bearings 933a, a stopper pin 933b, and the rotary shaft 933c.

As shown in Figs. 45 and 46, the swing bearings 933a are grooves provided at both ends in the lengthwise direction of the flat rotor 933, and hold, in a latched state, two swing shafts 934b provided so as to protrude from the side surfaces on both sides of the container holder 934 (discussed below). At this point, the container holder 934 is held in a state of being able to pivot with respect to the rotor 933 around the swing shafts 934b.

The stopper pin 933b is provided near the swing bearings 933a of the rotor 933, as shown in Figs. 45 and 46, and is provided so as to protrude toward the side surface of the container holder 934, having an elastic force produced by a spring (not shown). The stopper pin 933b is held in a state in which its distal end is inserted into a stopper hole 934c formed in the side surface of the container holder 934, which prevents the container holder 934 from pivoting with respect to the rotor 933.

At this point, the container holder 934 is held vertically along the substantially vertical direction with the swing shaft 934b as the center. When the centrifugal force exceeds a specific level due to the rotation of the motor 932 (discussed below), the distal end of the stopper pin 933b comes out of the stopper hole 934c, so that the container holder 934 pivots around the swing shafts 934b due to the centrifugal force, changing the orientation to substantially horizontal (see Figs. 50A and 50B).

Consequently, the orientation of the centrifugal container 910 held by the container holder 934 changes from vertical to horizontal along with the container holder 934 (see Figs. 50A and 50B).

The rotary shafts 933c are provided near the center of the flat rotor 933, and the lower ends thereof are coupled to the motor 932 so that the rotational drive force of the motor 932 is transmitted to provide rotational drive along with the rotor 933.

The container holder 934 is a member that holds the centrifugal container 910 into which the test liquid S is poured, and as shown in Fig. 45, is placed from above so that the swing shafts 934b are held with respect to the swing bearings 933a of the rotor 933. As shown in Figs. 45 and 46, the container holder 934 has a holding portion 934a, swing shafts 934b, and a stopper hole 934c.

The holding portion 934a is a recess formed in the container holder 934 to match the shape of the centrifugal container 910, and holds the centrifugal container 910. Various methods can be used for holding the centrifugal container 910 in the holding portion 934a, such as mechanical latching, fitting, and adhesive bonding.

As shown in Figs. 45 and 46, the swing shafts 934b are provided so as to protrude from both side surfaces of the container holder 934 and are latched in the swing bearings 933a provided to the rotor 933.

As shown in Figs. 45 and 46, the stopper hole 934c is a recess formed near the swing shaft 934b on one side surface of the container holder 934, into which the distal end of the stopper pin 933b provided to the rotor 933 is inserted. When the centrifugal force applied to the container holder 934 holding the centrifugal container 910 exceeds a specific level due to the rotation of the motor 932, the engagement of the stopper pin 933b and the stopper hole 934c is released by the elastic force of the spring (not shown), and the container holder 934 is able to pivot around the swing shafts 934b.

That is, the latched state between the stopper pin 933b and the stopper hole 934c is formed in order to hold the centrifugal container 910 (container holder 934) substantially vertically. When centrifugal force of at least a specific

magnitude is applied, the stopper pin 933b is unlatched from the stopper hole 934c, and the orientation of the centrifugal container 910 can be changed by the centrifugal force so that the centrifugal container 910 (container holder 934) is disposed substantially horizontally.

Here, the principle of capturing the target cells C1 contained in the test liquid S by using the centrifugal container 910 in this embodiment will be described with reference to Fig. 47.

With the centrifugal container 910, a cell adhesion layer 914 that adhesively captures the target cells C1 contained in the test liquid S is formed on an inner surface 910b in a holding space (capture chamber 912) in which the test liquid S (discussed below) is held (see Fig. 48, etc.).

The cell adhesion layer 914 is a layer that adsorbs the target cells C1 contained in the test liquid S, but does not adsorb contaminants C2 other than the target cells C1, and contains a first biocompatible polymeric material.

The first biocompatible polymeric material includes one or more of the polymers expressed by the following formulas 1 to 4, or a copolymer of a monomer constituting the polymer expressed by formula 1 and a monomer constituting one or more types of polymer selected from the group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

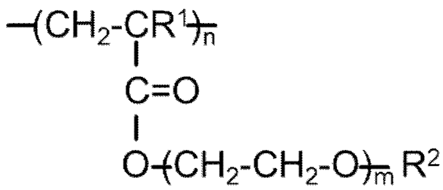

(1)

In formula 1, $R^1$ is a hydrogen atom or a methyl group, $R^2$ is a methyl group or an ethyl group, m is an integer of 1, 2, or 3, and n is a repeating unit.

Chemical Formula 2

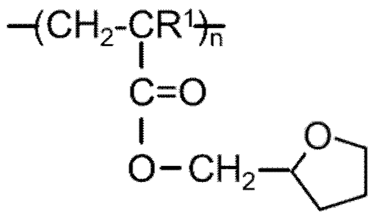

(2)

In formula 2, $R^1$ is a hydrogen atom or a methyl group, and n is a repeating unit.

Chemical Formula 3

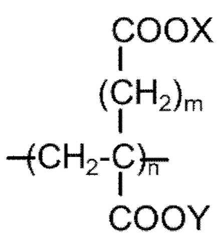

(3)

In formula 3, X and Y are each independently a hydrogen atom, an alkali metal atom, or an ammonium group, m is an integer of 0, 1, or 2, and n is a repeating unit.

Chemical Formula 4

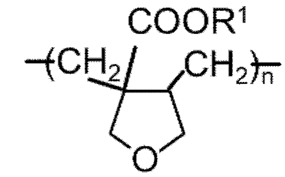

(4)

In formula 4, $R^1$ is a hydrogen atom or an organic group having 1 to 30 carbon atoms, and n is a repeating unit.

Fig. 47 is a schematic diagram illustrating the hydration structures of the target cell C1 and the biocompatible polymeric material 2.

The biocompatible polymeric material 2 refers to a first biocompatible polymeric material and a second biocompatible polymeric material.

The target cell C1 is surrounded by the hydration shell 1 as shown in Fig. 47, in the presence of a large amount of water, such as an intravascular fluid (test liquid).

The hydration shell 1 is made up of water molecules in various states. More specifically, the hydration shell 1 includes nonfreezing water 1a that interacts strongly with the target cell C1, free water 1c that interacts weakly with the target cell C1, and intermediate water 1b that interacts with the target cell C1 with an intermediate strength in between that of the nonfreezing water 1a and the free water 1c.

Similarly, in a hydrated state, a hydration structure 3 is formed on the surface of the biocompatible polymeric material 2 included in the cell adhesion layer 914.

The hydration structure 3 includes nonfreezing water 3a that is bound by strong interaction with the biocompatible polymeric material 2, free water 3c that interacts weakly with the target cell C1, and intermediate water 3b that interacts with the target cell C1 with an intermediate strength in between that of the nonfreezing water 3a and the free water 3c.

In Fig. 47, for the sake of explanation, the nonfreezing water 1a and 3a, the intermediate water 1b and 3b, and the free water 1c and 3c are shown as layers disposed in that order, starting from the target cell C1 and the biocompatible polymeric material 2, and the actual structure is not accurately reflected.

The nonfreezing water 1a, 3a is located close to atoms that can form hydrogen bonds, such as oxygen and nitrogen in the molecules constituting the target cell C1 and the biocompatible polymeric material 2.

The nonfreezing water 1a, 3a does not freeze (solidify) even at -150° C or below because it interacts strongly with the target cell C1 or the biocompatible polymeric material 2.

The free water 1c, 3c freezes or melts at 0° C.

The intermediate water 1b, 3b freezes or melts at a temperature below 0° C, such as at several tens of degrees below zero (Celsius). When water freezes or melts, heat is released or absorbed, so the proportion of water in these different states in the hydration shell 1 and hydration structure 3 can be found by using a differential scanning calorimeter to measure the amounts of heat released and absorbed, for example.

In general, a layer of intermediate water 3b and/or free water 3c is formed on the surface of biocompatible polymeric material 2 in a hydrated state. Therefore, when a target cell C1 surrounded by the hydration shell 1 approaches the surface of the biocompatible polymeric material 2, the hydration shell 1 of the target cell C1 is in contact with the intermediate water 3b or the free water 3c and interacts weakly. Since this interaction is not enough to pull out the water molecules of the hydration shell 1, there is no major change to the structure of the hydration shell 1, and the structure of the target cell C1 is also maintained, which is believed to result in biocompatibility, or in other words, the target cell C1 exhibits no foreign body reaction or rejection reaction.

On the other hand, with a bioincompatible molecular material, it is possible that almost no layers of intermediate water 1b, 3b and free water 1c, 3c will be formed. Therefore, when the target cell C1 surrounded by the hydration shell 1 approaches the surface of the bioincompatible polymeric material, the water molecules of the hydration shell 1 of the target cell C1 may interact strongly with the bioincompatible polymeric material itself or with the nonfreezing water captured on the surface of the bioincompatible polymeric material, causing the water molecules to be desorbed from the hydration shell 1, or the structure of the hydration shell 1 may be greatly changed, resulting in changes in the three-dimensional structure of the target cell, damage, etc., with the upshot being that the cell ceases to function.

As described above, since the first biocompatible polymeric material includes the intermediate water 3b in a hydrated state, the target cells C1 in the test liquid S can be selectively adsorbed and capture.

More specifically, the biocompatible polymeric material containing the intermediate water 3b can selectively capture the target cells C1, without capturing the contaminants C2, such as blood cell components and protein components in the blood serving as the test liquid S.

In this embodiment, the centrifugal container 910, into which the test liquid S containing the target cells C1 has been poured, is rotated around the rotary shaft 933c in a state of being mounted to the centrifuge 930. Consequently,

the centrifugal force G is applied to the test liquid S in the centrifugal container 910 and to the target cells C1 and contaminants C2 in the test liquid S.

At this point, in the step of capturing the target cells C1, the cell adhesion layer 914 containing the first biocompatible polymeric material is disposed along a plane substantially perpendicular to the centrifugal direction in which the centrifugal force is applied, and in the rinsing step of removing the contaminants C2 while leaving only the target cells C1 captured in the cell adhesion layer 914, the cell adhesion layer 914 is disposed along a plane substantially parallel to the centrifugal direction.

That is, in this embodiment, the centrifugal container 910 is configured such that its orientation changes between the capture step of capturing the target cells C1 and the rinsing step of removing contaminants C2 other than the captured target cells C1.

Consequently, the target cells C1 adhered to the cell adhesion layer 914 are held by the cell adhesion layer 914 even when centrifugal force is applied by rotation by the centrifuge 930, and the contaminants C2 other than the target cells C1 are subjected to the centrifugal force and move radially outward around the rotary shaft 933c.

As a result, when the centrifugal container 910 is rotated around the rotary shaft 933c, the contaminants C2 other than the target cells C1 contained in the test liquid S are moved radially outward by the centrifugal force, and the target cells C1 can be held by the cell adhesion layer 914, allowing the target cells C1 to be selectively captured.

(2) Configuration of Centrifugal Container 910

As shown in Fig. 48, the centrifugal container 910 according to this embodiment comprises a main body 910a and an inner surface 910b that forms a holding space for the test liquid S inside the main body 910a. More precisely, the centrifugal container 910 comprises a pouring chamber 911, a capture chamber 912, a channel (capillary channel) 913, a cell adhesion layer 914, an air hole 915, an overflow chamber 916, and an overflow channel 917.

As shown in Figs. 49A, 49B, and 49C, the pouring chamber 911 is provided in a space formed inside the main body 910a of the centrifugal container 910, and the test liquid S is poured through a pouring port 911a. The pouring chamber 911 communicates with the capture chamber 912 via the channel 913 as shown in Fig. 49B. Also, the pouring chamber 911 is disposed above the capture chamber 912 in side view, as shown in Figs. 49B and 49C, as a space formed in the main body 910a.

The capture chamber 912 holds the test liquid S that has moved through the channel 913 under centrifugal force in a state in which the test liquid S has been put into the pouring chamber 911. As shown in Figs. 49B and 49C, the capture chamber 912 is disposed below the pouring chamber 911 in side view as a space formed within the main body 910a, and is connected to the channel 913 at its upper portion. Furthermore, as shown in Fig. 49C, the capture chamber 912 is provided with an air hole 915 that releases the air inside the capture chamber 912 to the outside during filling with the test liquid S that has moved from the pouring chamber 911 through the channel 913 under the application of centrifugal force.

Here, the capture chamber 912 is provided at a position that is away from the center of rotation (rotary shaft 933c) in a vertical orientation (see Fig. 50A) when centrifugal force is applied to move the test liquid S from the pouring chamber 911.

As shown in Figs. 49A and 49B, the channel 913 is provided to allow the pouring chamber 911 and the capture chamber 912 to communicate with each other, and holds the test liquid S by capillary force. When a centrifugal force exceeding the capillary force of the chamber 912 is applied to the test liquid S, the test liquid S is moved from the pouring chamber 911 to the capture chamber 912.

As shown in Figs. 49B and 49C, the cell adhesion layer 914 is provided on the inner surface (bottom surface in the drawings) of the capture chamber 912, and as discussed above, adsorbs the target cells C1 contained in the test liquid S, and does not adsorb the contaminants C2.

Here, the surface of the capture chamber 912 on which the cell adhesion layer 914 is provided is disposed substantially vertically in the state shown in Fig. 50A. Then, when the centrifugal force rises over a specific level due to the rotation of the centrifuge 930, the surface on which the cell adhesion layer 914 is provided is disposed substantially horizontally as shown in Fig. 50B.

That is, in this embodiment, when centrifugal force is applied to the cell adhesion layer 914 by the rotation of the centrifuge 930, the container holder 934, which is able to pivot with respect to the rotor 933, pivots together with the centrifugal container 910, and its orientation (the orientation of the centrifugal container 910) changes from being substantially vertical to being substantially horizontal.

As shown in Figs. 49A and 49C, the air hole 915 is a through-hole that allows the capture chamber 912 to communicate with the outside of the centrifugal container 910, and is provided at a position closer to the side surface on the opposite side from the side surface where the channel 913 is provided in the capture chamber 912.

Consequently, when the test liquid S moves from the pouring chamber 911 to the capture chamber 912, the air inside the capture chamber 912 is released through the air hole 915 to the outside, which allows the test liquid S to

move smoothly to the capture chamber 912.

The overflow chamber 916 is provided to hold the test liquid S that has moved from the pouring chamber 911 to the capture chamber 912 and overflowed from the capture chamber 912. As shown in Figs. 49A and 49C, the overflow chamber 916 is located adjacent to the capture chamber 912 in the centrifugal container 910, and is provided between the pouring chamber 911 and the capture chamber 912.

Consequently, the test liquid S can be held in the capture chamber 912 until the capture chamber 912 is filled with the test liquid S. This allows a large amount of target cells C1 to be efficiently and selectively adsorbed in the cell adhesion layer 914 provided in the capture chamber 912.

As shown in Figs. 49A and 49C, the overflow channel 917 is a channel that allows the capture chamber 912 to communicate with the overflow chamber 916, and moves the test liquid S overflowing from the capture chamber 912 to move to the overflow chamber 916. Capturing Target Cells C1 using Centrifugal Container 910 and Removing Contaminants C2

As discussed above, with the cell capture apparatus 950 in this embodiment, when the centrifugal container 910 is placed in the centrifuge 930 and rotated, and the centrifugal force reaches at least a specific level, the orientation of the centrifugal container 910 changes, and as a result the target cells C1 contained in the test liquid S poured into the centrifugal container 910 are selectively captured.

More specifically, as shown in Fig. 50A, with the cell capture apparatus 950, in a state in which the centrifugal container 910 has been placed in the container holder 934 of the centrifuge 930, the centrifugal container 910 (the cell adhesion layer 914 provided to the inner surface 910b of the capture chamber 912) is disposed substantially along the vertical direction.

Then, when the centrifuge 930 is rotated and the centrifugal force applied to the centrifugal container 910 reaches at least a specific level, the distal end of the stopper pin 933b that has been restricting the pivoting of the container holder 934 comes out of the stopper hole 934c as shown in Fig. 50B, the container holder 934 pivots around the swing shafts 934b, and the centrifugal container 910 (the cell adhesion layer 914 provided on the inner surface 910b of the capture chamber 912) is disposed substantially horizontally.

That is, with the cell capture apparatus 950 of this embodiment, the control unit 951 controls the rotation of the motor 932 of the centrifuge 930 so that the target cells C1 contained in the test liquid S poured into the centrifugal container 910 are separated from the contaminants C2, and the target cells C1 are selectively captured.

Here, the steps up to the pouring of the test liquid S into the centrifugal container 910, the liquid transfer from the pouring chamber 911 to the capture chamber 912, the capture of the target cells C1 in the cell adhesion layer 914, the orientation change of the centrifugal container 910, and the removal of the contaminants C2 will now be described using Figs. 51A to 55.

Figs. 51A to 54 show only the centrifugal container 910 for the sake of explanation, but in reality, let us assume that the centrifugal container 910 is in a state in which it can be rotated, or is being rotated, by the centrifuge 930 while located in the container holder 934.

First, when the test liquid S is poured from the pouring port 911a of the centrifugal container 910, the pouring chamber 911 is filled with the test liquid S as shown in Fig. 51A. At this point, the channel 913 that allows the pouring chamber 911 to communicate with the capture chamber 912 holds the test liquid S by capillary action, so that the test liquid S is retained in the pouring chamber 911.

The pouring of the test liquid S into the centrifugal container 910 may be performed with the centrifugal container 910 in place in the container holder 934, or may be performed with the centrifugal container 910 removed from the container holder 934.

Next, with the cell capture apparatus 950, when the control unit 951 rotates the centrifuge 930 to apply centrifugal force to the centrifugal container 910, the test liquid S that was being held in the channel 913 is sent to the capture chamber 912 side by centrifugal force, as shown in Fig. 51B.

At this point, the centrifugal force applied to the centrifugal container 910 is not strong enough to remove the stopper pin 933b from the stopper hole 934c and unlatch the container holder 934, so the centrifugal container 910 rotates while still oriented substantially in the vertical direction, and the liquid is transferred from the pouring chamber 911 to the capture chamber 912.

At this point, as shown in Fig. 51B, the control unit 951 of the cell capture apparatus 950 rotates the motor 932 (centrifugal container 910) at a rotation speed V1 (first speed) (see Fig. 55) (liquid transfer step).

Next, with the cell capture apparatus 950, the control unit 951 rotates the centrifuge 930 at the rotation speed V1, and as shown in Fig. 52A, once the transfer of the liquid S from the pouring chamber 911 of the centrifugal container 910 to the capture chamber 912 is complete, the capture chamber 912 is filled with the test liquid S.

Here, when the capture chamber 912 is filled with the test liquid S, the air inside the capture chamber 912 is released through the air hole 915 to the outside, and any test liquid S overflowing from the capture chamber 912 moves through the overflow channel 917 to the overflow chamber 916 and is stored there.

Next, when the control unit 951 raises the centrifuge 930 to the rotation speed V2 (second speed) (see Fig.

55), as shown in Fig. 52B, the target cells C1 and contaminants C2 contained in the test liquid S in the capture chamber 912 are subjected to centrifugal force and pressed against the cell adhesion layer 914 provided on the inner surface 910b of the capture chamber 912 (capture step).

This allows the cell adhesion layer 914 to capture the target cells C1 effectively.

Next, when the control unit 951 increases the centrifuge 930 to the rotation speed V3 (third speed) (see Fig. 55), as shown in Fig. 53A, a centrifugal force of at least a specific level is reached, which removes the distal end of the stopper pin 933b that latches the container holder 934 from the stopper hole 934c, and the centrifugal container 910 pivots around the swing shafts 934b and changes to a substantially horizontal orientation (orientation change step).

This allows the container holder 934 to become able to pivot around the swing shafts 934b, and then the container holder 934 pivots under the centrifugal force, and as shown in Fig. 53B, the centrifugal container 910 changes from being oriented substantially vertically to being oriented substantially horizontally.

At this point, the target cells C1, etc., adsorbed to the cell adhesion layer 914 are still held within the capture chamber 912.

Next, when the control unit 951 raises the centrifuge 930 to the rotation speed V4 (fourth speed) (see Fig. 55), as shown in Fig. 54, the centrifugal container 910 is still oriented substantially horizontally, and the target cells C1 and contaminants C2 contained in the test liquid S in the capture chamber 912 are subjected to centrifugal force.

At this point, the target cells C1 that were adsorbed to the cell adhesion layer 914 are still held on the cell adhesion layer 914 because the adsorption force is greater than the applied centrifugal force. On the other hand, the contaminants C2 contained in the test liquid S have almost no adsorptive strength with respect to the cell adhesion layer 914, and therefore move outward in the centrifugal direction due to the centrifugal force, as shown in Fig. 54.

Consequently, only the target cells C1 are selectively captured on the cell adhesion layer 914 in the capture chamber 912, and the contaminants C2 are removed from the cell adhesion layer 914 by moving outward in the centrifugal direction (rinsing step).

With the cell capture apparatus 950 of this embodiment, as described above, the control unit 951 increases the rotation speed of the motor 932 of the centrifuge 930 in steps from V1 to V2, V3, and V4 as shown in Fig. 55 to change the amount of centrifugal force that is applied to the centrifugal container 910.

Consequently, the steps of pouring the test liquid S into the centrifugal container 910, transferring liquid from the pouring chamber 911 to the capture chamber 912, capturing the target cells C1 in the cell adhesion layer 914, changing the orientation of the centrifugal container 910, and removing the contaminants C2 can be performed with the centrifugal container 910 in the optimal orientation.

The movement of the target cells C1 and the contaminants C2 contained in the test liquid S as the liquid is transferred into the capture chamber 912 will now be described in greater detail through reference to Figs. 56A to 58B.

The target cells C1 and the contaminants C2 contained in the test liquid S sent from the pouring chamber 911 are floating around in the capture chamber 912 as shown in Fig. 56A.

Then, when the control unit 951 rotates the motor 932 to rotate the centrifugal container 910 at the rotation speed V2, the target cells C1 and the contaminants C2 move outward in the centrifugal direction inside the capture chamber 912 as shown in Fig. 56B.

At this point, the cell adhesion layer 914 is provided at a position facing outward in the centrifugal direction of the capture chamber 912. Therefore, the target cells C1 and contaminants C2 subjected to centrifugal force are pressed against the surface of the cell adhesion layer 914.

Next, when the control unit 951 increases the rotation speed of the motor 932 to the rotation speed V3, as shown in Fig. 57A, the centrifugal force changes the orientation of the container 910 from being substantially vertical to being substantially horizontal, while the target cells C1 are still adsorbed to the cell adhesion layer 914.

Next, when the control unit 951 increases the rotation speed of the motor 932 to the rotation speed V4, as shown in Fig. 57B, only the target cells C1 adsorbed to the cell adhesion layer 914 remain on the cell adhesion layer 914, while the contaminants C2 move outward in the centrifugal direction under the centrifugal force.

Consequently, the contaminants C2 can be removed from the cell adhesion layer 914 in the capture chamber 912, but not the target cells C1.

Here, if air is mixed into the test liquid S transferred into the capture chamber 912, as shown in Fig. 58A, in the state up to the rinsing step, air A1 will be present somewhere in the test liquid S.

When the rinsing step is performed from this state, as shown in Fig. 58B, the air A1, which has a lower specific gravity than the test liquid S, moves inward in the centrifugal direction on the opposite side from the contaminants C2. At this point, as the air A1 moves, there is the risk that the target cells C1 adsorbed to the cell adhesion layer 914 will be separated from the cell adhesion layer 914 due to contact with the air A1.

If this should happen, it may be difficult to selectively capture the target cells C1 in the cell adhesion layer 914.

In view of this, the centrifugal container 910 of this embodiment comprises the above-mentioned air hole 915 in order to make it less likely that the air A1 will be mixed into the test liquid S transferred to the capture chamber 912.

Consequently, when the test liquid S is transferred from the pouring chamber 911 to the capture chamber 912,

the air inside the capture chamber 912 is released to the outside through the air hole 915 as the test liquid S fills the chamber. This allows mixing of the air A1 into the test liquid S to be effectively suppressed.

Cell Capture Method

With the cell capture apparatus 950 of this embodiment, because of the above configuration, the cell capture method is executed according to the flowchart shown in Fig. 59.

That is, in step S11, the test liquid S containing the target cells C1 is poured into the pouring chamber 911 of the centrifugal container 910.

Next, in step S12, in a state in which the centrifugal container 910 has been placed in the container holder 934 of the centrifuge 930, the control unit 951 increases the rotation speed of the motor 932 to V1.

Next, in step S13, once the rotation speed V1 is reached, the test liquid S is transferred from the pouring chamber 911 of the centrifugal container 910 to the capture chamber 912 (liquid transfer step).

Next, in step S14, the control unit 951 determines whether or not a specific length of time has elapsed since the start of rotation at the rotation speed V1, and if the determination is affirmative, the processing proceeds to step S15.

Next, in step S15, the control unit 951 increases the rotation speed of the motor 932 to V2.

Next, in step S16, once the rotation speed reaches V2, the target cells C1 and contaminants C2 contained in the test liquid S in the capture chamber 912 of the centrifugal container 910 are pressed against the cell adhesion layer 914 by centrifugal force (capture step).

Next, in step S17, it is determined whether or not a specific length of time has elapsed since the rotation at the rotation speed V2 was started, and if the determination is affirmative, the processing proceeds to step S18.

Next, in step S18, the control unit 951 increases the rotation speed of the motor 932 to V3.

Next, in step S19, once the rotation speed reaches V3 and the centrifugal force is at least a specific level, the distal end of the stopper pin 933b that is latching the container holder 934 comes out of the stopper hole 934c, and the centrifugal container 910 changes from being oriented substantially vertically to being oriented substantially horizontally (orientation change step).

Next, in step S20, the control unit 951 increases the rotation speed of the motor 932 to V4.

Next, in step S21, once the rotation speed reaches V4, the target cells C1 adsorbed to the cell adhesion layer 914 are still held on the cell adhesion layer 914, while the contaminants C2 are moved outward in the centrifugal direction by centrifugal force (rinsing step).

Consequently, just the target cells C1 that are supposed to be captured can be selectively adsorbed onto the cell adhesion layer 914, so the target cells C1 can be easily taken out.

Next, in step S22, the control unit 951 determines whether or not a specific length of time has elapsed since the start of rotation at the rotation speed V4, and when if the determination is affirmative, the processing proceeds to step S23.

Next, in step S23, it is determined that the rinsing step has ended, and the control unit 951 controls the motor 932 to stop the rotation of the centrifuge 930.

Next, in step S24, when the centrifuge 930 stops spinning, the target cells C1 are separated from the cell adhesion layer 914 and taken out, and the processing is concluded.

Eleventh Embodiment

The centrifugal container 1010 according to another embodiment of the present invention will now be described with reference to Figs. 60 to 61C.

Of the members appearing in this embodiment, those having the same function, shape, etc., as those of the tenth embodiment will be numbered the same and will not be described again in detail.

As shown in Fig. 60, the centrifugal container 1010 of this embodiment differs from the centrifugal container 910 described in the tenth embodiment in having a simpler configuration that does not have the overflow chamber 916 or the overflow channel 917, but otherwise the configurations are the same.

Just as with the centrifugal container 910 of the tenth embodiment, the centrifugal container 1010 comprises the pouring chamber 911, the capture chamber 912, the channel 913, the cell adhesion layers 914, and the air hole 915, as shown in Figs. 61A, 61B, and 61C.

As a result, when the rotation speed is changed in a state in which in the centrifugal container 910 has been placed in the centrifuge 930, thereby changing the amount of centrifugal force applied, the target cells C1 can be selectively adsorbed to the cell adhesion layer 914 and efficiently removed from the test liquid S, just as in the tenth embodiment above.

Since the centrifugal container 1010 of this embodiment does not have the overflow chamber 916, etc., the configuration of the centrifugal container 910 of the tenth embodiment is preferable in that a sufficient amount of the test

liquid S can be held in the capture chamber 912.

Twelfth Embodiment

The centrifugal container 1110 according to yet another embodiment of the present invention will now be described with reference to Figs. 62 to 63C.
Of the members appearing in this embodiment, those having the same function, shape, etc., as those of the tenth embodiment will be numbered the same and will not be described again in detail.
As shown in Fig. 62, the centrifugal container 1110 of this embodiment differs from the centrifugal container 910 described in the tenth embodiment in that the pouring chamber 911 and the capture chamber 912 communicate with each other via a substantially L-shaped channel 1113, but otherwise the configurations are the same.
In the centrifugal container 1110, the test liquid S poured into the pouring chamber 911 is transferred to the capture chamber 912, just as with the centrifugal container 910 of the tenth embodiment.
At this point, as shown in Figs. 63A and 63B, the channel 1113 allowing the pouring chamber 911 and the capture chamber 912 to communicate is connected near the bottom surface (in the drawing) of the inner surface 910b forming the capture chamber 912. The cell adhesion layer 914 is provided on this bottom surface.
This effectively suppresses the mixing of air into the test liquid S that has been transferred from the pouring chamber 911 to the capture chamber 912.
Then, as the inside of the capture chamber 912 is filled with the test liquid S, the air inside the capture chamber 912 is released to the outside through the air hole 915 shown in Fig. 63c.

Thirteenth Embodiment

The centrifugal container 1210 according to yet another embodiment of the present invention will now be described with reference to Figs. 64 to 65C.
Of the members appearing in this embodiment, those having the same function, shape, etc., as those of the tenth embodiment will be numbered the same and will not be described again in detail.
As shown in Fig. 64, the centrifugal container 1210 of this embodiment differs from the centrifugal container 910 described in the tenth embodiment in that bottom surface of the pouring chamber 911 and the upper surface of the capture chamber 912 are inclined surfaces 1211a and 1212a, respectively, but otherwise the configurations are the same.
In the centrifugal container 1210, the test liquid S poured into the pouring chamber 911 is transferred to the capture chamber 912, just as with the centrifugal container 910 of the tenth embodiment.
At this point, as shown in Figs. 65A and 65B, the bottom surface of the pouring chamber 911 is an inclined surface (first inclined surface) 9311a that inclines downward toward the capture chamber 912 in side view. Furthermore, as shown in Figs. 65A and 65B, the upper surface of the capture chamber 912 is an inclined surface (second inclined surface) 1212a that inclines upward toward the pouring chamber 911 in side view.
Consequently, when the centrifugal container 1210 is in oriented substantially vertically, the test liquid S in the pouring chamber 911 moves smoothly toward the capture chamber 912.
Furthermore, when the centrifugal container 1210 is oriented substantially vertically in the above-mentioned liquid transfer step, the air contained in the test liquid S in the capture chamber 912 moves along the inclined surface 1212a that is inclined toward the air hole shown in Fig. 65c, and can be efficiently released through the air hole 915 to the outside. This allows the venting of air from the capture chamber 912 to be completed prior to the rinsing step.
Also, because of the positional relation between the pouring chamber 911 and the capture chamber 912 shown in Figs. 65A to 65C, after the liquid transfer process and the capture process are complete, even when the rotation speed of the centrifuge is raised and the orientation is changed to be substantially horizontal in the orientation change step, it is still possible to effectively suppress the test liquid S from leaking out of the capture chamber 912, or air from becoming mixed into the capture chamber 912.

Other Embodiments

Embodiments of the present invention were described above, but the present invention is not limited to the above embodiments, and various modifications are possible without departing from the gist of the invention.
(F) In the above embodiment, an example was given in which the control unit 951 controlled the rotation of the motor 932 of the centrifuge 930 to increase the centrifugal force in stages, so that when centrifugal force over a specific level was applied, the orientation of the container 910 was automatically switched. However, the present invention is not limited to this.
For example, as shown in Fig. 66, the configuration may be such that a bowl-shaped rotor 1333 provided with holding portions of different orientations is rotated in order to manually change the orientation of the centrifugal container

910, thereby applying centrifugal force to the centrifugal container 910, so that the target cells C1 are efficiently taken out of the test liquid S in the centrifugal container 910.

More specifically, as shown in Fig. 66, the rotor 1333 comprises a main body 1333a, first container holding portions 1334a, and second container holding portions 1334b.

The main body 1333a has a bowl-like shape, and is provided with the first container holding portions 1334a on the inner peripheral surface side and the second container holding portions 1334b on the bottom surface.

The first container holding portions 1334a consist of two recesses provided at positions opposite each other on the inner peripheral surface of the main body 1333a, and hold the centrifugal container 910 substantially in the vertical direction.

The second container holding portions 1334b consist of two recesses provided on the bottom surface of the main body 1333a at opposing positions across the center of rotation, and hold the centrifugal container 910 substantially horizontally.

As a result, the centrifugal container 910 held by the first container holding portions 1334a is such that the cell adhesion layer 914 is disposed substantially vertically, and the centrifugal container 910 held by the second container holding portions 1334b is such that the cell adhesion layer 914 is disposed substantially horizontally, so the target cells C1 can be efficiently taken out of the test liquid S, which is the same effect as in the above embodiment.

(G) In the above embodiment, an example was given in which the orientation of the centrifugal container 910 was changed according to the amount of centrifugal force by causing the container holder 934 to pivot around the swing shafts 934b. However, the present invention is not limited to this.

For example, the mechanism for changing the orientation of the centrifugal container may be configured such that the orientation of the centrifugal container is mechanically changed, in addition to the method of pivoting around the swing shafts.

(H) In the above embodiment, an example was given in which the above steps were carried out while switching the rotation speed of the centrifuge 930 between four stages of rotation speed, V1 to V4. However, the present invention is not limited to this.

For example, the rotation speed V3 for changing the orientation of the centrifugal container and the rotation speed V4 for removing contaminants C2 in the capture chamber 912 after the orientation change (rinsing step) may be substantially the same rotation speed.

(I) In the above embodiment, an example was given in which two centrifugal containers 910 were attached to the rotor 933 and the centrifuge 930 was rotated. However, the present invention is not limited to this.

For example, the number of centrifugal containers attached to the centrifuge may be one, or may be three or more.

(J) In the above embodiment, an example was given in which the centrifugal container 910 was substantially fan-shaped. However, the present invention is not limited to this.

For example, the centrifugal container is not limited to having a fan shape, and may have some other shape, such as a substantially cuboid shape, or a substantially cylindrical shape.

## INDUSTRIAL APPLICABILITY

The cell capture device, cell capture apparatus, and cell capture method disclosed herein allow cells such as circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells contained in intravascular fluids such as blood and lymphatic fluid to be favorably captured, and as such can be used to advantage in various medical applications.

## REFERENCE SIGNS LIST

101-105 cell capture devices
110, 110', 120 centrifugal container
110s space
111 cell adhesion layer
112, 114 cell adhesion suppression layer
113 inner surface
113's side surface portion
113a inner side surface portion
113b bottom surface portion

113b' outer side surface portion
113c, 113d, 113s side surface portion
120a upper surface
120b lower surface
120c rotation axis
120s space
121' base
122 cover
122h opening
150, 150a, 150b target cell
151 hydration shell
161 hydration structure
160 biocompatible polymeric material
152, 162 nonfreezing water
153, 163 intermediate water
154, 164 free water
170 test liquid
201, 202 centrifuge
211 bucket
211a axis
212 rotary shaft
213 arm
220' turntable
301, 302 cell capture apparatus
10 cell capture apparatus
11 centrifuge
12 main body
13 lid
14 mounting portion
15 display unit
20 centrifugal container
21 main body
22 cover
22a vent hole
23 rotation axis
24 holding space
24a bottom surface (inner surface, bottom surface portion)
24b outer peripheral surface (inner surface, outer side surface portion, first inclined portion)
24c inner peripheral surface (inner surface, second inclined portion)
25 discharge port
26 collection chamber
27 cell adhesion layer
28a, 28b cell adhesion suppression layer
31 hydration shell
32 nonfreezing water
33 intermediate water
34 free water
40 biocompatible polymeric material
41 hydration structure
42 nonfreezing water
43 intermediate water
44 free water
120 centrifugal container
121 main body
124 holding space
124a bottom surface (inner surface, bottom surface portion)
124b outer peripheral surface (inner surface, outer side surface portion, first inclined portion)
124c inner peripheral surface (inner surface, second inclined portion)

125 discharge port
220 centrifugal container
221 main body
224 holding space
224a bottom surface (inner surface, bottom surface portion)
224b outer peripheral surface (inner surface, outer side surface portion, first inclined portion)
224c inner peripheral surface (inner surface, second inclined portion)
228 cell adhesion suppression layer
320 centrifugal container
321 main body
324 holding space
324a bottom surface (inner surface, bottom surface portion)
324b outer peripheral surface (inner surface, outer side surface portion, first inclined portion)
324c inner peripheral surface (inner surface, second inclined portion)
420 centrifugal container
421 main body
424 holding space
424a bottom surface (inner surface, bottom surface portion)
424b outer peripheral surface (inner surface, outer side surface portion, first inclined portion)
424c inner peripheral surface (inner surface, second inclined portion)
520 centrifugal container
521 body
524 holding space
524a bottom surface (inner surface, bottom surface portion)
524b outer peripheral surface (inner surface, outer side surface portion, first inclined portion)
524c inner peripheral surface (inner surface, second inclined portion)
525 discharge port
620 centrifugal container
624 holding space
624a bottom surface (inner surface, bottom surface portion)
624b outer peripheral surface (inner surface, outer side surface portion, first inclined portion)
624c inner peripheral surface (inner surface, second inclined portion)
720 centrifugal container
724 holding space
724a bottom surface (inner surface, bottom surface portion)
724b outer peripheral surface (inner surface, outer side surface portion, first inclined portion)
724c inner peripheral surface (inner surface, second inclined portion)
810 cell capture apparatus
811 centrifuge
814 mounting portion
820 centrifugal container
821 main body
822 cover
823 rotation axis
824 holding space
824a bottom surface (inner surface, bottom surface portion)
824b outer peripheral surface (inner surface, outer side surface portion)
824c inner peripheral surface (inner surface)
827 cell adhesion layer
828 cell adhesion suppression layer
C1 target cell
C2 contaminant
d1 first distance
d2 second distance
F, F1, F2, F3 force
G centrifugal force
1 hydration shell
1a nonfreezing water

1b intermediate water
1c free water
2 biocompatible polymeric material
3 hydration structure
3a nonfreezing water
3b intermediate water
3c free water
910 centrifugal container
910a main body
910b inner surface
911 pouring chamber
911a pouring port
912 capture chamber
913 channel (capillary channel)
914 cell adhesion layer
915 air hole
916 overflow chamber
917 overflow channel
930 centrifuge
931 base
932 motor
933 rotor
933a swing bearing
933b stopper pin
933 c rotary shaft
934 container holder
934a holding portion
934b swing axis
934c stopper hole
950 cell capture apparatus
951 control unit
1010 centrifugal container
1110 centrifugal container
1113 channel
1210 centrifugal container
1211a inclined surface (first inclined surface)
1212a inclined surface (second inclined surface)
1333 rotor
1333a main body
1334a first container holder
1334b second container holder
A1 air
G centrifugal force
S test liquid
V1 rotation speed (first speed)
V2 rotation speed (second speed)
V3 rotation speed (third speed)
V4 rotation speed (fourth speed)

## Claims

**1.** A cell capture device, comprising:

a centrifugal container that can be mounted in a centrifuge, and that has a space in which a test liquid containing target cells is held, and an inner surface that defines the space; and
a cell adhesion layer that is disposed on the inner surface and is configured to adsorb the target cells in the test liquid.

**2.** The cell capture device according to claim 1, wherein the cell adhesion layer is disposed in a capture region of the inner surface that is not parallel to a centrifugal direction of the centrifugal container.

**3.** The cell capture device according to claim 2, further comprising a cell adhesion suppression layer that is disposed in a region of the inner surface other than the capture region, and that is configured to suppress adsorption of the target cells in the test liquid.

**4.** The cell capture device according to any of claims 1 to 3, wherein the target cells include at least one type selected from a group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells, and the cell adhesion layer adsorbs the target cells.

**5.** The cell capture device according to any of claims 1 to 4, wherein the cell adhesion layer includes a first biocompatible polymeric material, and
the first biocompatible polymeric material includes one or more of polymers expressed by the following formulas 1 to 4, or a copolymer of a monomer constituting the polymer expressed by formula 1 and a monomer constituting one or more types of polymer selected from a group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

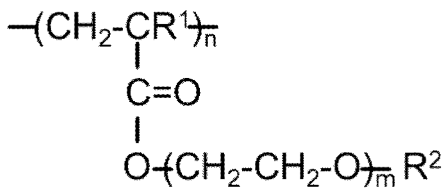

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

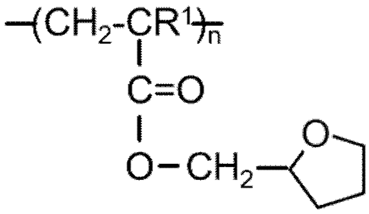

(2)

(In formula 2, $R^1$ is a hydrogen atom or a methyl group, and n is a repeating unit.)

Chemical Formula 3

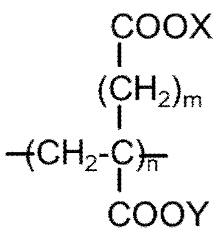

(3)

(In formula 3, X and Y are each independently a hydrogen atom, an alkali metal atom, or an ammonium group, m is an integer of 0, 1, or 2, and n is a repeating unit.)

Chemical Formula 4

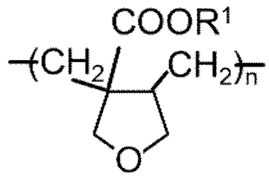

(4)

(In formula 4, $R^1$ is a hydrogen atom or an organic group having 1 to 30 carbon atoms, and n is a repeating unit.)

**6.** The cell capture device according to claim 3, wherein the cell adhesion suppression layer includes a second biocompatible polymeric material, and
the second biocompatible polymeric material includes a polymer having phospholipid polar groups.

**7.** The cell capture device according to claim 3, wherein the centrifugal container has a cylindrical shape having the space,

the space has a columnar shape,
the inner surface includes a side surface portion that defines a side portion of the columnar shape, and a bottom surface portion that defines a bottom portion of the columnar shape, and
the cell adhesion layer covers the bottom surface portion of a columnar inner surface.

**8.** The cell capture device according to claim 7, wherein the bottom surface portion is partially spherical.

**9.** The cell capture device according to claim 8, wherein the cell adhesion layer is disposed on an arc with respect to a center of rotation.

**10.** The cell capture device according to any of claims 7 to 9, wherein the cell adhesion suppression layer covers the side surface portion of the columnar inner surface.

**11.** The cell capture device according to claim 3, wherein the centrifugal container has a disk shape having an upper surface, a lower surface, and a rotary shaft,

the space is formed at a position that is away from the rotary shaft of the disk shape in a radial direction,
the inner surface includes an outer side surface portion that is farthest away from the rotary shaft in the radial direction, and
the cell adhesion layer is disposed on the outer side surface portion.

**12.** The cell capture device according to claim 11, wherein the cell adhesion suppression layer covers a surface of the inner surface other than the outer side surface portion.

**13.** A cell capture apparatus, comprising:

the cell capture device according to any of claims 1 to 12; and
a centrifuge in which the cell capture device can be mounted, and that is configured to apply centrifugal force to the cell capture device by rotating the cell capture device.

**14.** A cell capture method, comprising following steps:

introducing a test liquid containing target cells into a space of a cell capture device comprising a space for holding a test liquid containing target cells, a centrifugal container having an inner surface that defines the space, and a cell adhesion layer including a first biocompatible polymeric material that is disposed on the inner surface and adsorbs the target cells in the test liquid; and
mounting the cell capture device in a centrifuge and rotating the cell capture device to adsorb the target cells

to the cell adhesion layer.

**15.** The cell capture method according to claim 14, wherein, in the step of adsorption, a centrifugal force of at least 500 G and no more than 10,000 G is applied to the test liquid held in the cell capture device.

**16.** The cell capture method according to claim 14 or 15, further comprising a step of bringing a liquid containing water into contact with the cell adhesion layer prior to the step of introduction.

**17.** The cell capture method according to any of claims 14 to 16, wherein the cell adhesion layer is disposed in a capture region of the inner surface that is not parallel to a centrifugal direction of the centrifugal container.

**18.** The cell capture method according to any of claims 14 to 17, wherein the target cells include at least one type selected from a group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells, and the cell adhesion layer adsorbs the target cells.

**19.** The cell capture method according to any of claims 14 to 18, wherein the first biocompatible polymeric material includes one or more of polymers expressed by the following formulas 1 to 4, or a copolymer of a monomer constituting the polymer expressed by formula 1 and a monomer constituting one or more types of polymer selected from a group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

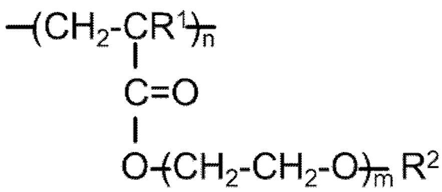

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

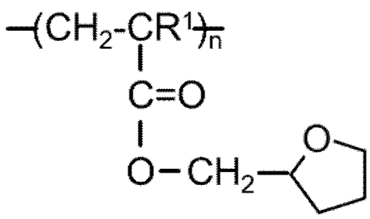

(2)

(In formula 2, $R^1$ is a hydrogen atom or a methyl group, and n is a repeating unit.)

Chemical Formula 3

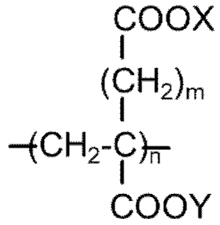

(3)

(In formula 3, X and Y are each independently a hydrogen atom, an alkali metal atom, or an ammonium group, m is an integer of 0, 1, or 2, and n is a repeating unit.)

Chemical Formula 4

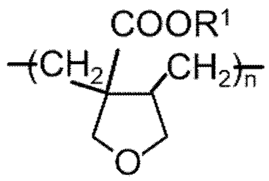

(4)

(In formula 4, $R^1$ is a hydrogen atom or an organic group having 1 to 30 carbon atoms, and n is a repeating unit.)

**20.** A centrifugal container that is rotationally driven while mounted in a centrifuge, comprising:

an inner surface that forms a holding space for holding a test liquid containing target cells and contaminants other than the target cells; and
a cell adhesion layer that is disposed in a capture region of the inner surface that is substantially parallel to a centrifugal direction in which centrifugal force is applied by the centrifuge, and that is configured to adsorb the target cells in the test liquid.

**21.** The centrifugal container according to claim 20,
further comprising a cell adhesion suppression layer that is disposed in a region of the inner surface other than the capture region, and that is configured to suppress an adsorption of the target cells in the test liquid.

**22.** The centrifugal container according to claim 20 or 21,

further comprising a rotary shaft that serves as a center of rotation when rotated by the centrifuge,
wherein the holding space is formed in a substantially arc shape at a position that is away from the rotary shaft in a radial direction,
the inner surface includes a bottom surface portion that is substantially parallel to the centrifugal direction, and
the cell adhesion layer is disposed on the bottom surface portion.

**23.** The centrifugal container according to claim 22,

further comprising a cell adhesion suppression layer that is disposed in a region of the inner surface other than the capture region, and that is configured to suppress the adsorption of the target cells in the test liquid,
wherein the cell adhesion suppression layer is provided on a surface of the inner surface other than the bottom surface portion.

**24.** The centrifugal container according to claim 22 or 23,
further comprising a first inclined portion that is provided on an outer peripheral side of the holding space and inclines downward from the outer peripheral side toward an inner peripheral side in the radial direction centered on the rotary shaft.

**25.** The centrifugal container according to any of claims 22 to 24,
wherein the inner surface includes an outer side surface portion that is provided at a position of the inner surface farthest away from a rotation axis in the radial direction.

**26.** The centrifugal container according to claim 25,
wherein the outer side surface portion has an eccentric shape including a first distance and a second distance different from the first distance, in the radial direction centered on the rotary shaft.

**27.** The centrifugal container according to claim 26,
further comprising a discharge port that is provided on the outer side surface portion and is configured to discharge the contaminants to the outside of the holding space.

**28.** The centrifugal container according to claim 27,
wherein the discharge port is provided at a position of the outer side surface portion farthest away from the rotary shaft in the radial direction.

**29.** The centrifugal container according to claim 25,
wherein the outer side surface portion is disposed along an arc at a constant radial distance from the rotary shaft.

**30.** The centrifugal container according to claim 29,
further comprising a discharge port that is provided on the outer side surface portion and is configured to discharge the contaminants to the outside of the holding space.

**31.** The centrifugal container according to claim 30,
wherein a plurality of the discharge ports are provided at positions of the outer side surface portion farthest away in the radial direction from the rotary shaft.

**32.** The centrifugal container according to claim 27, 28, 30, or 31,
further comprising a collection chamber configured to collect the contaminants discharged from the discharge port.

**33.** The centrifugal container according to claim 22 or 23,
further comprising a second inclined portion that is provided on an inner peripheral side of the holding space and inclines downward from the inner peripheral side toward an outer peripheral side in the radial direction centered on the rotary shaft.

**34.** The centrifugal container according to claim 33,
wherein the second inclined portion is formed along a substantially circular arc centered on the rotary shaft.

**35.** The centrifugal container according to any of claims 25 to 31,
wherein the outer side surface portion has a curved surface in a cross-sectional view including the rotary shaft.

**36.** The centrifugal container according to any of claims 20 to 35,
wherein the target cells include at least one type selected from a group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells.

**37.** The centrifugal container according to any of claims 20 to 36,

wherein the cell adhesion layer includes a first biocompatible polymeric material, and
the first biocompatible polymeric material includes one or more of polymers expressed by the following formulas 1 to 4, or a copolymer of a monomer constituting the polymer expressed by formula 1 and a monomer constituting one or more types of polymer selected from a group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

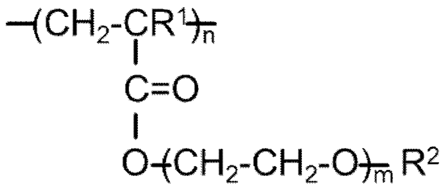 (1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

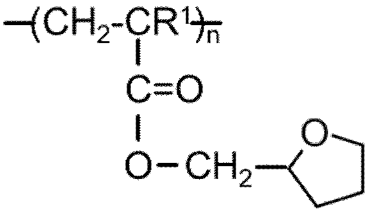 (2)

(In formula 2, $R^1$ is a hydrogen atom or a methyl group, and n is a repeating unit.)

Chemical Formula 3

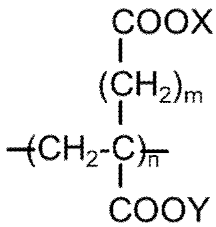 (3)

(In formula 3, X and Y are each independently a hydrogen atom, an alkali metal atom, or an ammonium group, m is an integer of 0, 1, or 2, and n is a repeating unit.)

Chemical Formula 4

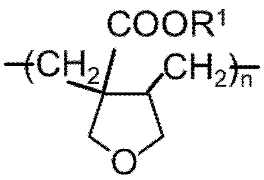 (4)

(In formula 4, $R^1$ is a hydrogen atom or an organic group having 1 to 30 carbon atoms, and n is a repeating unit.)

**38.** The centrifugal container according to claim 20 or 21,

further comprising a main body that is substantially fan shaped, and a virtual rotary shaft serving as a center of rotation when rotated by the centrifuge,
wherein the holding space is formed in a substantially arc shape at a position away from a rotary shaft in a radial direction,

the inner surface includes a bottom surface portion that is substantially parallel to the centrifugal direction, and the cell adhesion layer is disposed on the bottom surface portion.

**39.** A cell capture apparatus, comprising:

the centrifugal container according to any of claims 20 to 38; and
a centrifuge in which the centrifugal container can be mounted, and that is configured to apply centrifugal force to the test liquid including target cell in the centrifugal container by rotating the centrifugal container.

**40.** A cell capture method that makes use of the centrifugal container according to any of claims 20 to 38, the method comprising following steps:

introducing a test liquid containing target cells into the holding space of the centrifugal container; and
mounting the centrifugal container in a centrifuge, rotating the centrifugal container to adsorb the target cells to the cell adhesion layer, and washing out contaminants other than the target cells.

**41.** A centrifugal container that is mounted in a centrifuge and rotationally driven while an orientation is varied as needed in each step, the centrifugal container comprising:

a main body;
an inner surface that is provided inside the main body and forms a holding space for holding a test liquid containing target cells and contaminants other than the target cells; and
a cell adhesion layer that is provided to a part of the inner surface in order to adsorb the target cells in the test liquid, and that is disposed along a substantially vertical direction with respect to a direction of a centrifugal force applied by a rotation of the centrifuge when capturing the target cells contained in the test liquid poured into the holding space, and is disposed along a substantially horizontal direction with respect to the centrifugal force when removing the contaminants.

**42.** The centrifugal container according to claim 41,
wherein the holding space has a pouring chamber into which the test liquid is poured, and a capture chamber into which the test liquid is moved from the pouring chamber by an application of the centrifugal force, and at least a part of which is provided with the cell adhesion layer.

**43.** The centrifugal container according to claim 42,
wherein the capture chamber is provided at a position that is farther away from a center of rotation than the pouring chamber in an orientation in which the test liquid moves under the application of centrifugal force.

**44.** The centrifugal container according to claim 42 or 43,
further comprising a capillary channel configured to connect the pouring chamber and the capture chamber and hold the test liquid by capillary force.

**45.** The centrifugal container according to claim 44,
wherein the capillary channel is connected to the capture chamber near the inner surface on which the cell adhesion layer is formed.

**46.** The centrifugal container according to claim 44,
wherein the capillary channel is formed to be substantially L-shaped.

**47.** The centrifugal container according to claim 42 or 43,
further comprising an air hole that is provided in the capture chamber and is configured to release air inside the capture chamber to an atmosphere as the test liquid moves from the pouring chamber to the capture chamber.

**48.** The centrifugal container according to claim 42 or 43,
further comprising an overflow chamber that is connected to the capture chamber and is configured to hold any test liquid that has overflowed when the test liquid that has moved from the pouring chamber to the capture chamber fills up the capture chamber.

**49.** The centrifugal container according to claim 42 or 43,

wherein the pouring chamber has a first inclined surface that is inclined so that a side connected to the capture chamber is farther away from a center of rotation than an opposite side in an orientation in which the test liquid is moved to the capture chamber under the application of centrifugal force.

**50.** The centrifugal container according to claim 47,
wherein the capture chamber has a second inclined surface that is inclined so that a discharge side provided with the air hole is closer to a center of rotation than an opposite side in an orientation in which the test liquid is moved to the capture chamber under the application of centrifugal force.

**51.** The centrifugal container according to any of claims 41 to 43,
wherein the target cells include at least one type selected from a group consisting of circulating tumor cells, stem cells, vascular endothelial cells, nerve cells, dendritic cells, smooth muscle cells, fibroblasts, cardiomyocytes, skeletal muscle cells, hepatic parenchymal cells, hepatic stellate cells, and pancreatic islet cells.

**52.** The centrifugal container of any according to claims 41 to 43,

wherein the cell adhesion layer includes a first biocompatible polymeric material, and
the first biocompatible polymeric material includes one or more of polymers expressed by the following formulas 1 to 4, or a copolymer of a monomer constituting the polymer expressed by formula 1 and a monomer constituting one or more types of polymer selected from the group consisting of polyethylene glycol, polyvinyl pyrrolidone, polyvinyl methyl ether, polymethoxyethyl acrylamide, polymethoxyethyl methacrylamide, polymethoxyethyl vinyl ether, polytetrahydrofurfuryl acrylate, and polytetrahydrofurfuryl methacrylate.

Chemical Formula 1

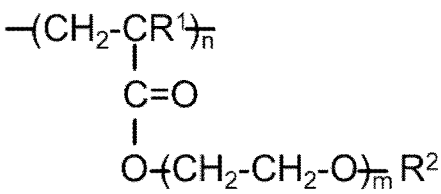

(1)

(In formula 1, $R^1$ is a hydrogen atom or a methyl group, $R^2$ is a methyl group or an ethyl group, m is an integer of 1, 2, or 3, and n is a repeating unit.)

Chemical Formula 2

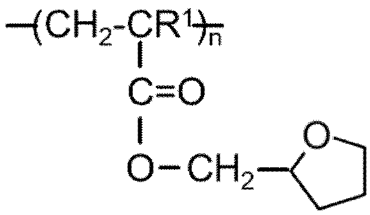

(2)

(In formula 2, $R^1$ is a hydrogen atom or a methyl group, and n is a repeating unit.)

Chemical Formula 3

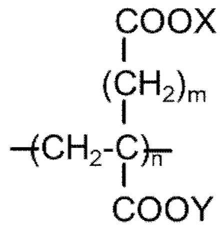

(3)

(In formula 3, X and Y are each independently a hydrogen atom, an alkali metal atom, or an ammonium group, m is an integer of 0, 1, or 2, and n is a repeating unit.)

Chemical Formula 4

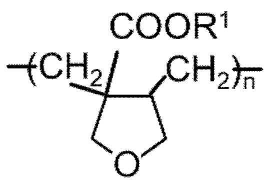

(4)

(In formula 4, $R^1$ is a hydrogen atom or an organic group having 1 to 30 carbon atoms, and n is a repeating unit.)

**53.** A cell capture device, comprising:

the centrifugal container according to any of claims 41 to 43;
a centrifuge that has a container holding unit in which the centrifugal container is mounted, and a rotary drive unit configured to rotate the centrifugal container held by the container holding unit to apply centrifugal force to the test liquid containing the target cell placed in the centrifugal container; and
a control unit configured to control a rotation speed of the rotary drive unit.

**54.** The cell capture device according to claim 53,
wherein the control unit changes the rotation speed of the rotary drive unit in steps to change an orientation of the centrifugal container.

**55.** The cell capture device according to claim 54,

wherein the holding space has a pouring chamber into which the test liquid is poured, and a capture chamber into which the test liquid is moved from the pouring chamber by an application of the centrifugal force, and at least a part of which is provided with the cell adhesion layer, and
the control unit rotates the centrifugal container at a first speed when moving the test liquid from the pouring chamber to the capture chamber.

**56.** The cell capture device according to claim 55,
wherein the control unit rotates the centrifugal container at a second speed that is higher than the first speed when the target cells contained in the test liquid that has moved to the capture chamber are adsorbed in the cell adhesion layer.

**57.** The cell capture device according to claim 56,
wherein the control unit rotates the centrifugal container at a third speed that is higher than the second speed to change the orientation of the centrifugal container so that the cell adhesion layer is substantially horizontal in the direction of the centrifugal force in a state in which the target cells have been adsorbed to the cell adhesion layer in the capture chamber.

**58.** The cell capture device according to claim 57,
wherein the control unit rotates the centrifugal container at a fourth speed equal to or higher than the third speed when moving the contaminants adsorbed in the cell adhesion layer of the capture chamber to the outside in a centrifugal direction while maintaining the orientation of the centrifugal container in which the cell adhesion layer is

facing substantially horizontally in the direction of the centrifugal force.

**59.** The cell capture device according to claim 57,
wherein the control unit rotates the centrifugal container at a fourth speed that is higher than the second speed when moving the contaminants adsorbed in the cell adhesion layer of the capture chamber to the outside in the centrifugal direction while maintaining the orientation of the centrifugal container in which the cell adhesion layer is facing substantially horizontally in the direction of the centrifugal force.

**60.** A cell capture method that makes use of the centrifugal container according to any of claims 41 to 43, the method comprising following steps:

introducing a test liquid containing target cells into the holding space of the centrifugal container; and
mounting the centrifugal container in a centrifuge, and rotating the centrifugal container to adsorb the target cells to the cell adhesion layer; and
washing out contaminants other than the target cells by rotating the centrifugal container in a state in which the orientation of the centrifugal container has been changed.

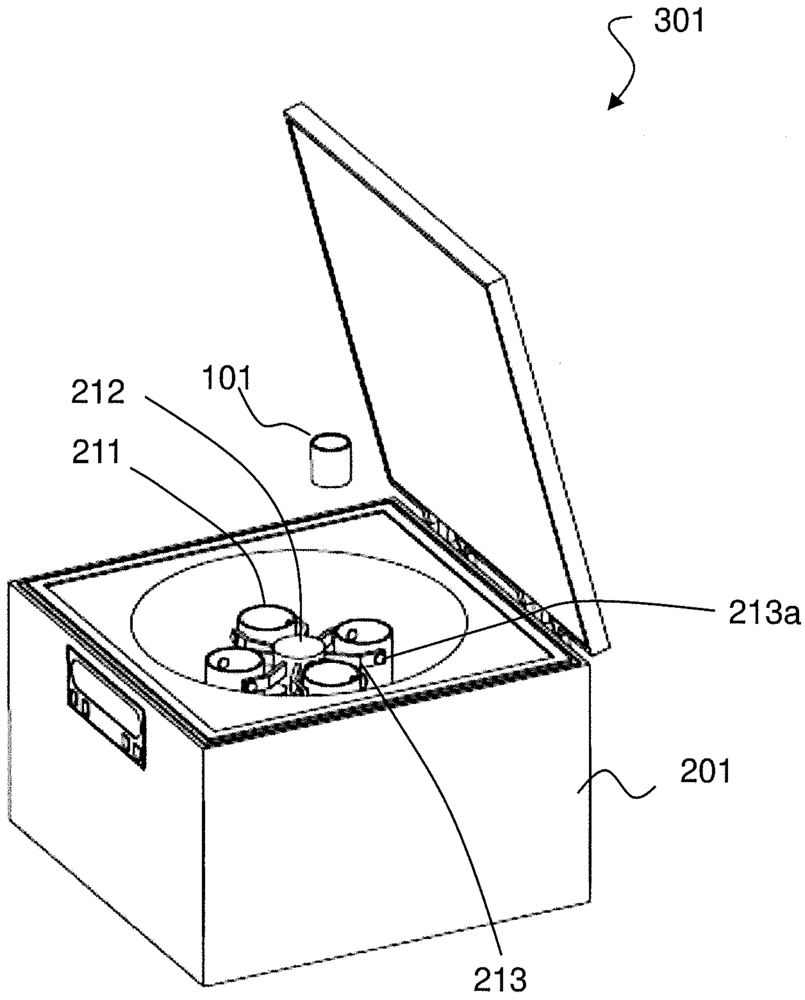


FIG. 1

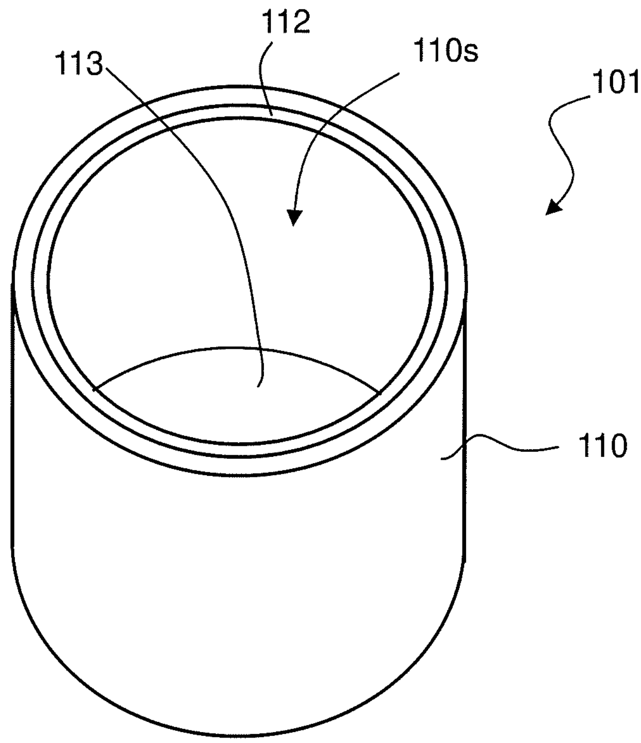


FIG. 2

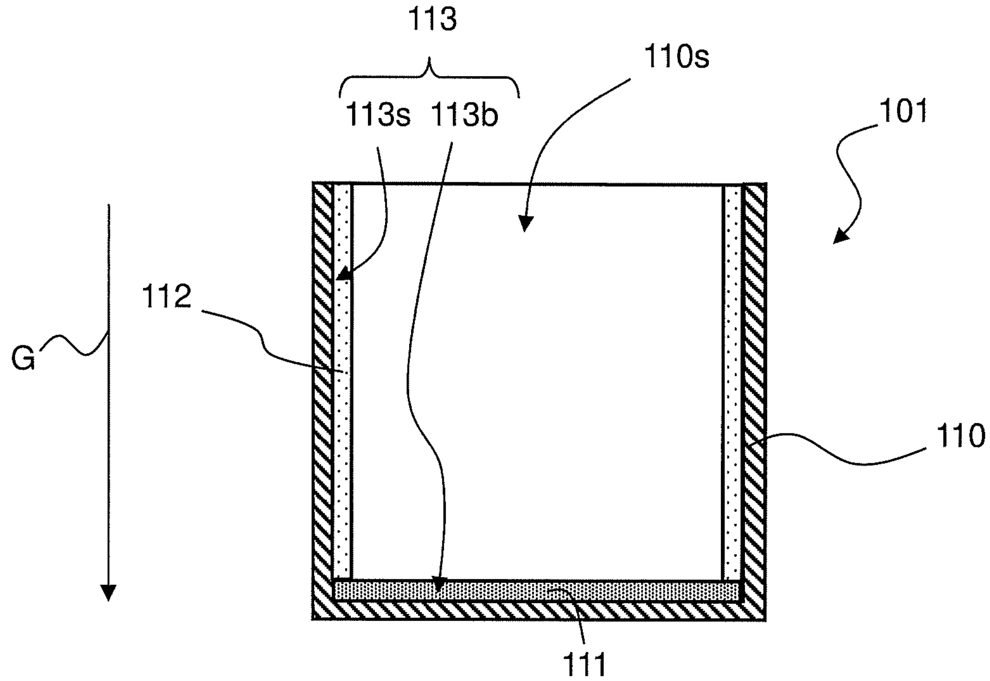


FIG. 3

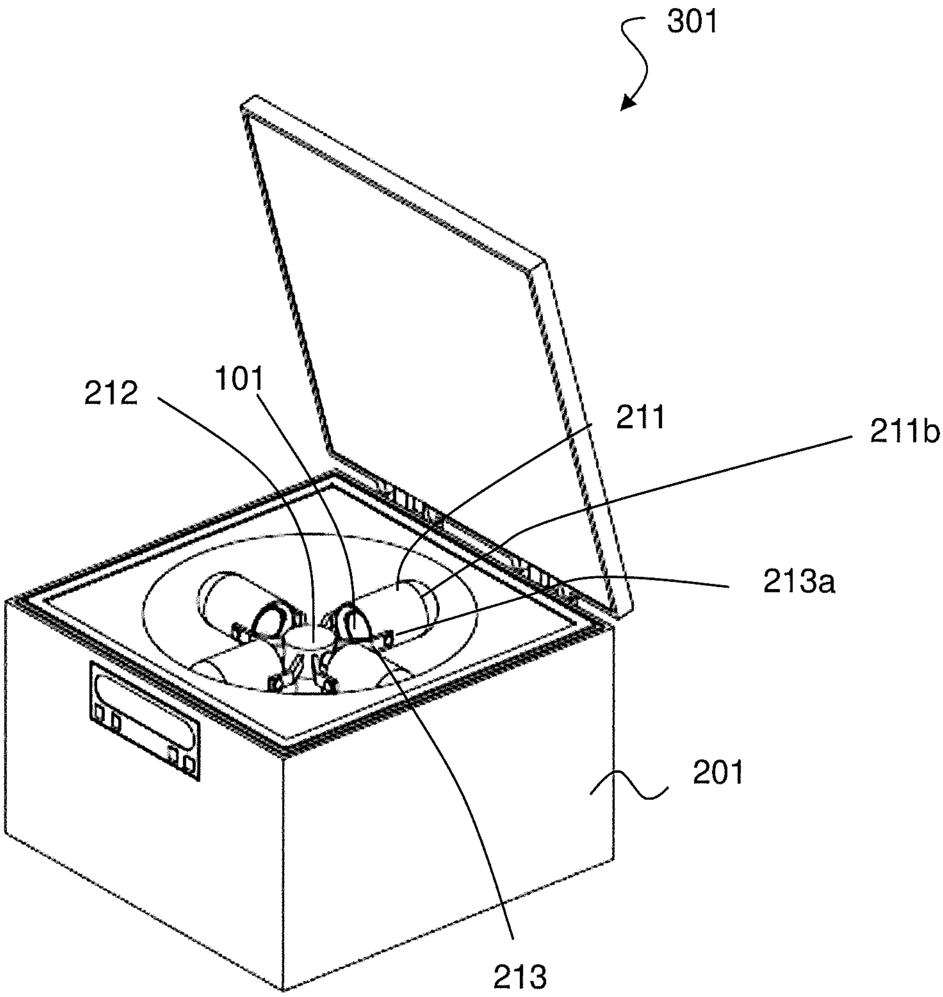


FIG. 4

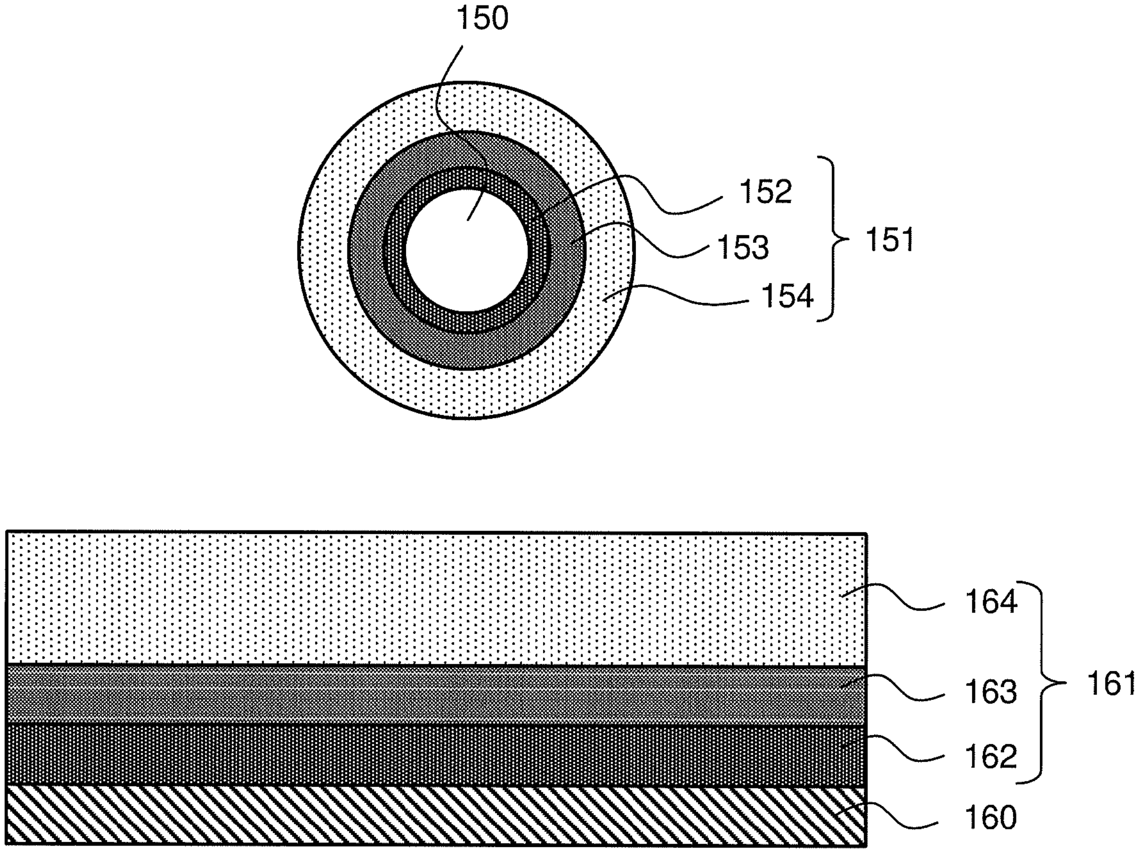


FIG. 5

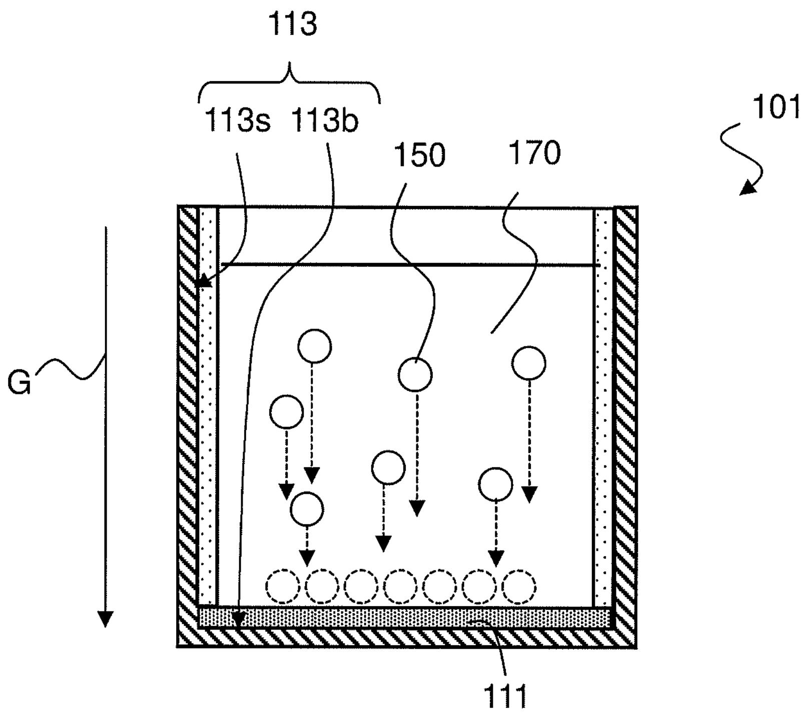


FIG. 6

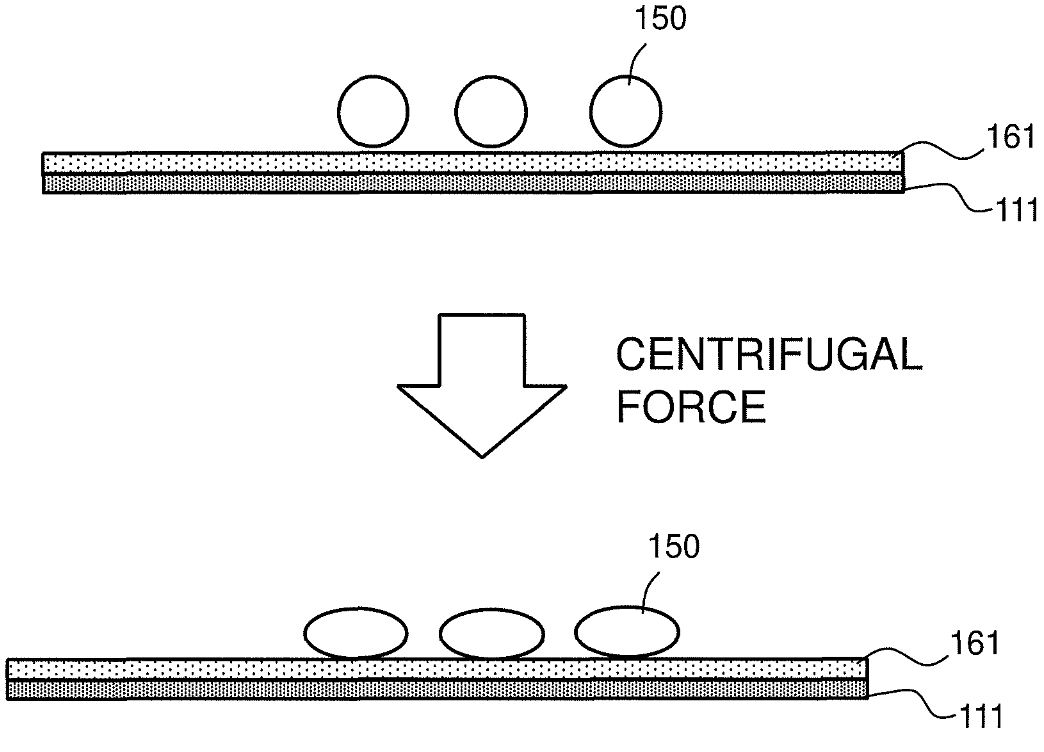


FIG. 7

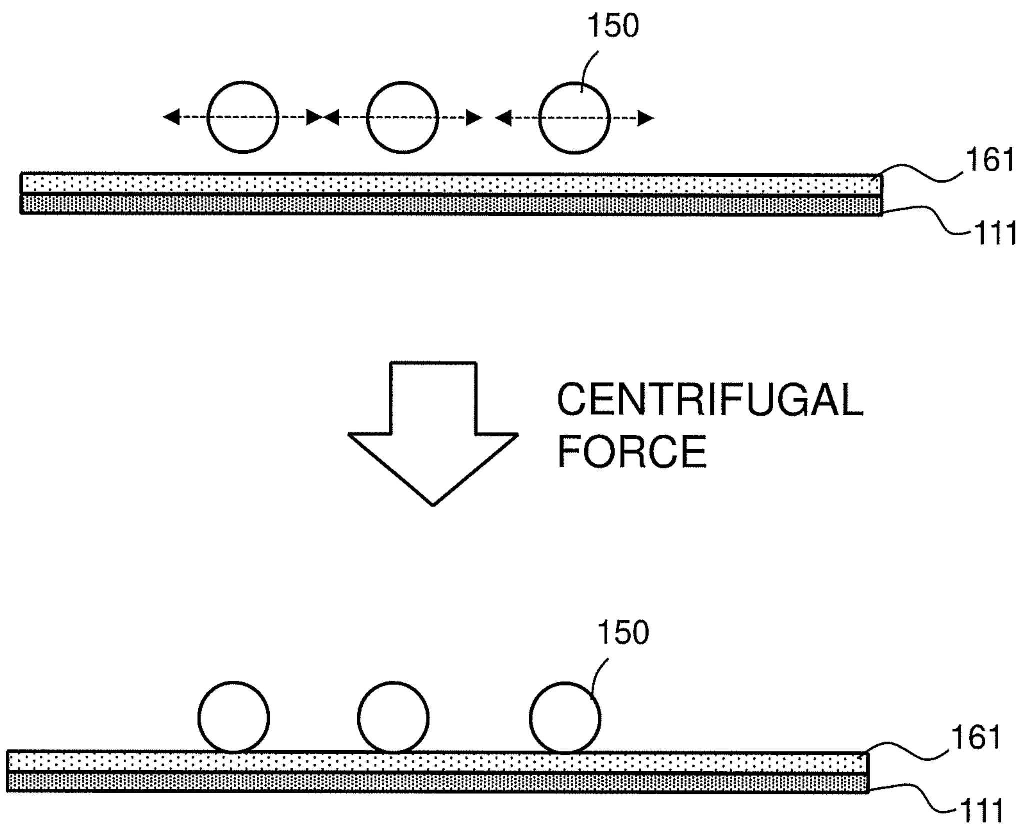


FIG. 8

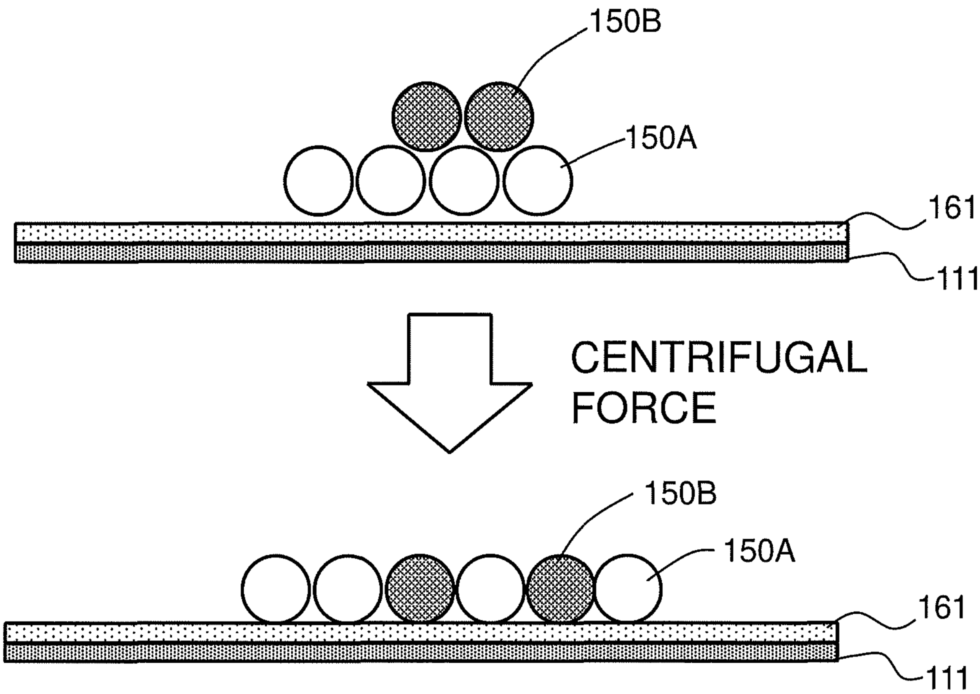
150B
150A
161
111
CENTRIFUGAL
FORCE
150B
150A
161
111

FIG. 9

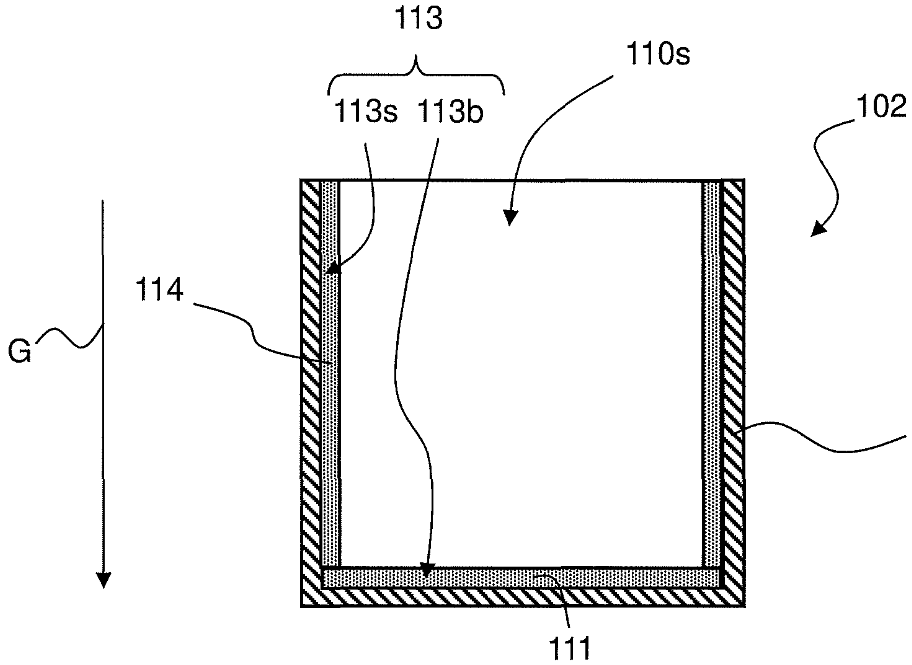
113
113s 113b
110s
102
114
G
110
111

FIG. 10

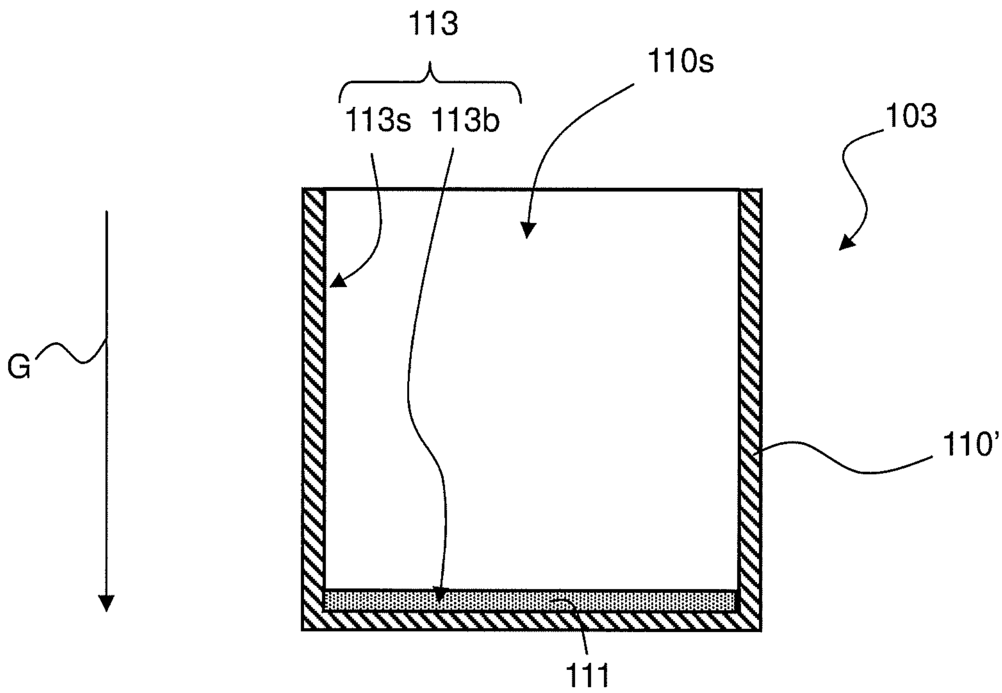


FIG. 11

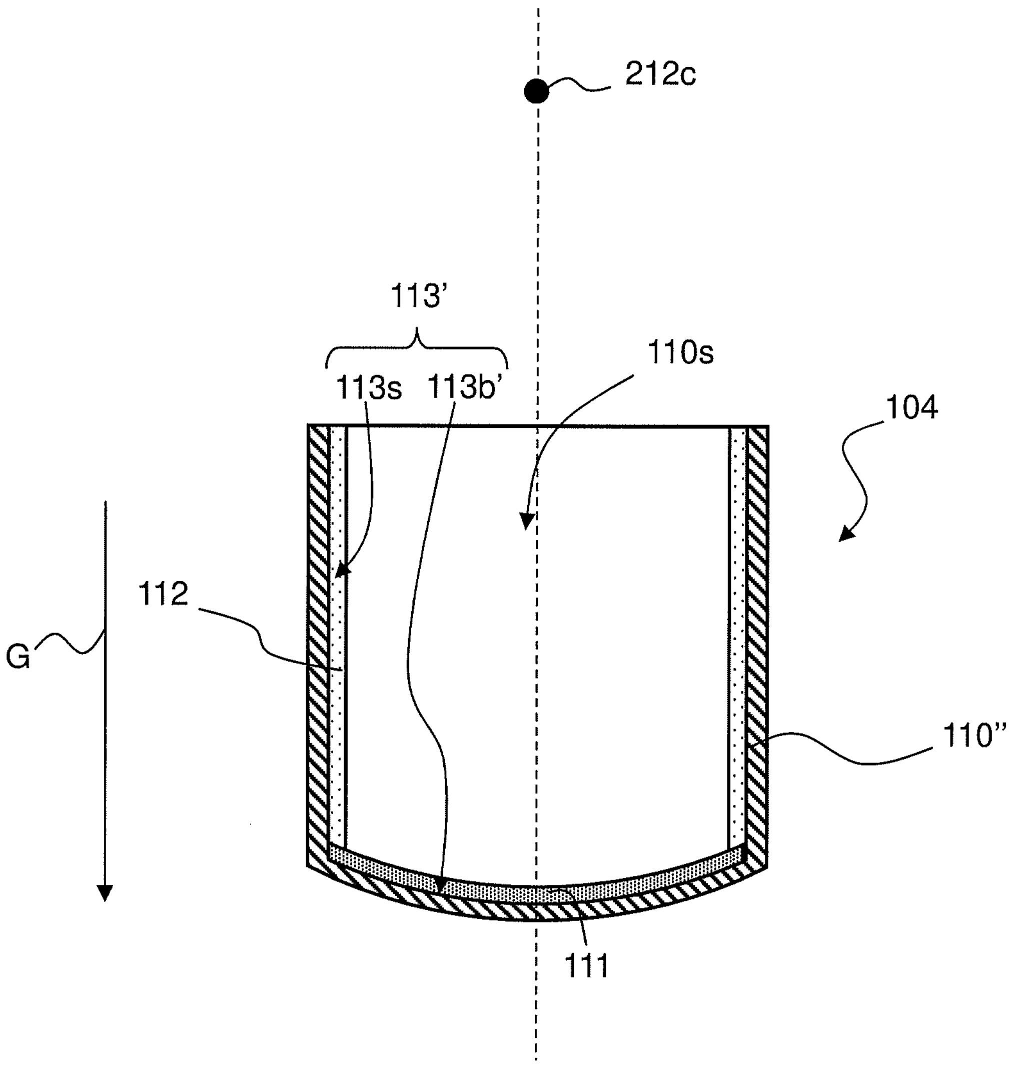


FIG. 12

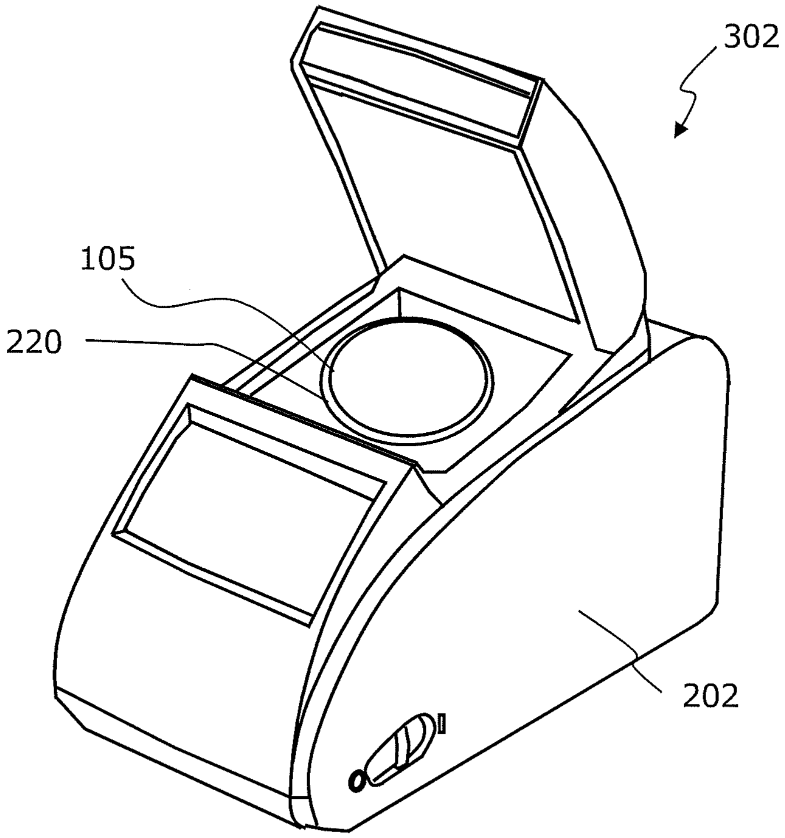
302
105
220
202

FIG. 13

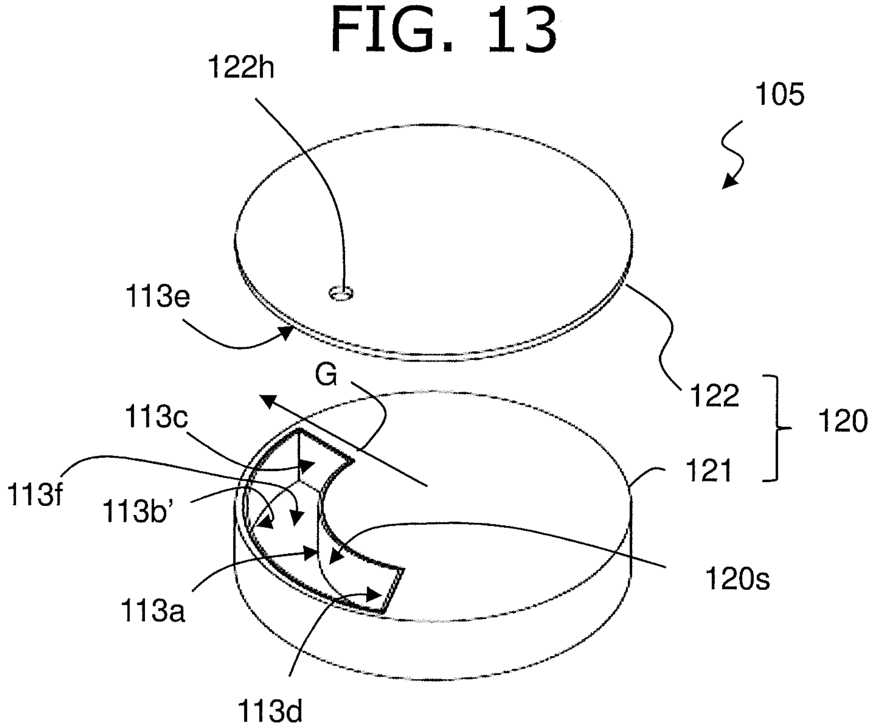
122h
105
113e
G
122
120
113c
121
113f
113b'
120s
113a
113d

FIG. 14

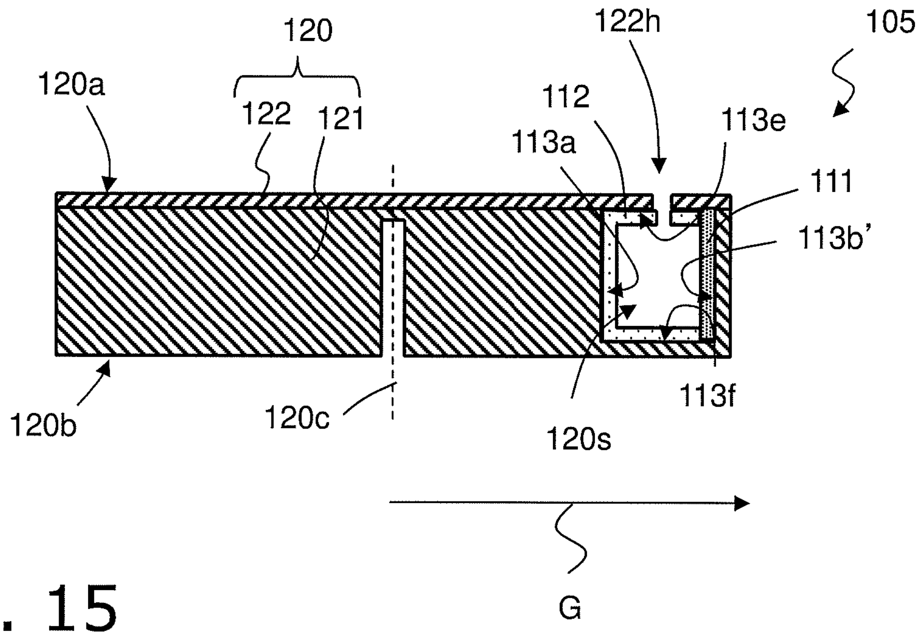
120
120a
122
121
122h
105
112
113a
113e
111
113b'
120b
120c
120s
113f
G

FIG. 15

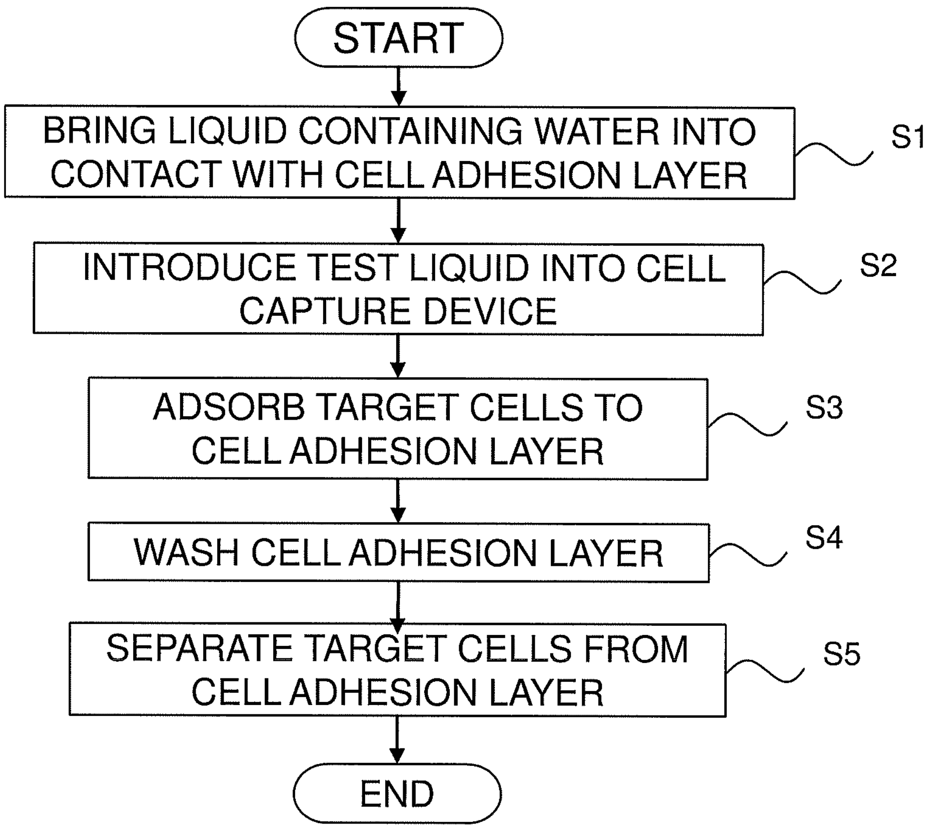
START
BRING LIQUID CONTAINING WATER INTO CONTACT WITH CELL ADHESION LAYER
S1
INTRODUCE TEST LIQUID INTO CELL CAPTURE DEVICE
S2
ADSORB TARGET CELLS TO CELL ADHESION LAYER
S3
WASH CELL ADHESION LAYER
S4
SEPARATE TARGET CELLS FROM CELL ADHESION LAYER
S5
END

FIG. 16

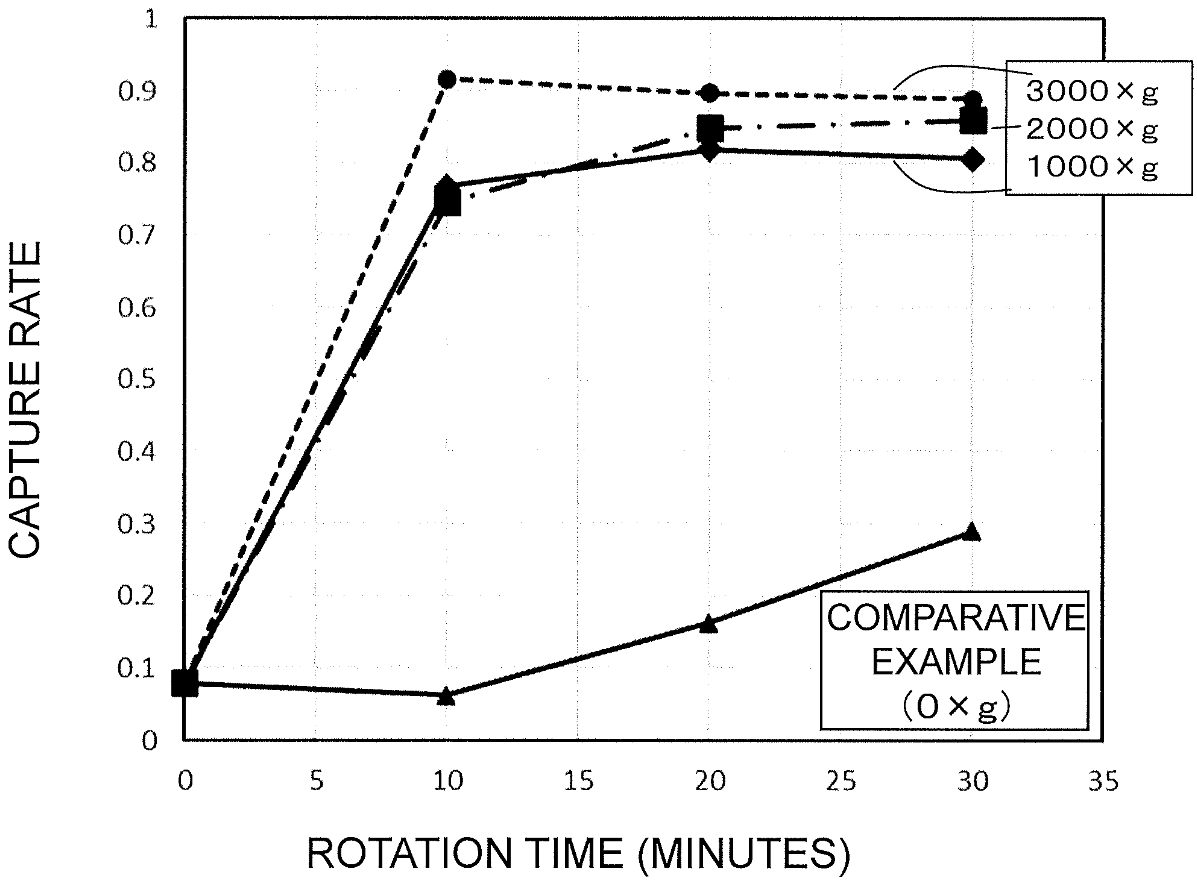
1
0.9
0.8
0.7
0.6
0.5
0.4
0.3
0.2
0.1
0
CAPTURE RATE
0
5
10
15
20
25
30
35
ROTATION TIME (MINUTES)
3000×g
2000×g
1000×g
COMPARATIVE EXAMPLE (0×g)

FIG. 17

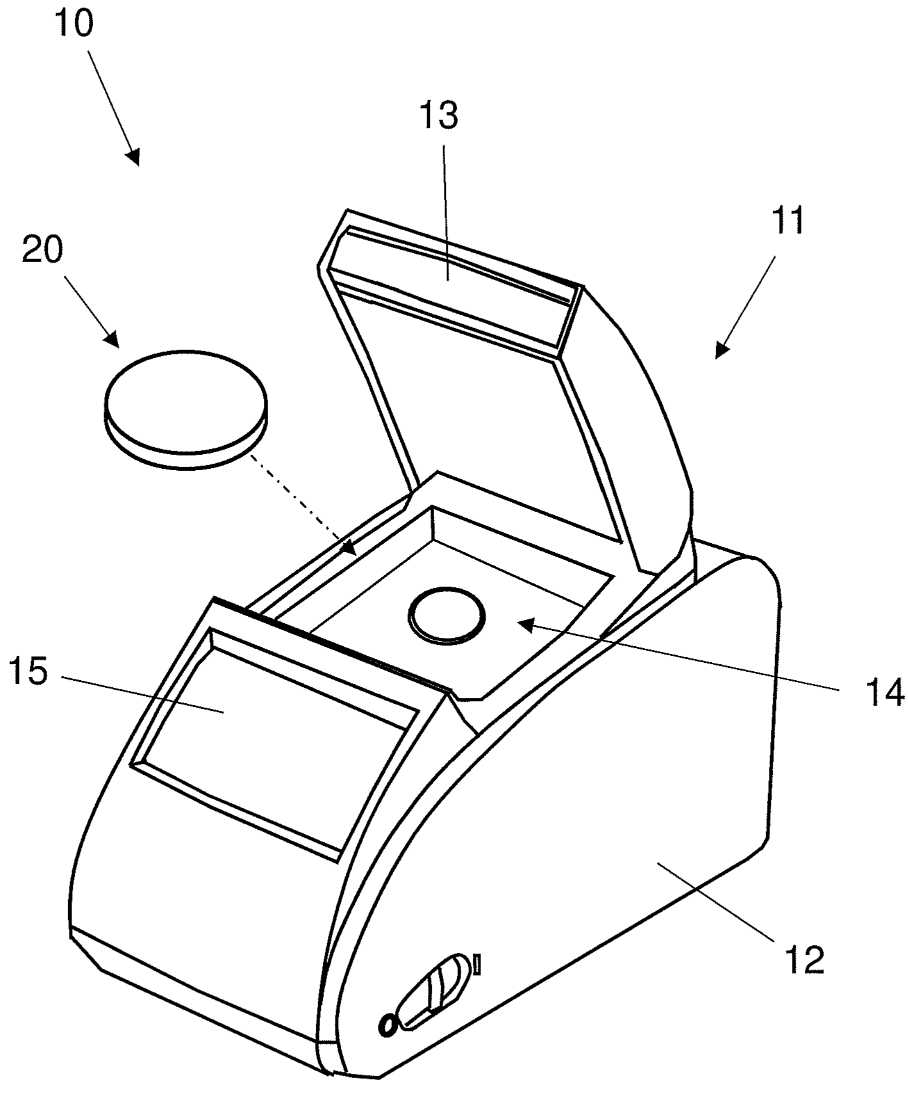


FIG. 18

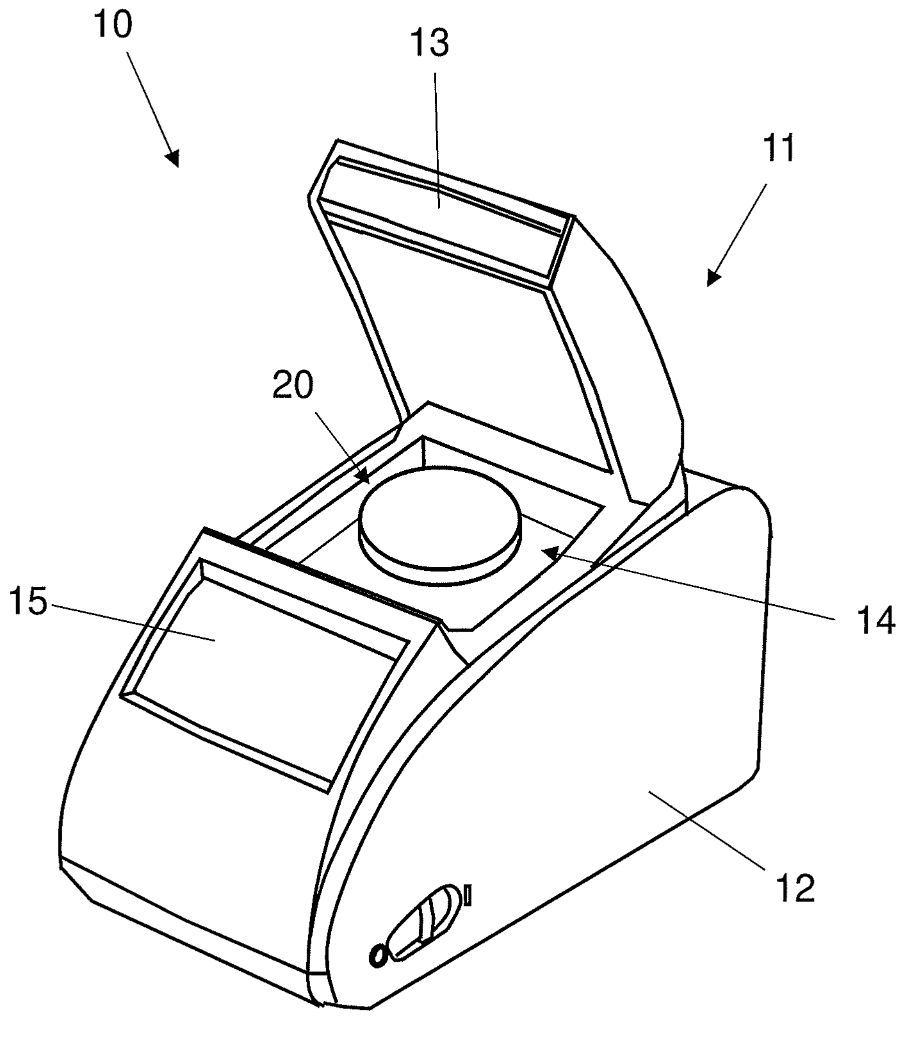


FIG. 19

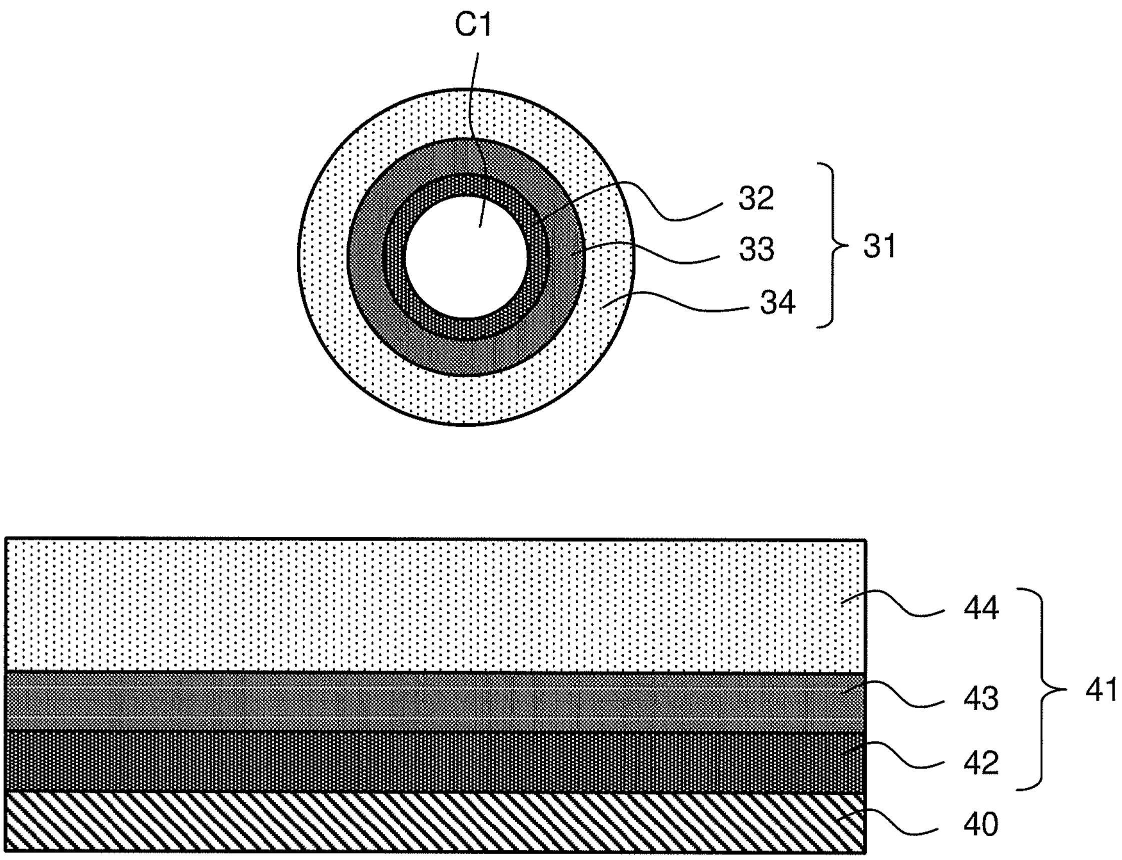


FIG. 20

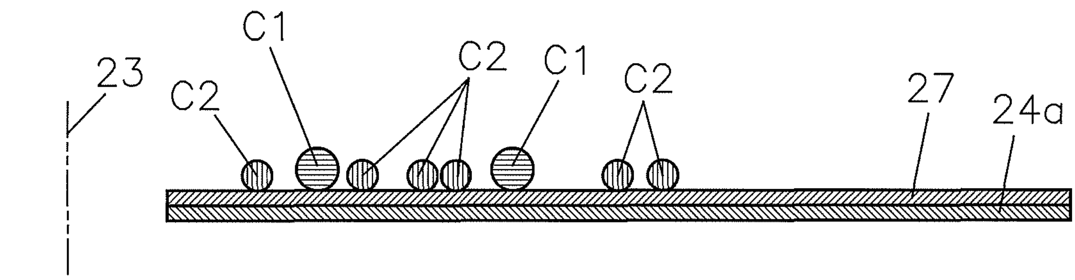


FIG. 21A

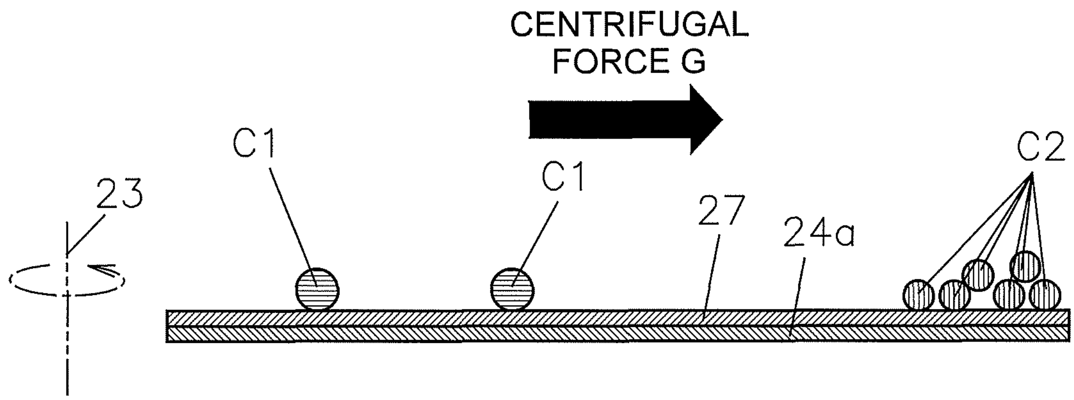


FIG. 21B

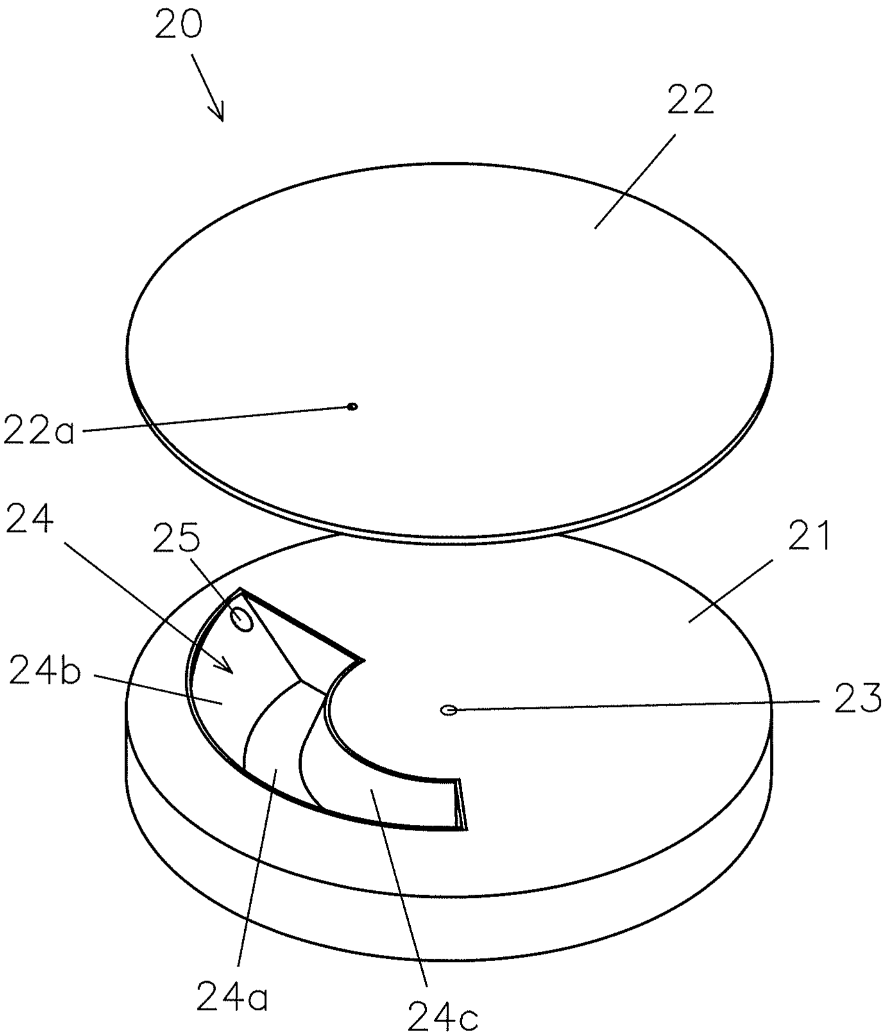


FIG. 22

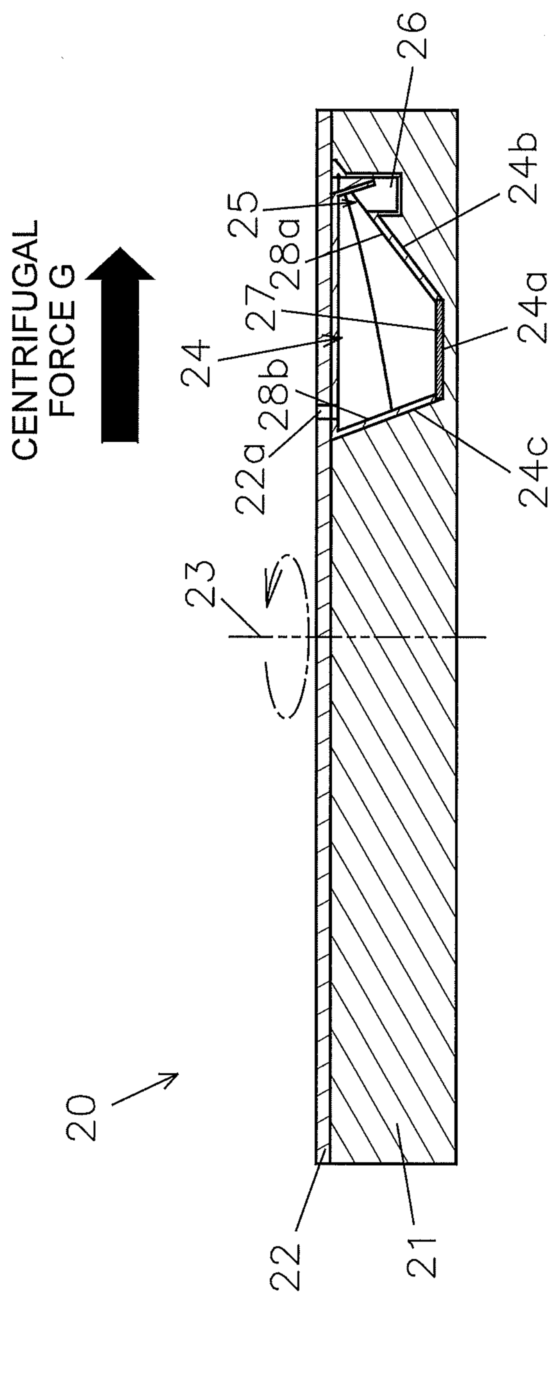
CENTRIFUGAL FORCE G
26
24b
25
28a
27
24a
24
28b
22a
24c
23
20
22
21

FIG. 23

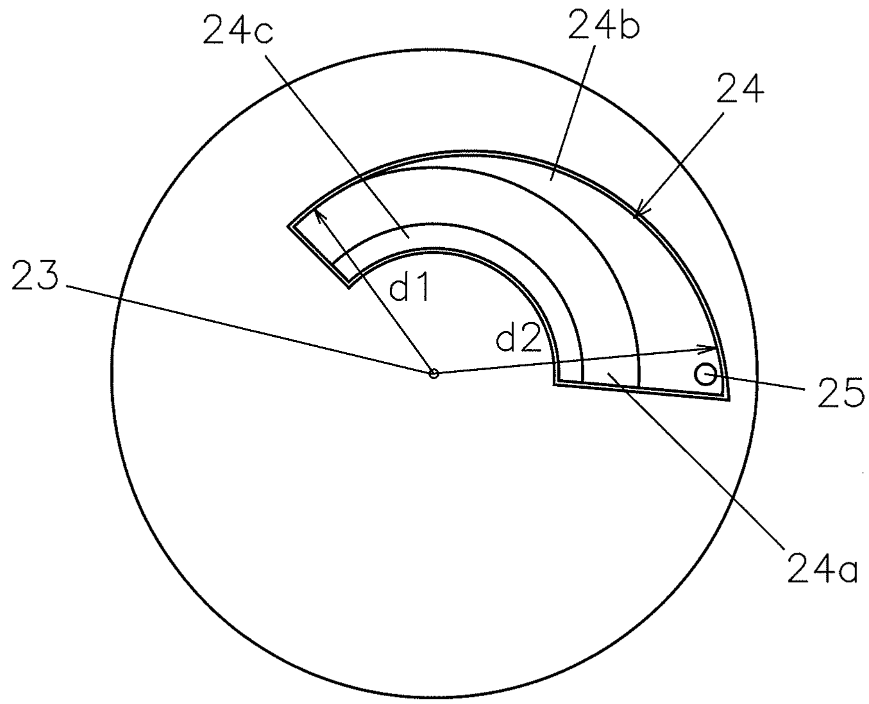


FIG. 24

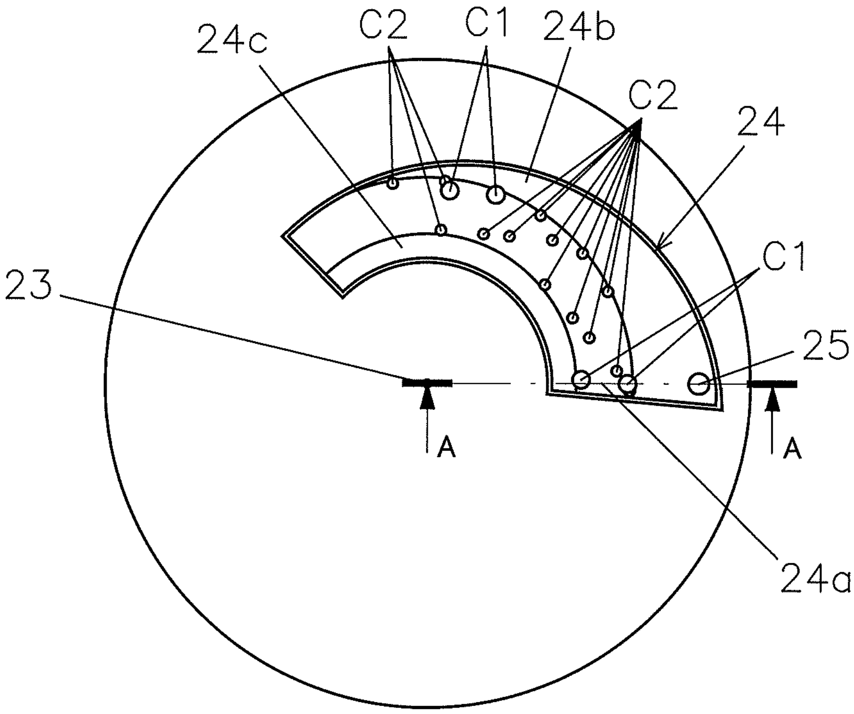


FIG. 25A

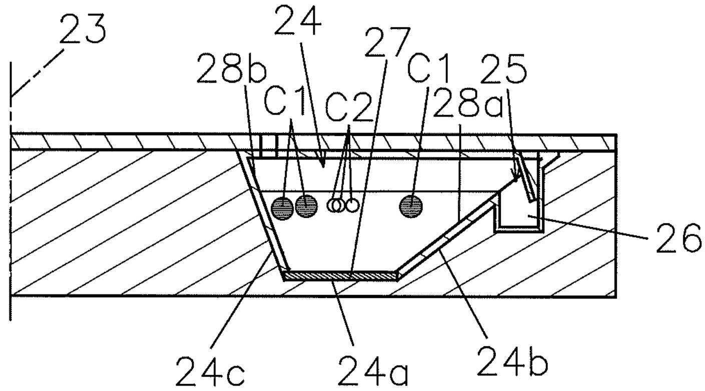


FIG. 25B

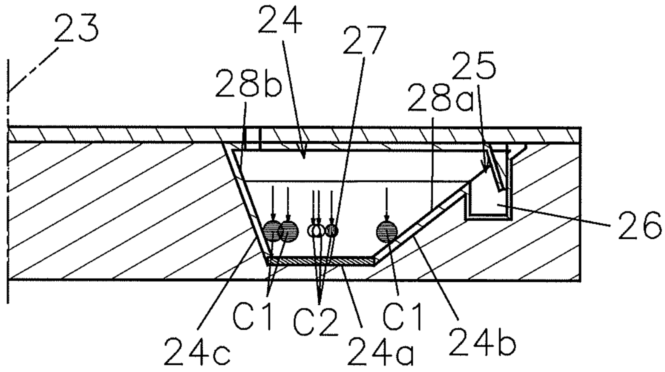


FIG. 26A

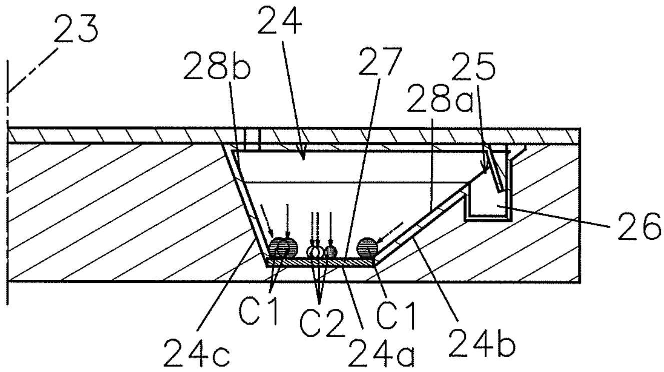


FIG. 26B

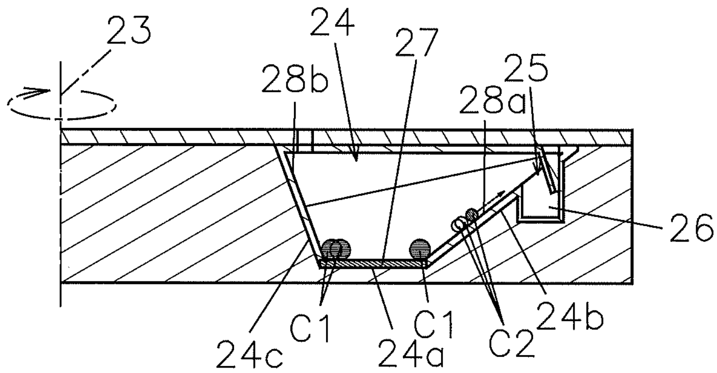


FIG. 26C

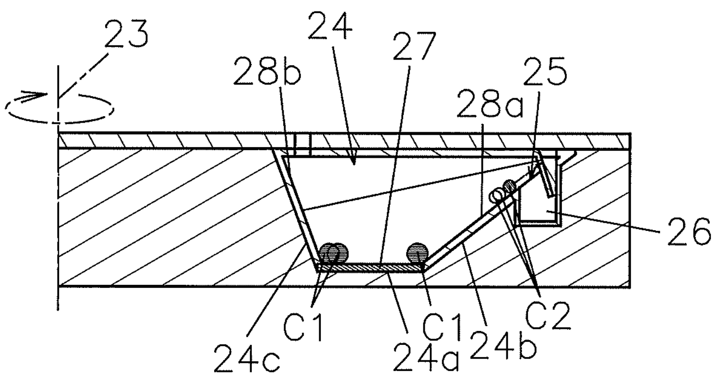


FIG. 26D

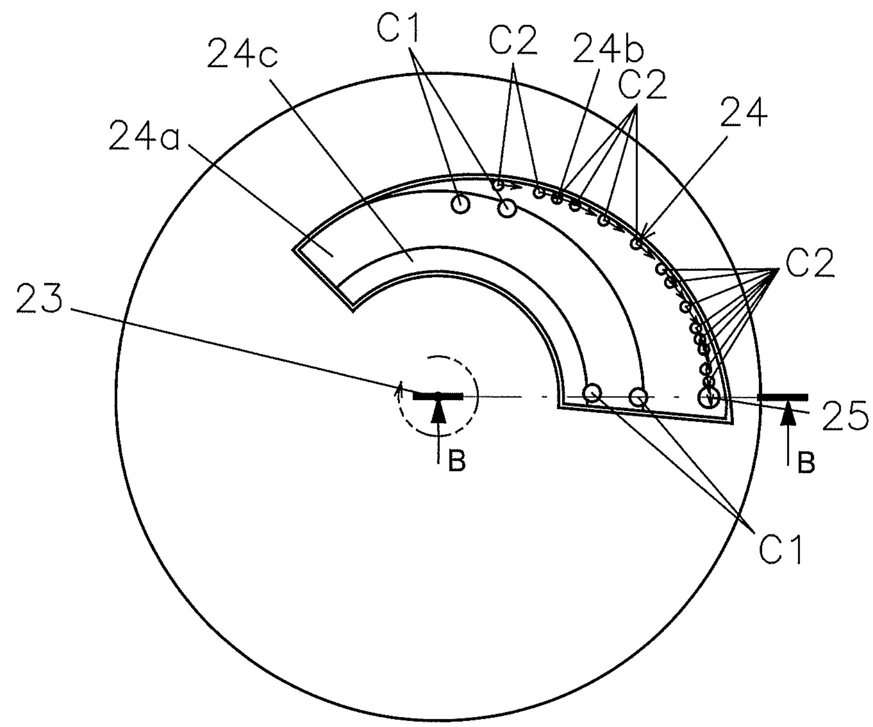


FIG. 27A

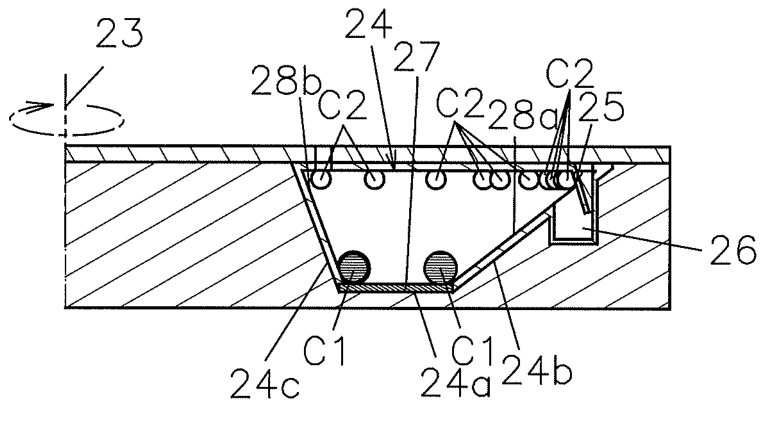


FIG. 27B

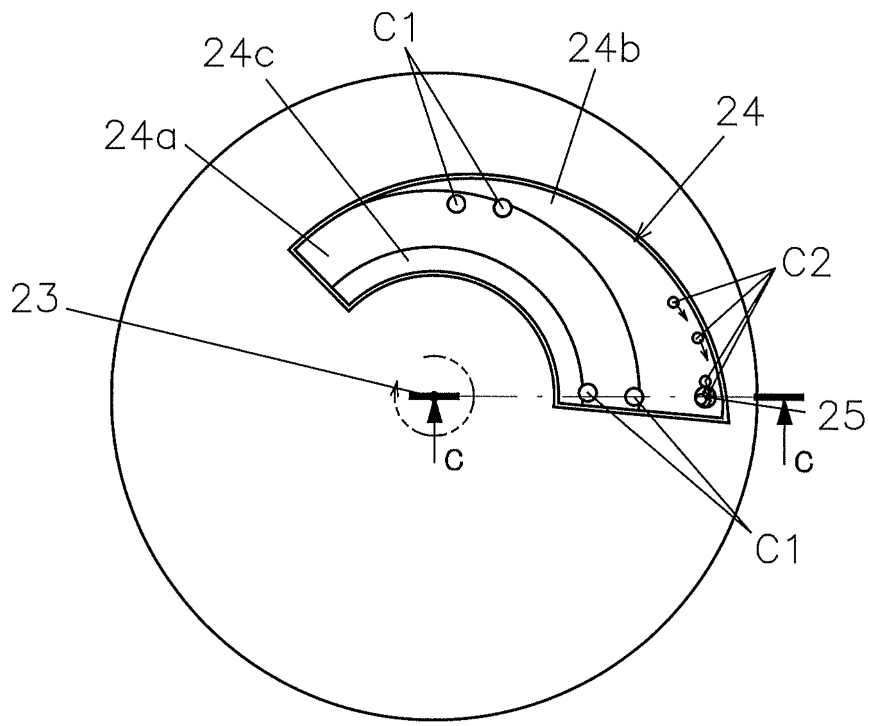


FIG. 28A

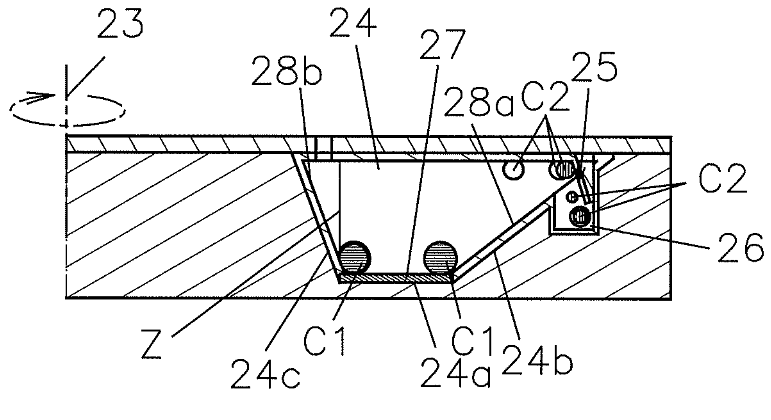


FIG. 28B

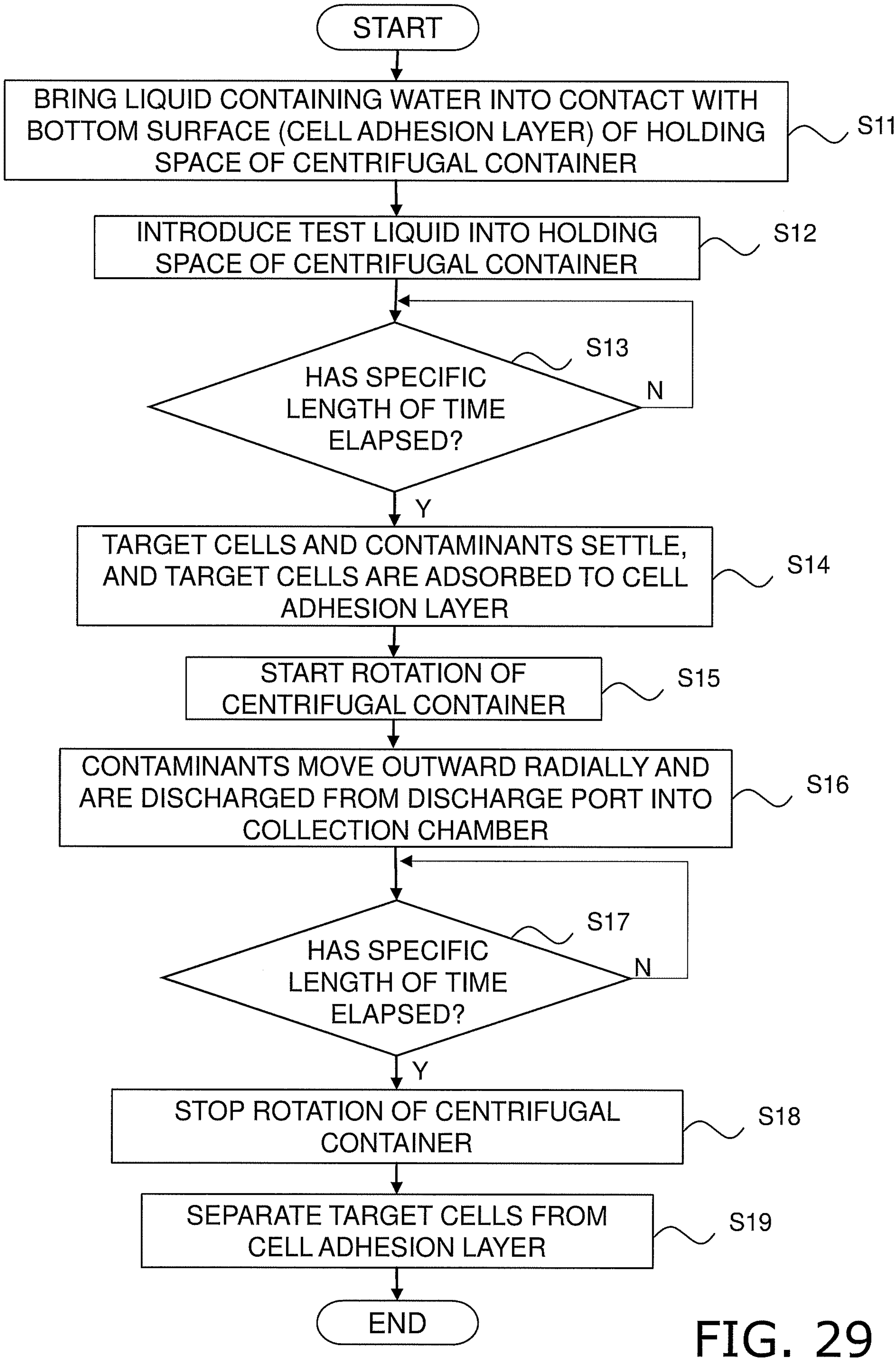
START
BRING LIQUID CONTAINING WATER INTO CONTACT WITH BOTTOM SURFACE (CELL ADHESION LAYER) OF HOLDING SPACE OF CENTRIFUGAL CONTAINER
S11
INTRODUCE TEST LIQUID INTO HOLDING SPACE OF CENTRIFUGAL CONTAINER
S12
HAS SPECIFIC LENGTH OF TIME ELAPSED?
S13
N
Y
TARGET CELLS AND CONTAMINANTS SETTLE, AND TARGET CELLS ARE ADSORBED TO CELL ADHESION LAYER
S14
START ROTATION OF CENTRIFUGAL CONTAINER
S15
CONTAMINANTS MOVE OUTWARD RADIALLY AND ARE DISCHARGED FROM DISCHARGE PORT INTO COLLECTION CHAMBER
S16
HAS SPECIFIC LENGTH OF TIME ELAPSED?
S17
N
Y
STOP ROTATION OF CENTRIFUGAL CONTAINER
S18
SEPARATE TARGET CELLS FROM CELL ADHESION LAYER
S19
END

FIG. 29

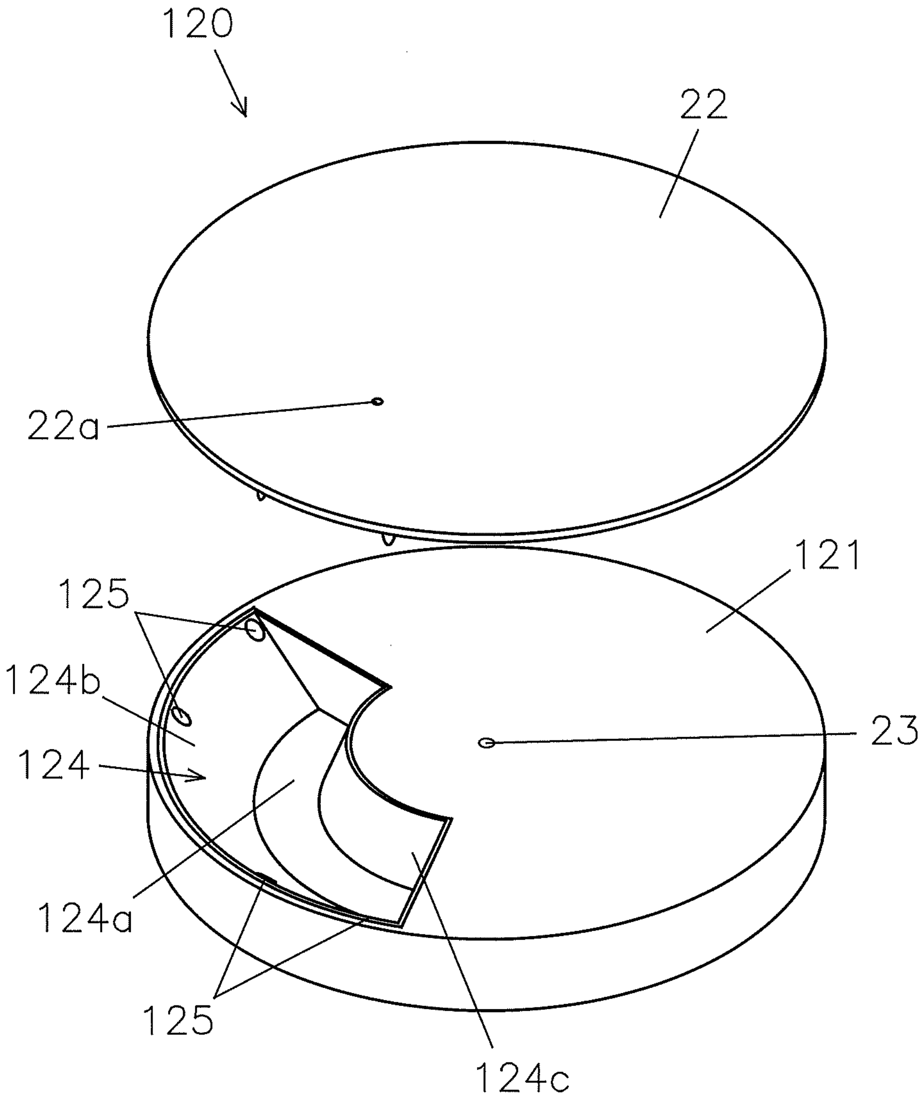


FIG. 30

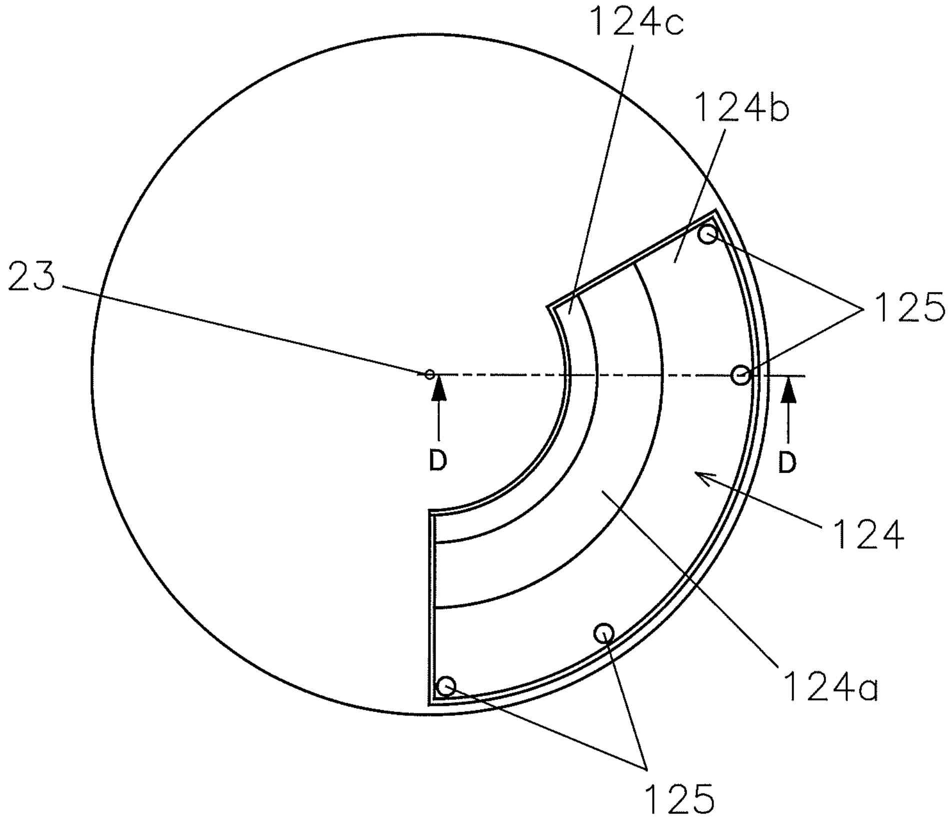


FIG. 31A

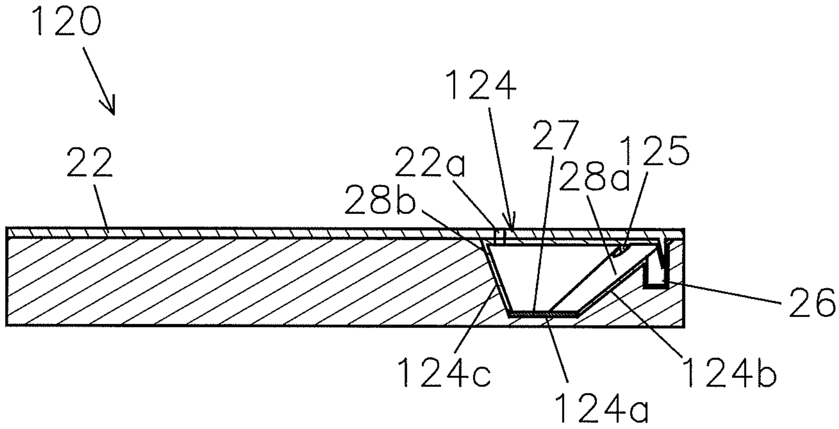


FIG. 31B

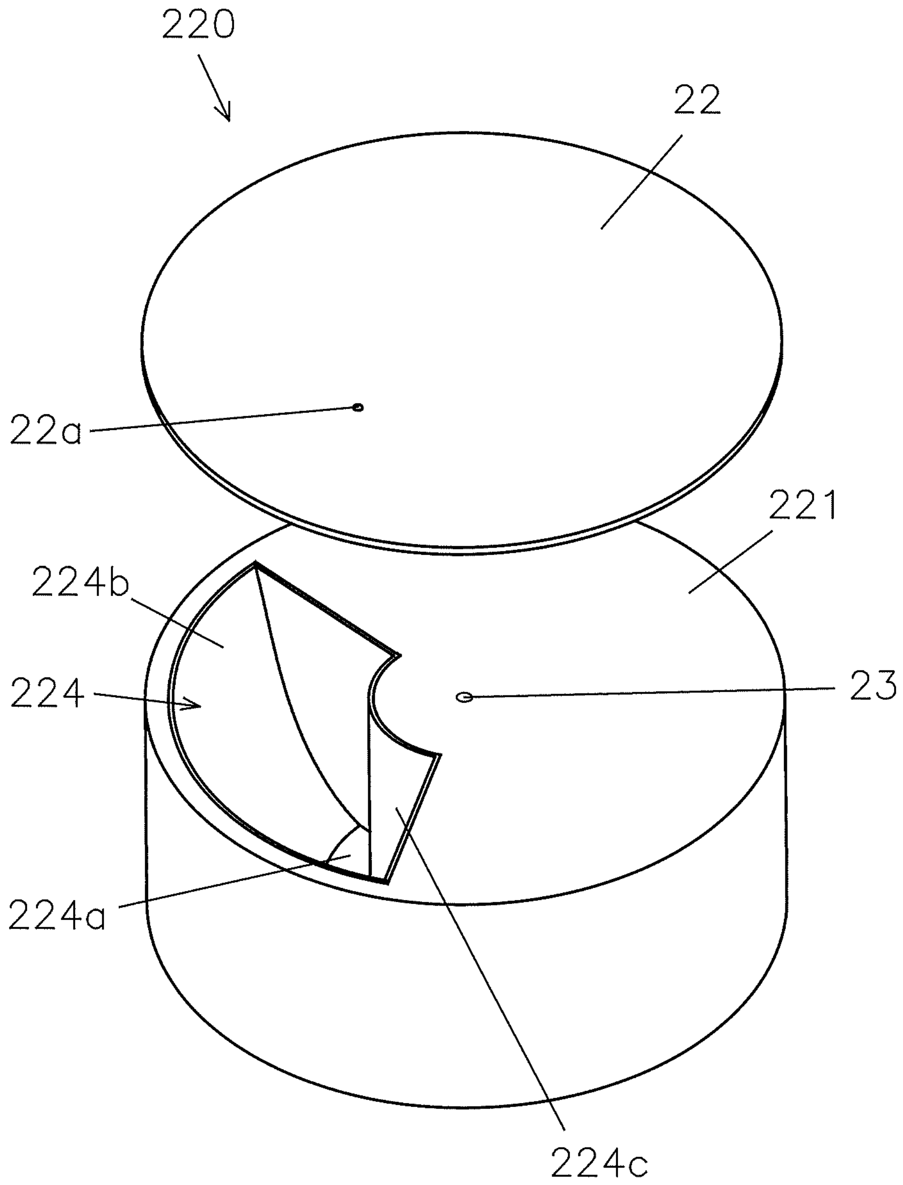


FIG. 32

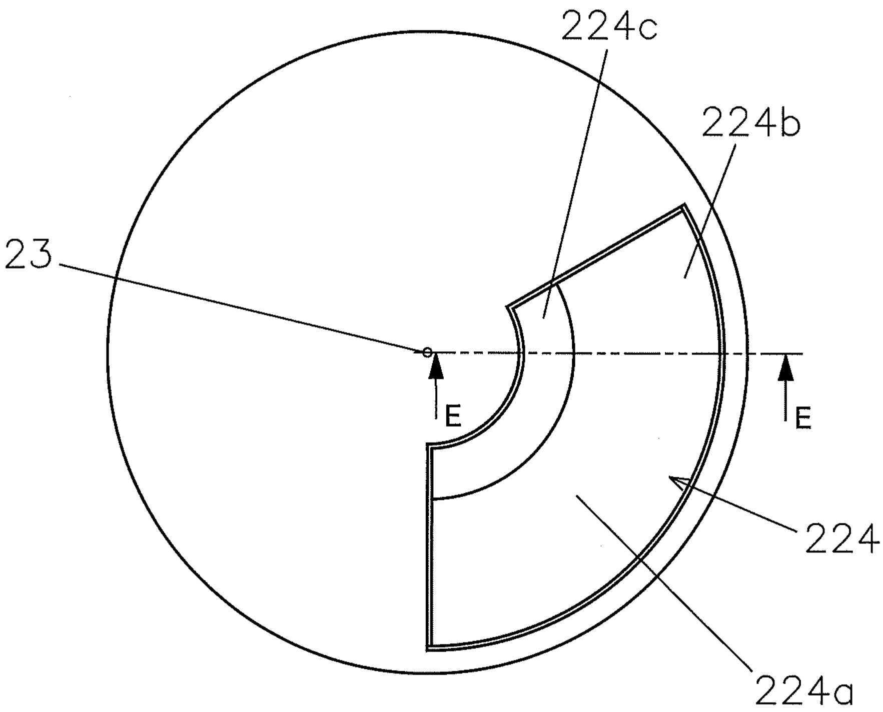


FIG. 33A

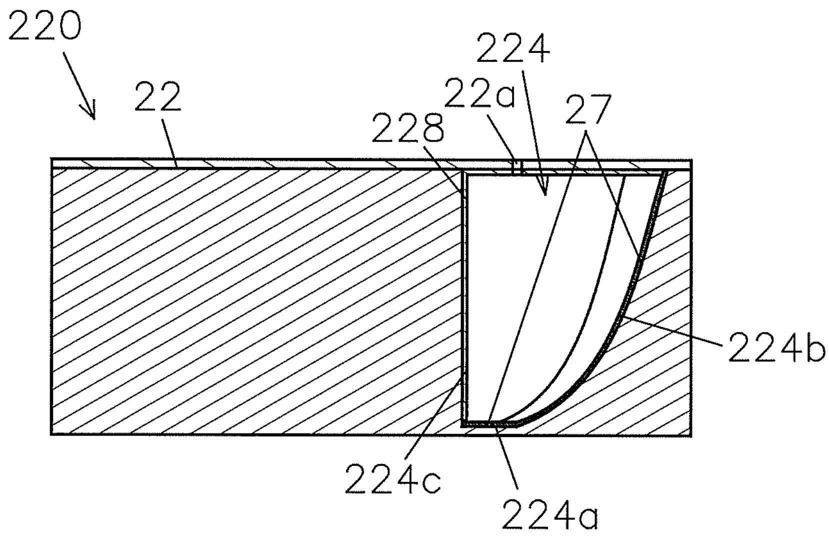


FIG. 33B

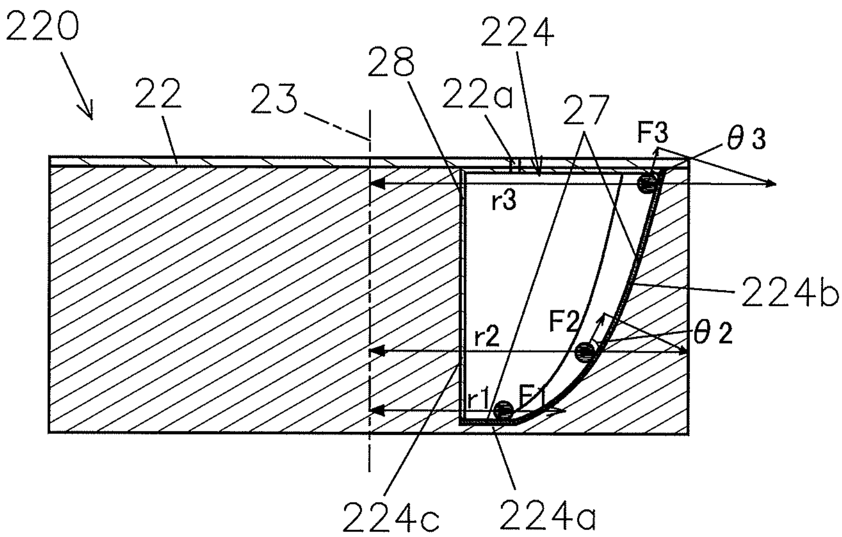


FIG. 34

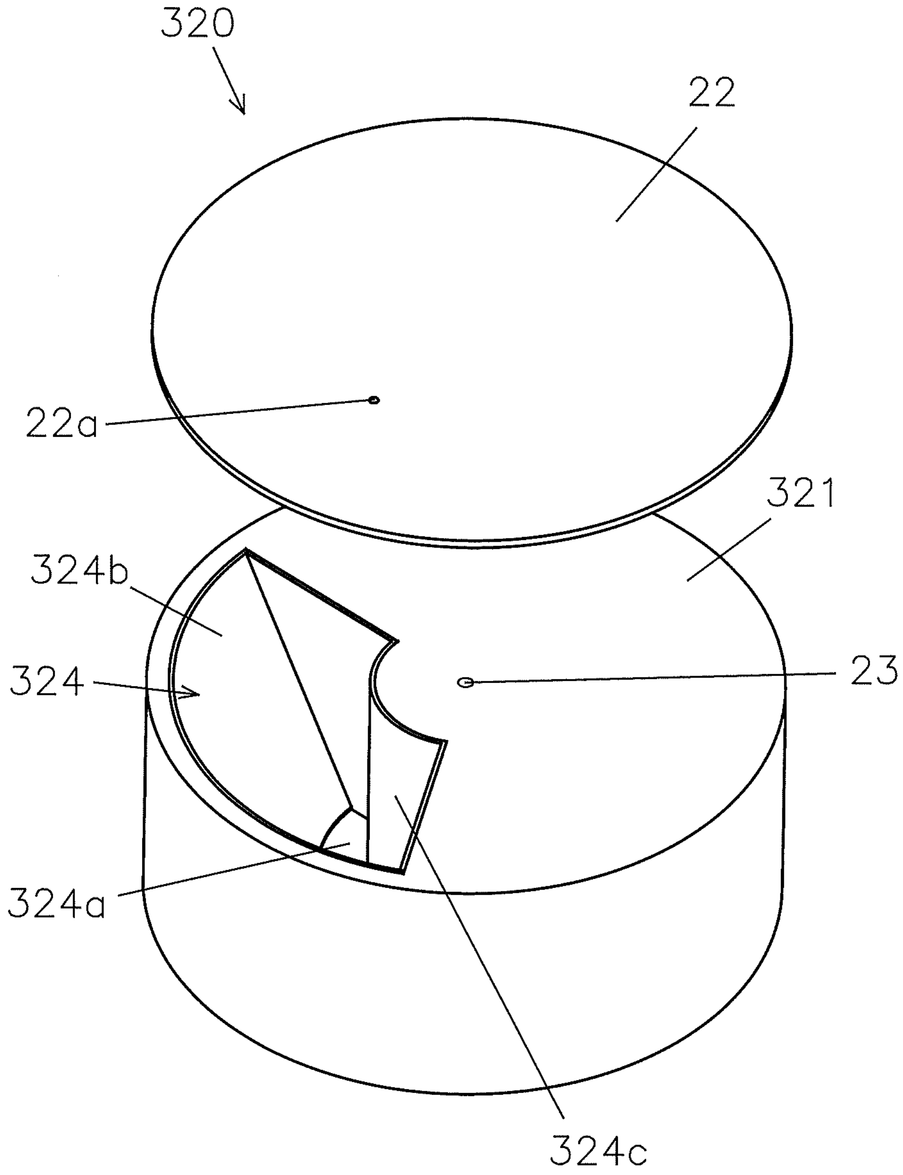


FIG. 35

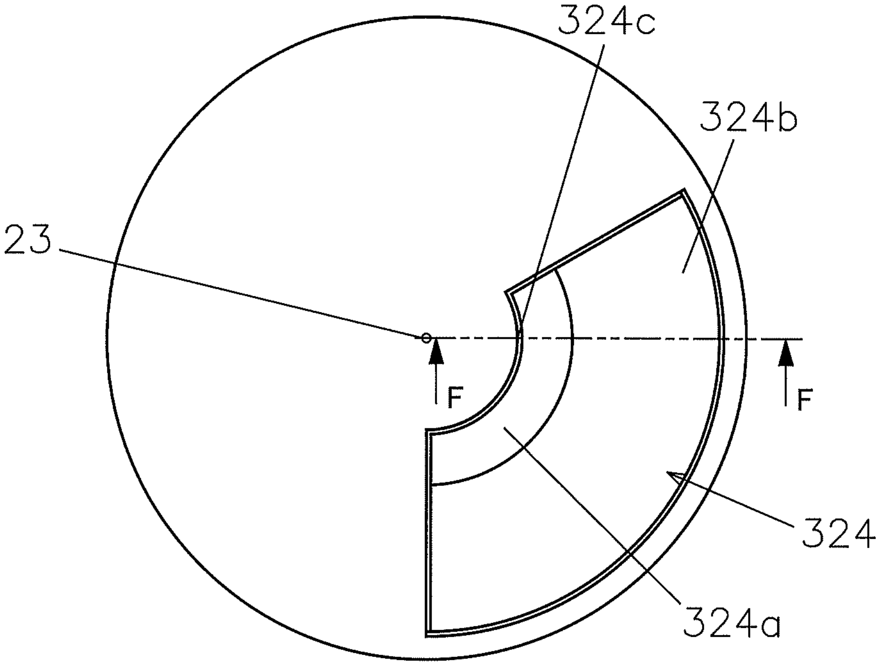


FIG. 36A

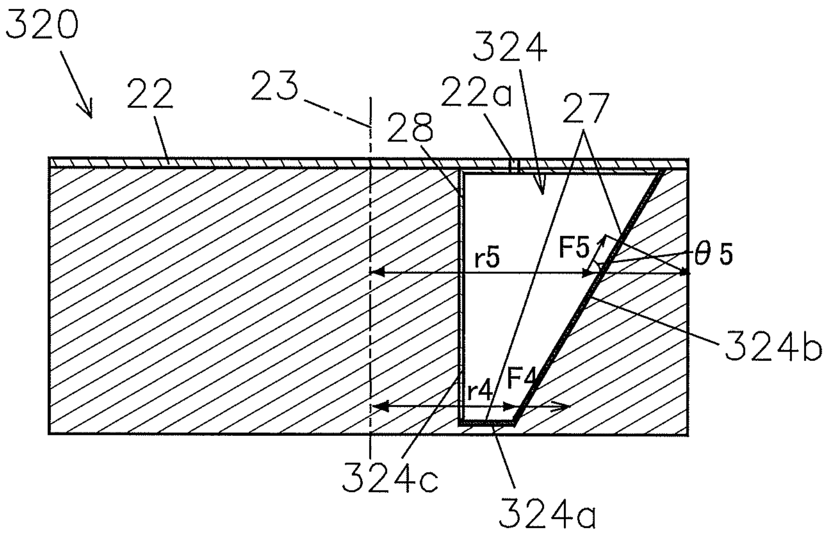


FIG. 36B

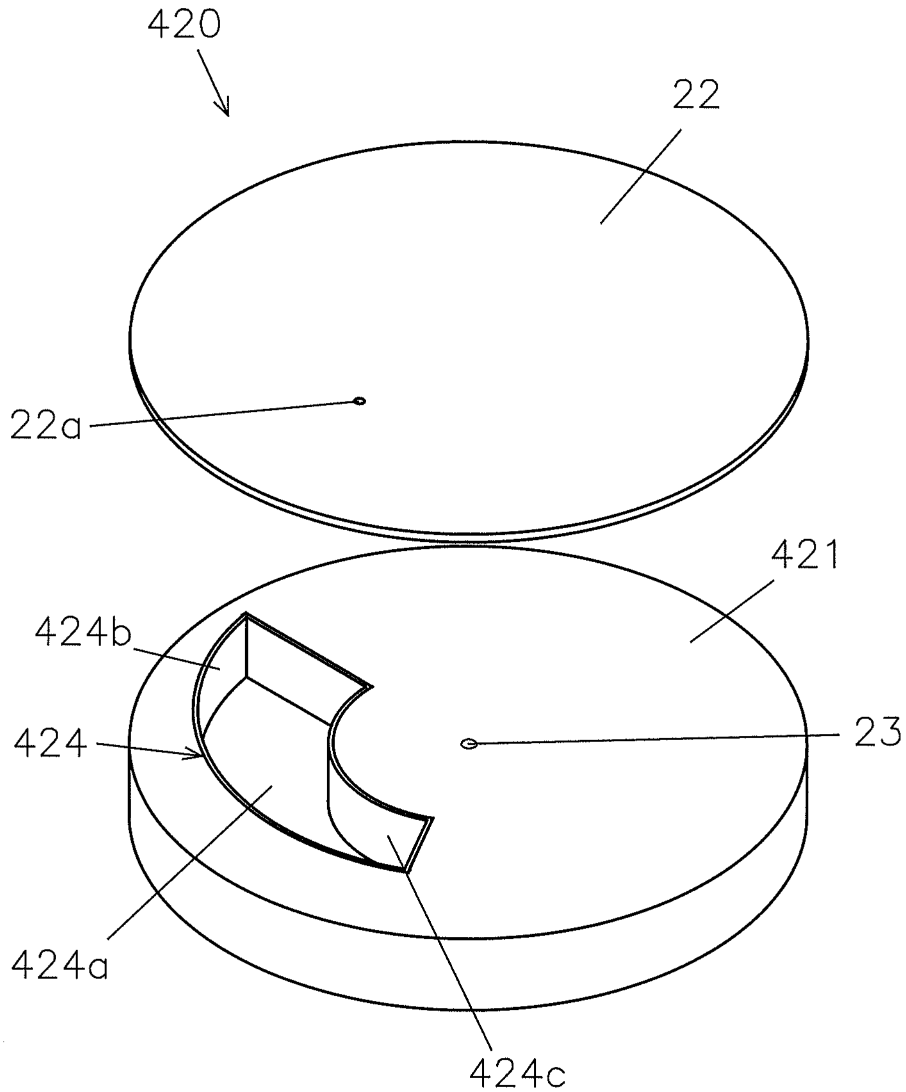


FIG. 37

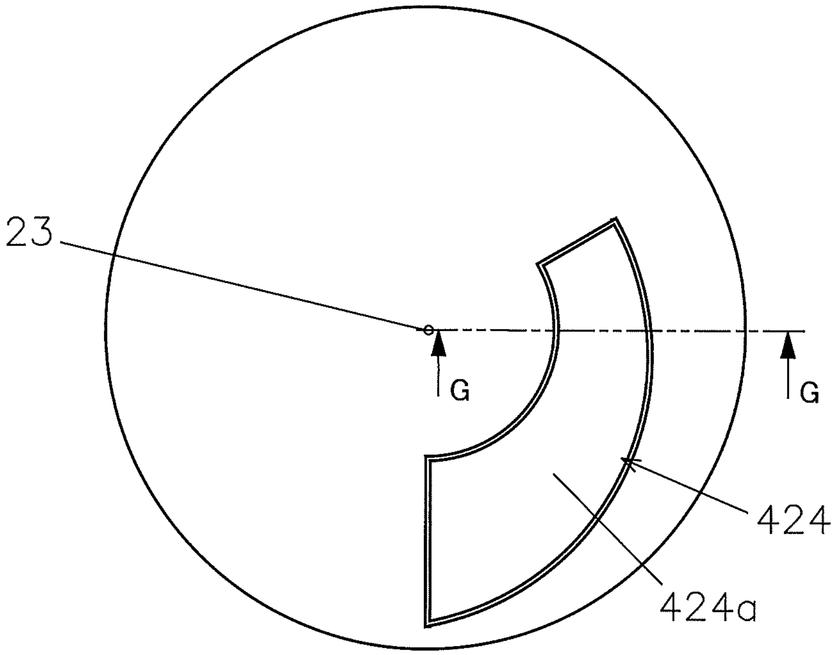


FIG. 38A

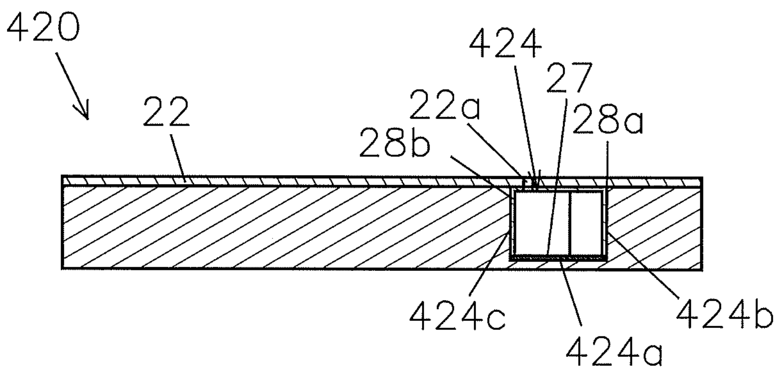


FIG. 38B

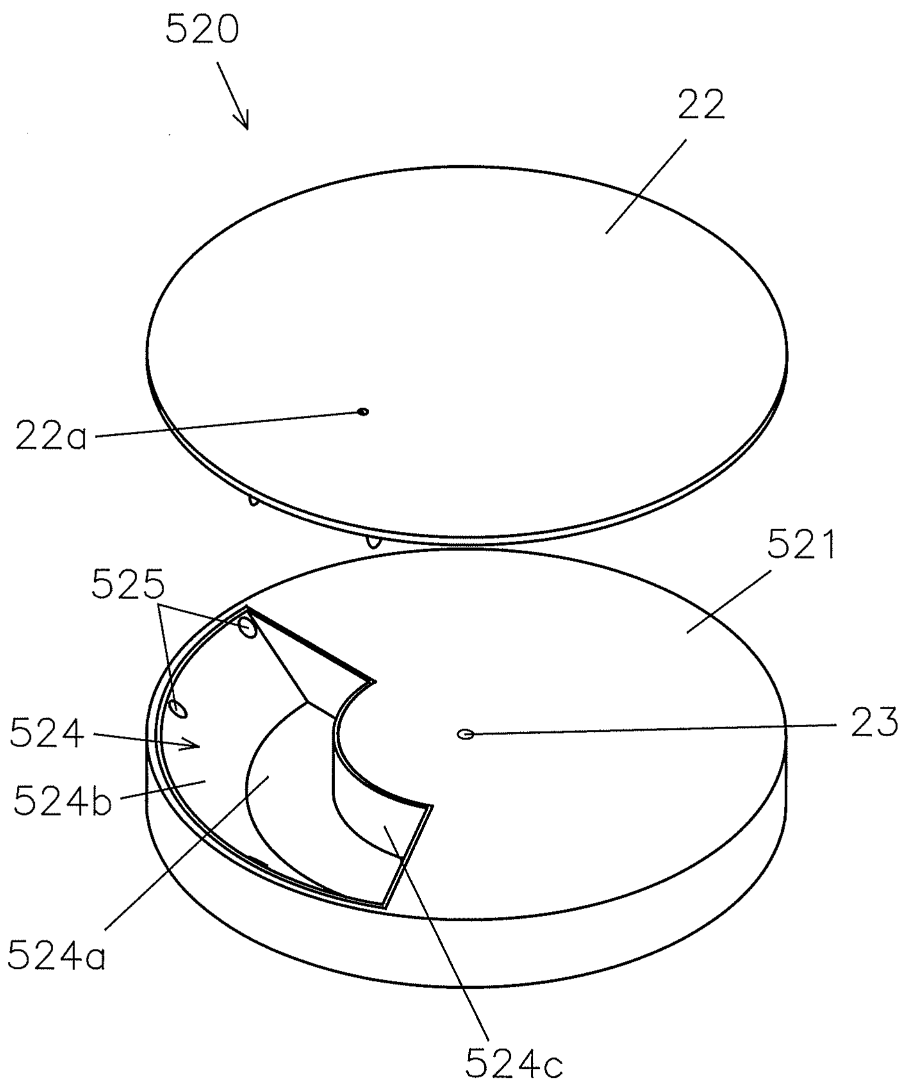


FIG. 39

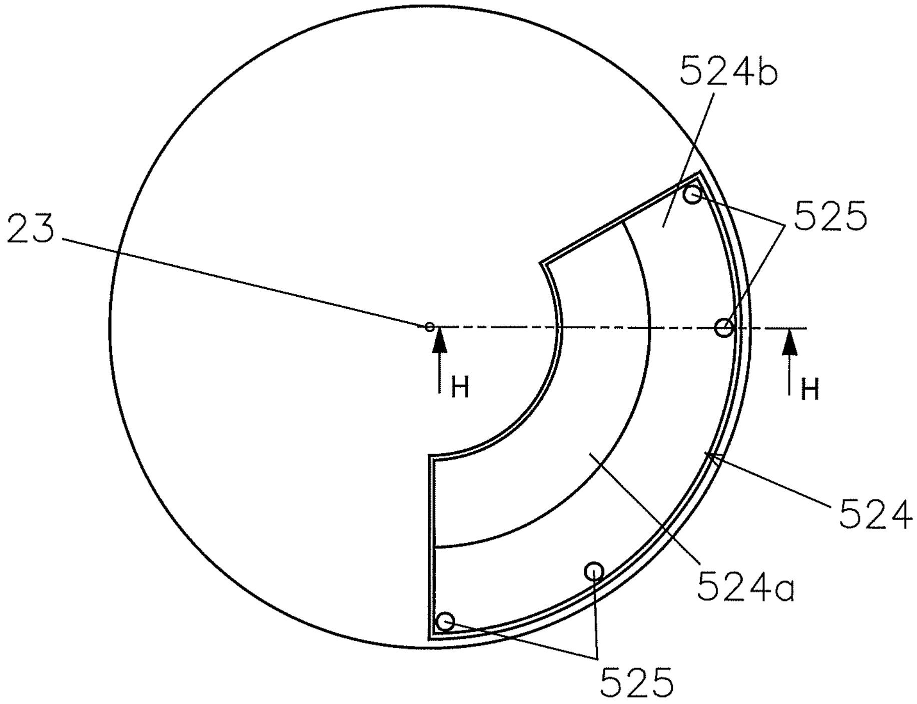


FIG. 40A

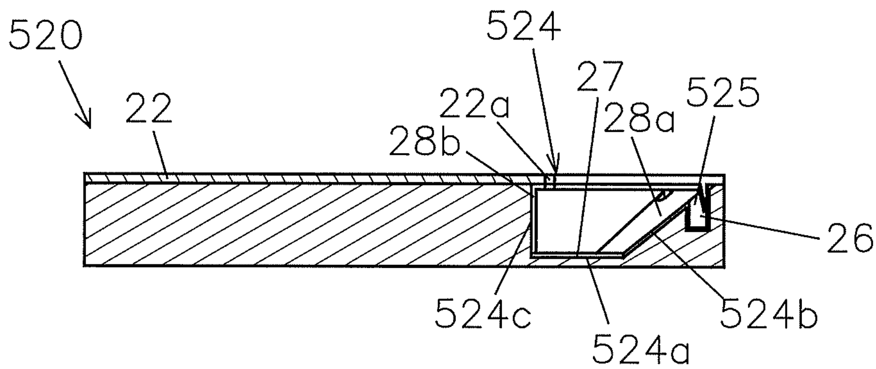


FIG. 40B

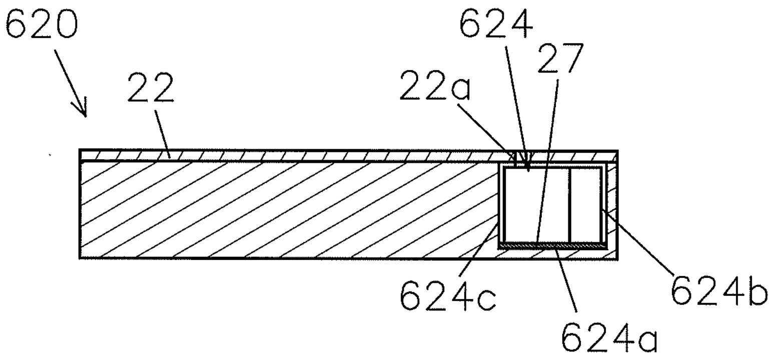


FIG. 41A

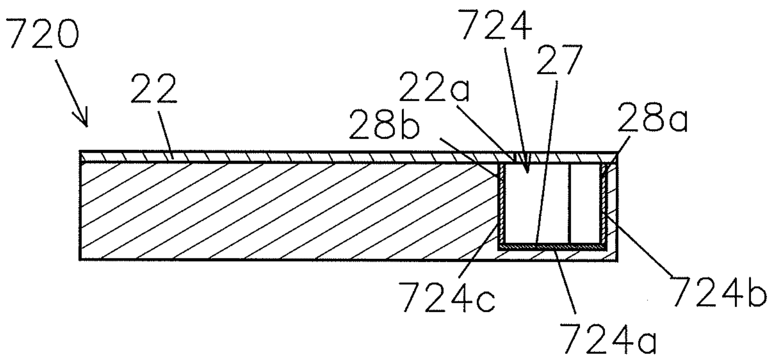


FIG. 41B

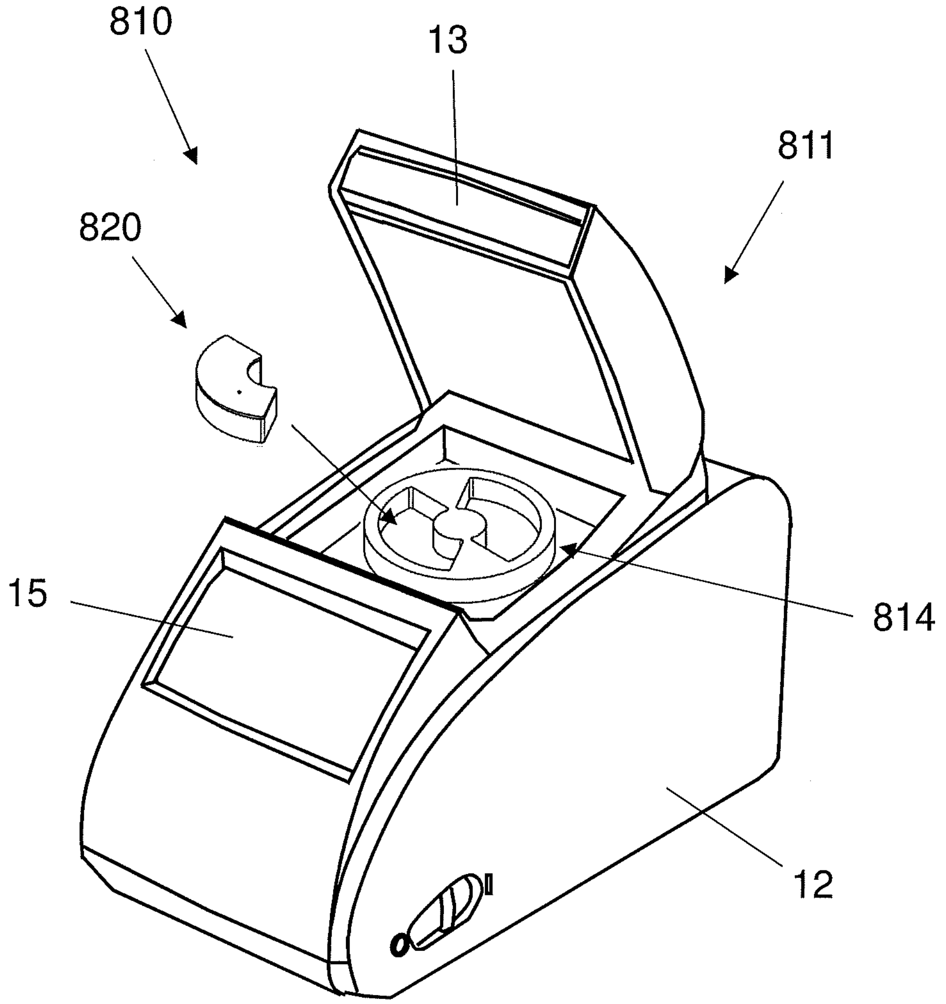


FIG. 42

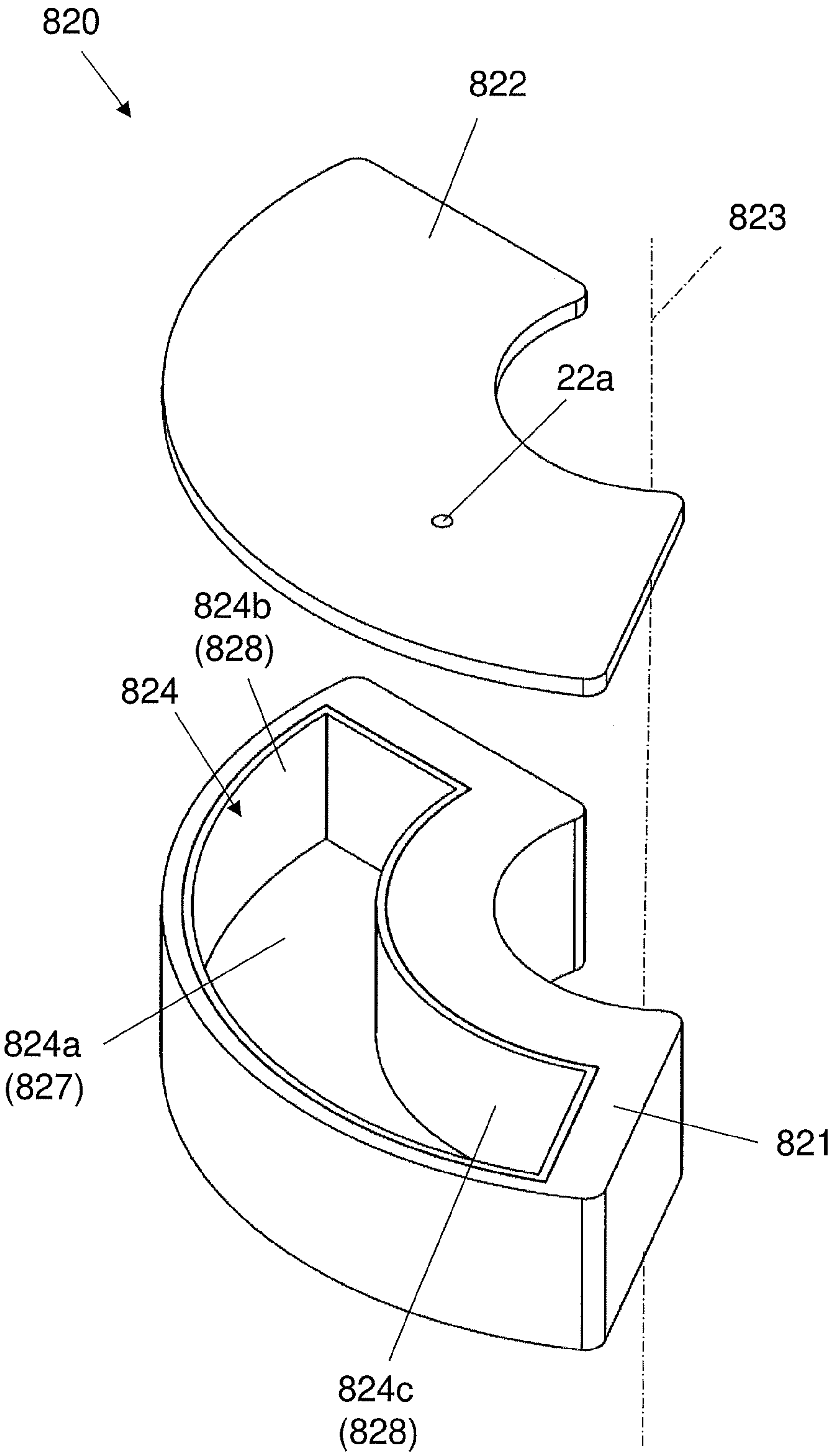


FIG. 43

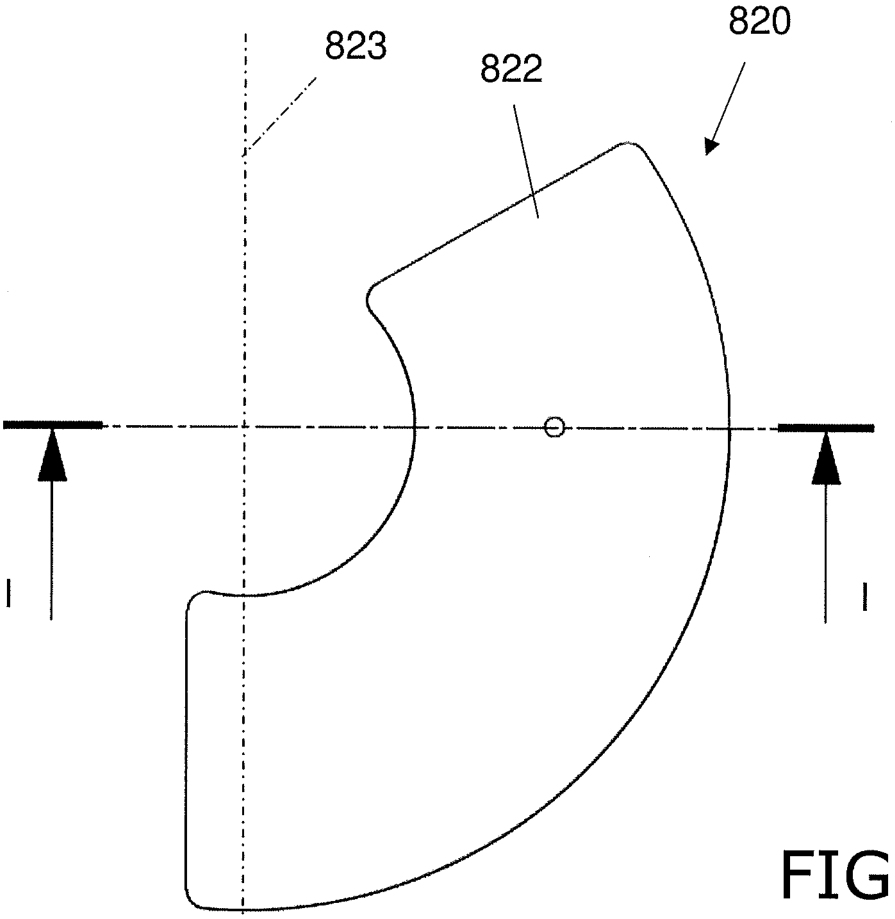


FIG. 44A

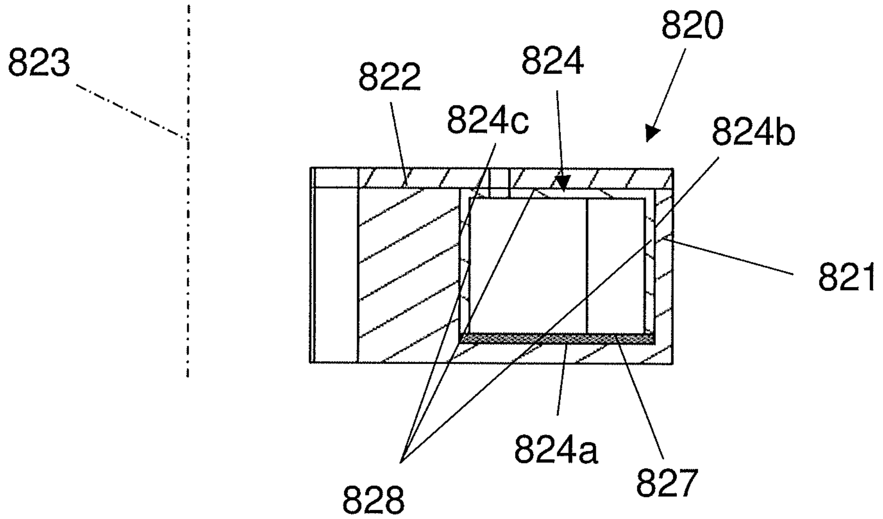


FIG. 44B

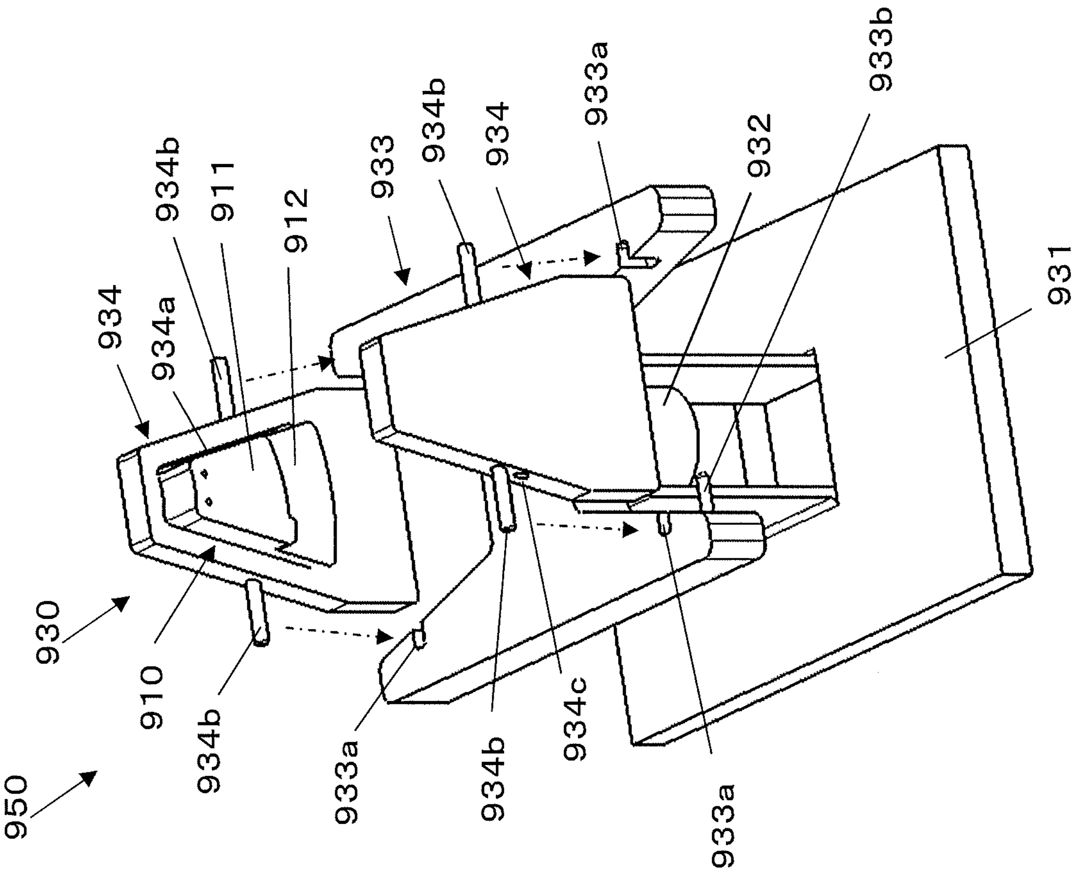
950
930
910
934b
933a
934b
934c
933a
934
934a
934b
911
912
933
934b
934
933a
932
933b
931

FIG. 45

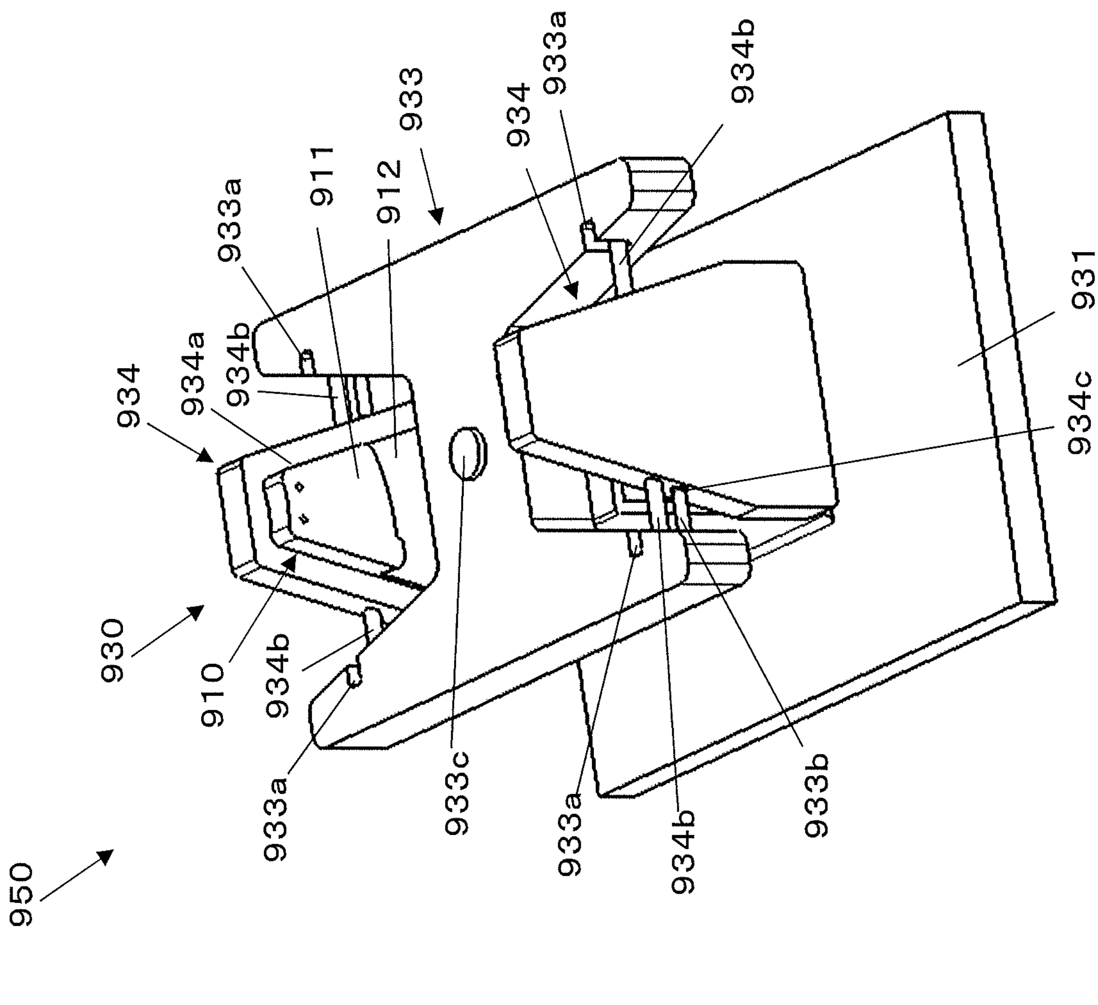
950
930
910
934b
933a
934
934a
934b
933a
911
912
933
934
933a
934b
931
934c
933c
933a
934b
933b

FIG. 46

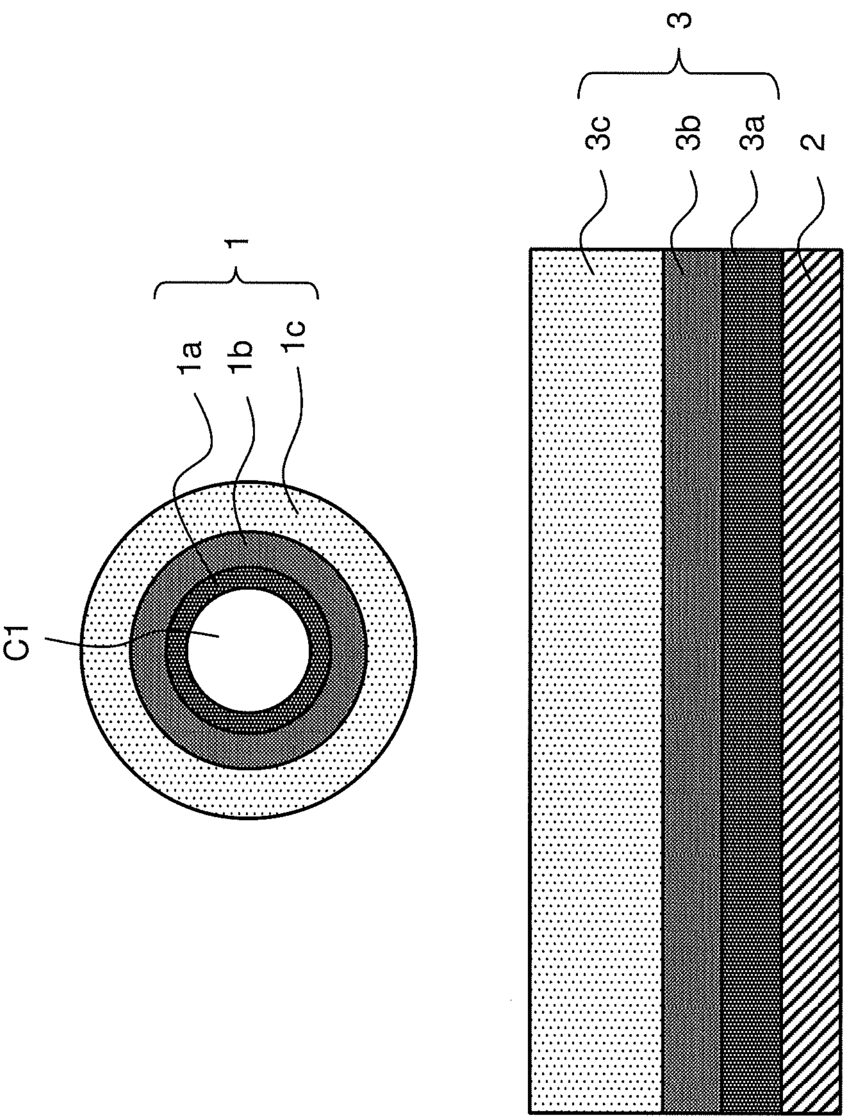


FIG. 47

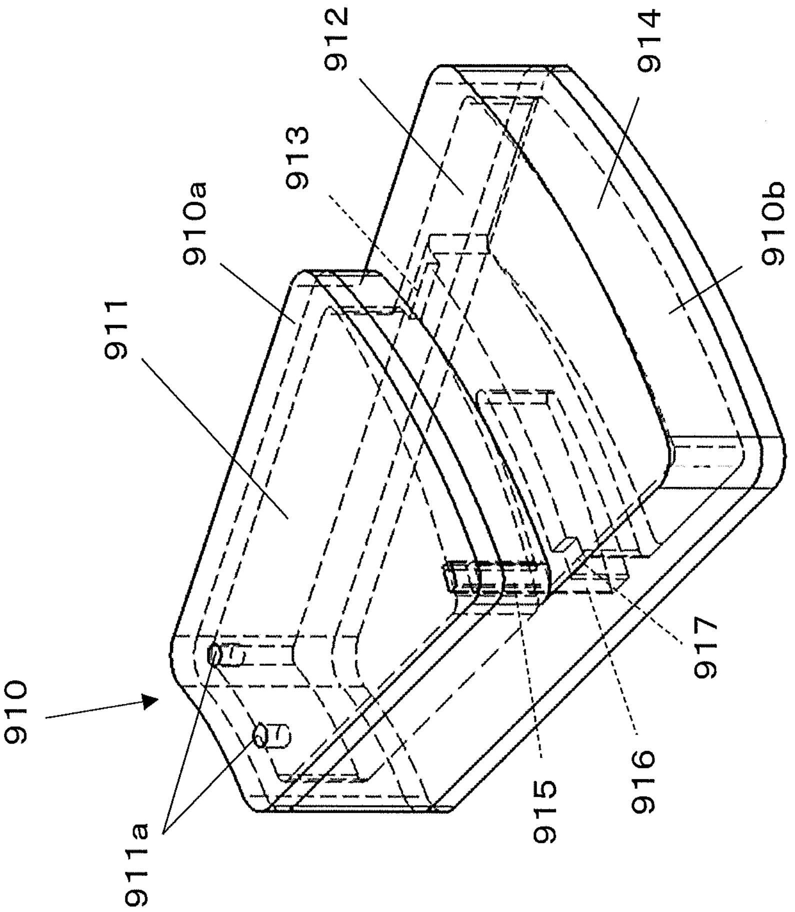


FIG. 48

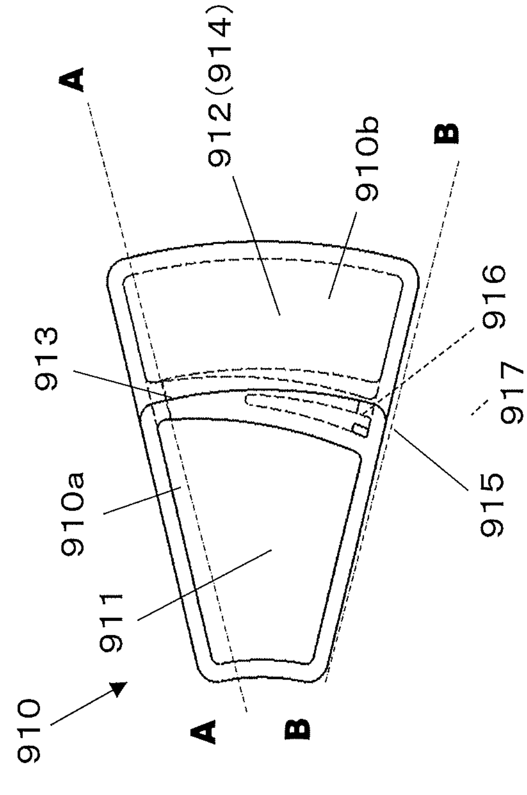


FIG. 49A

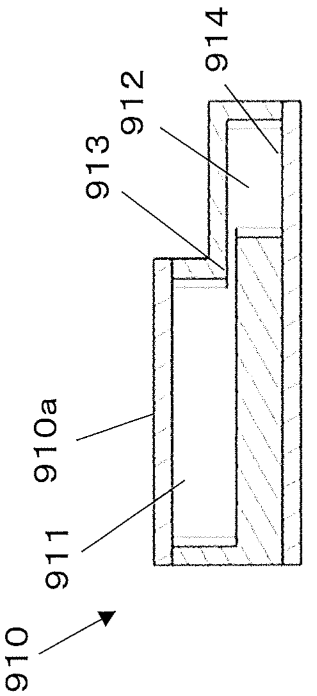


A-A CROSS-SECTIONAL VIEW

FIG. 49B

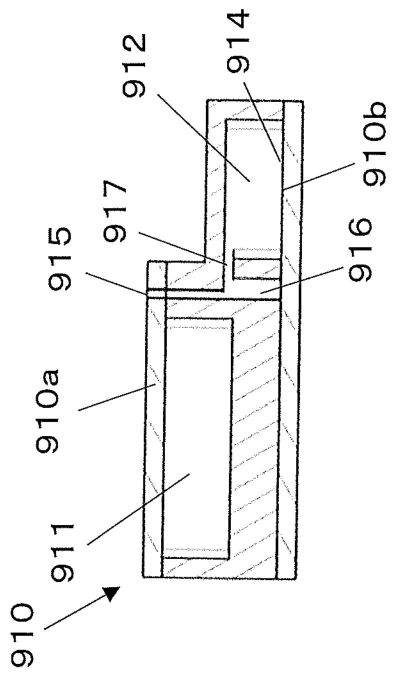


B-B CROSS-SECTIONAL VIEW

FIG. 49C

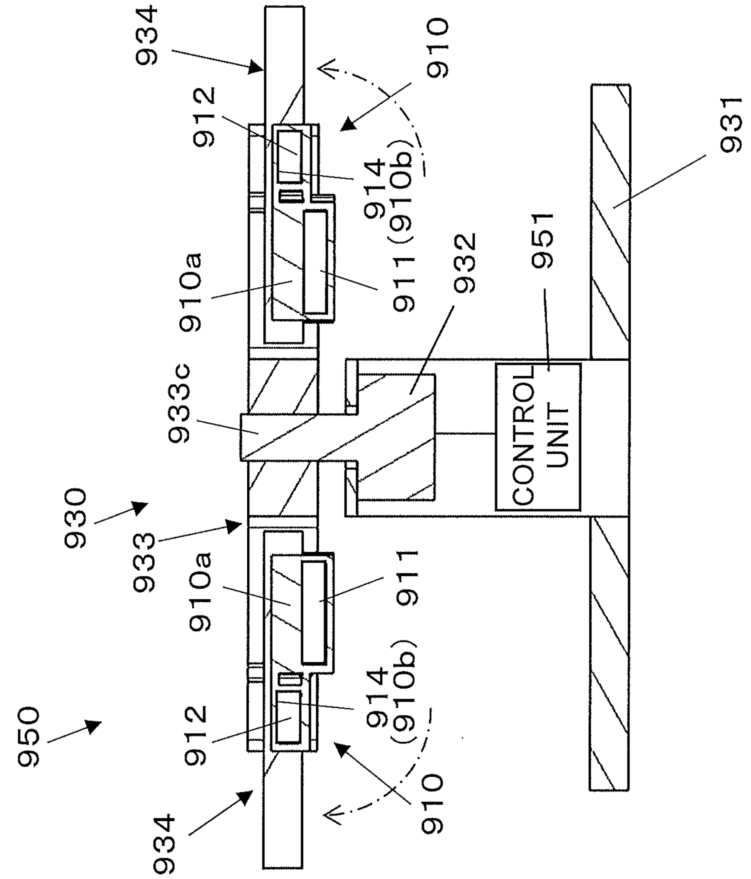


FIG. 50B

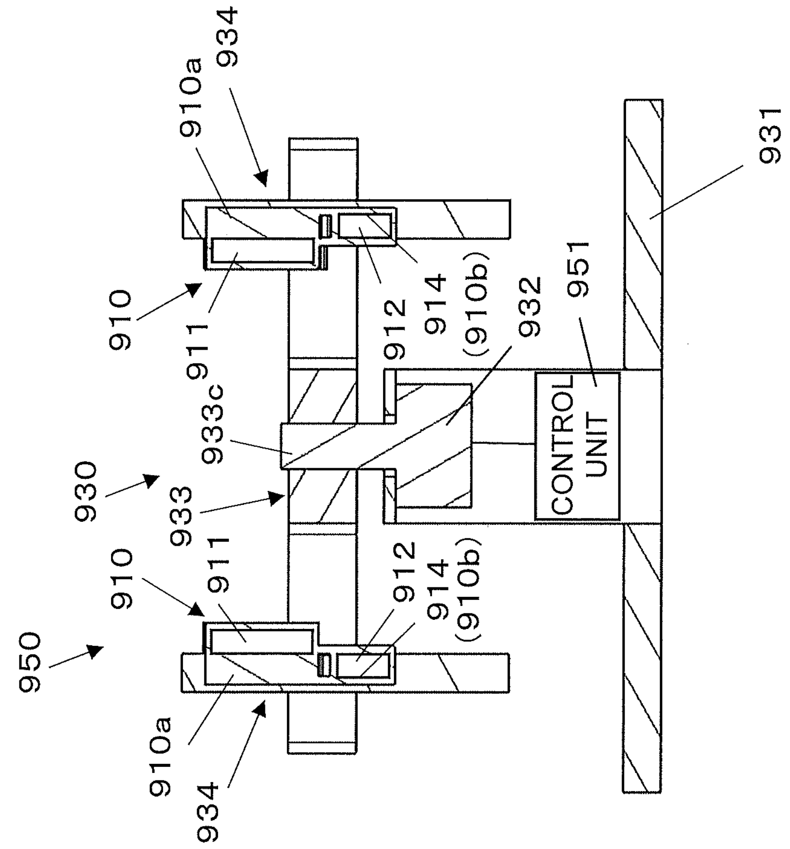


FIG. 50A

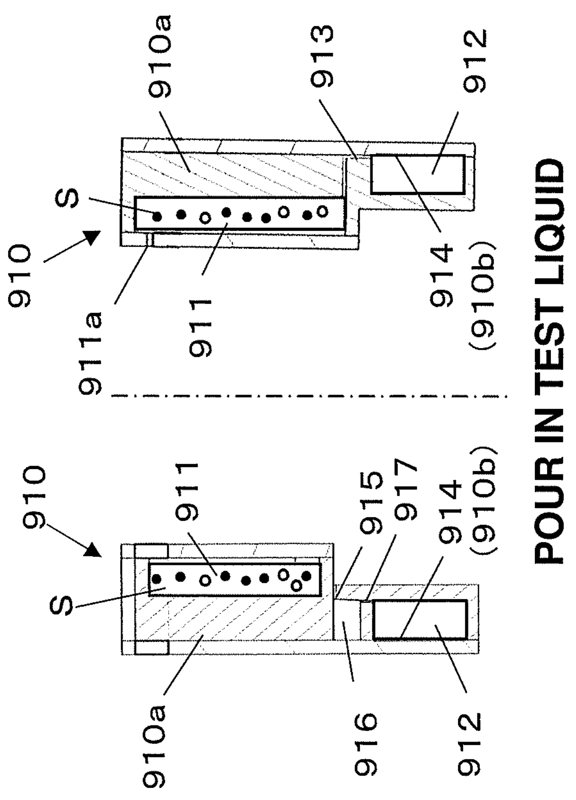


FIG. 51A

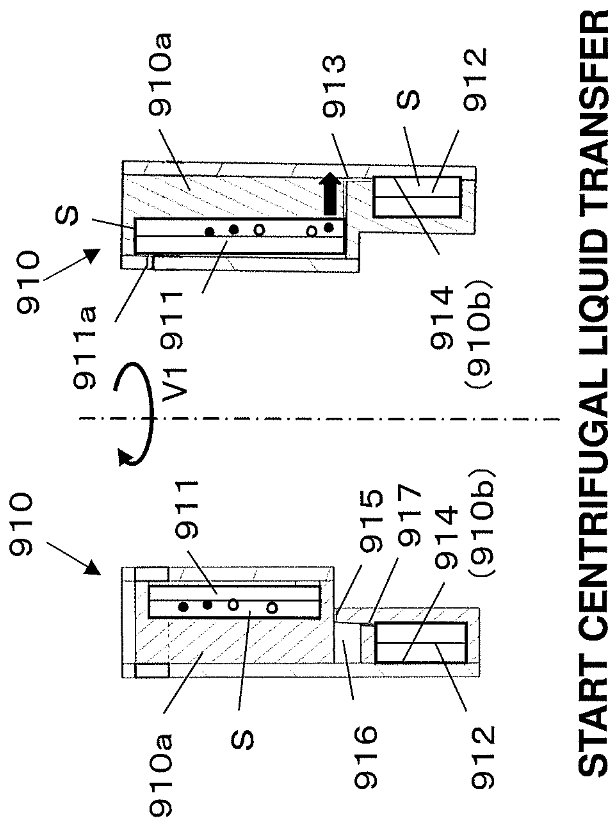


FIG. 51B

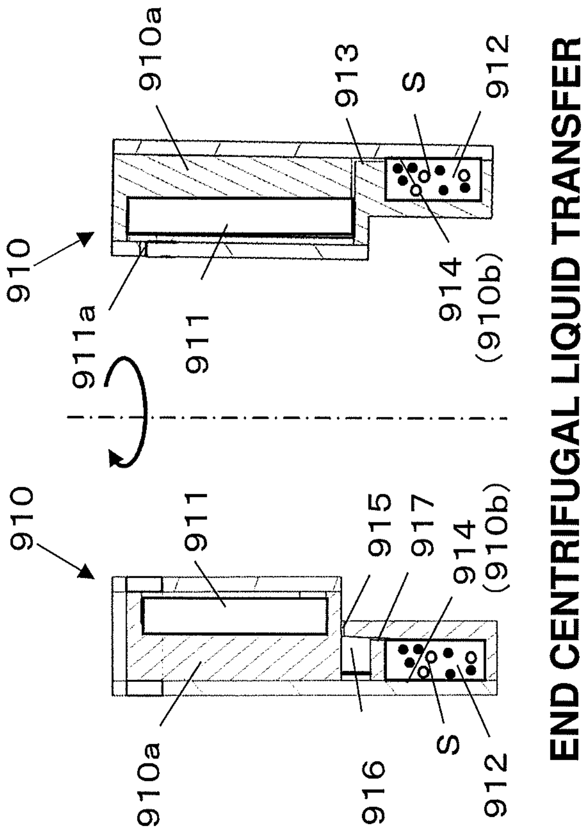


FIG. 52A

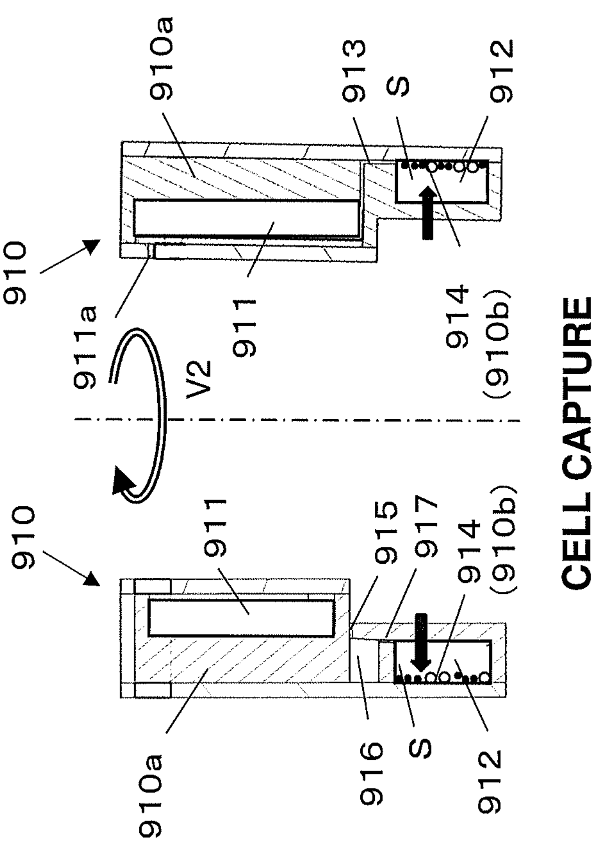


FIG. 52B

FIG. 53A

FIG. 53B

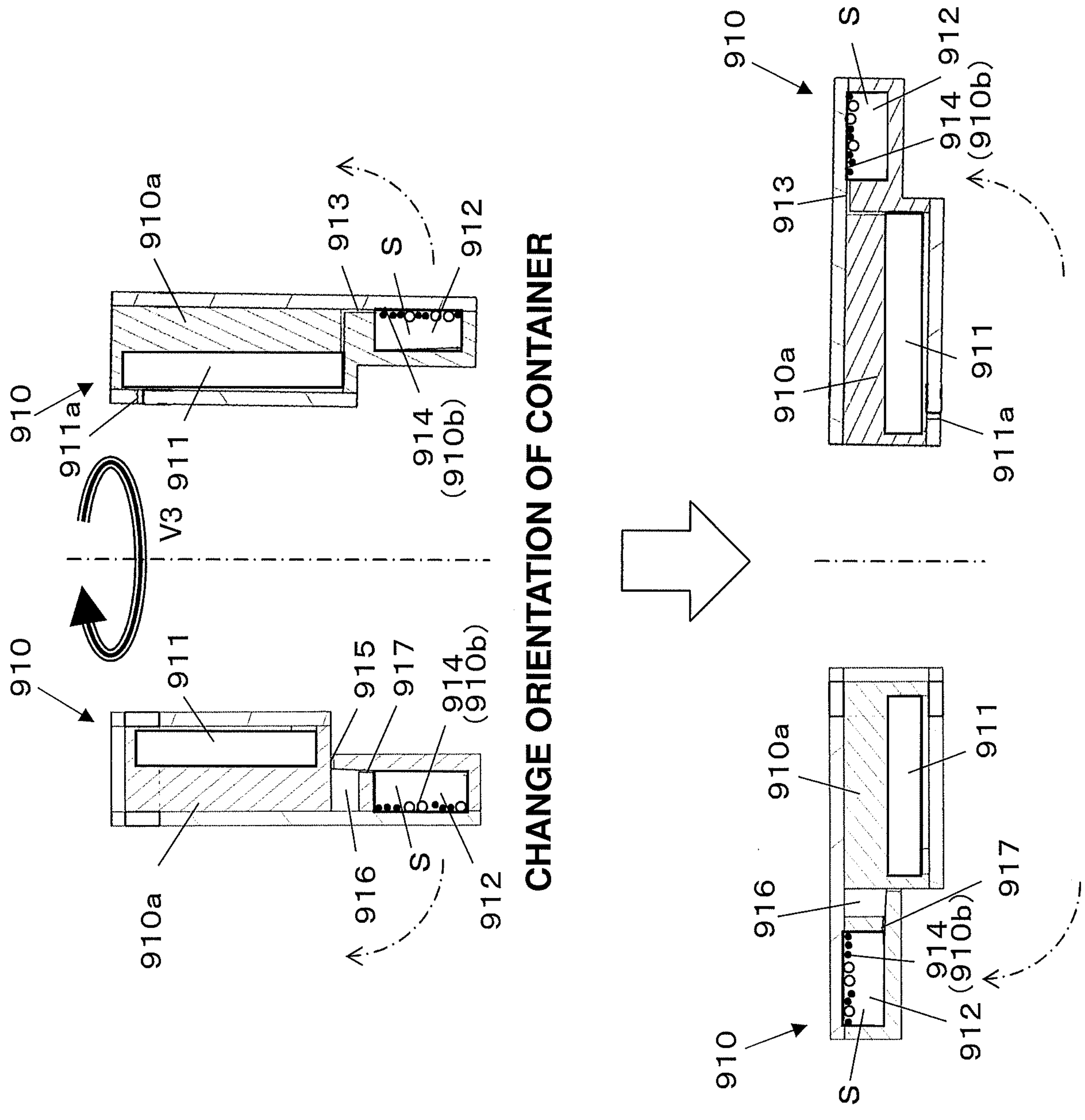

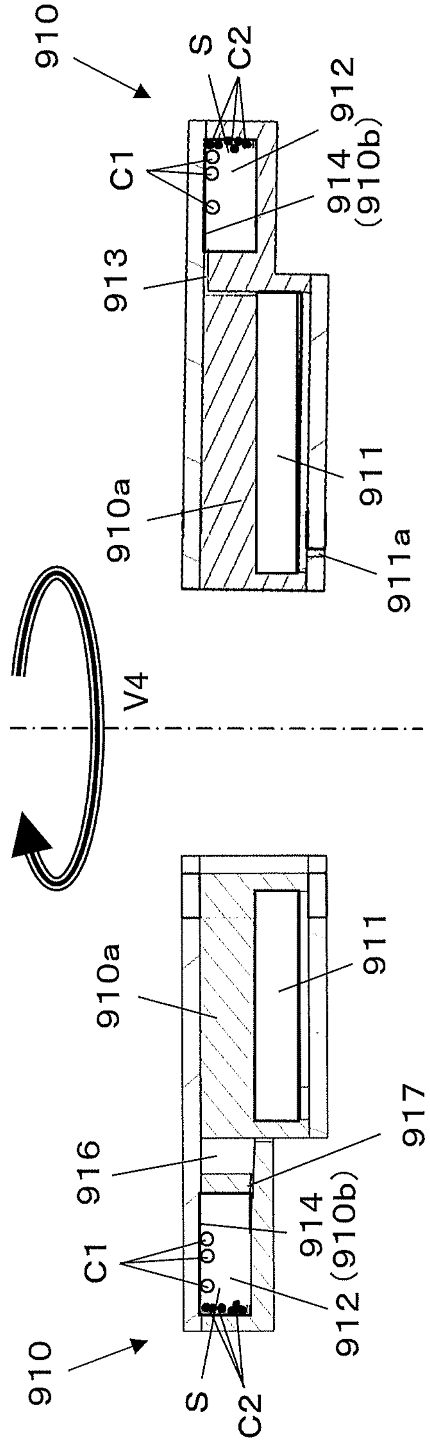


CENTRIFUGAL RINSING

FIG. 54

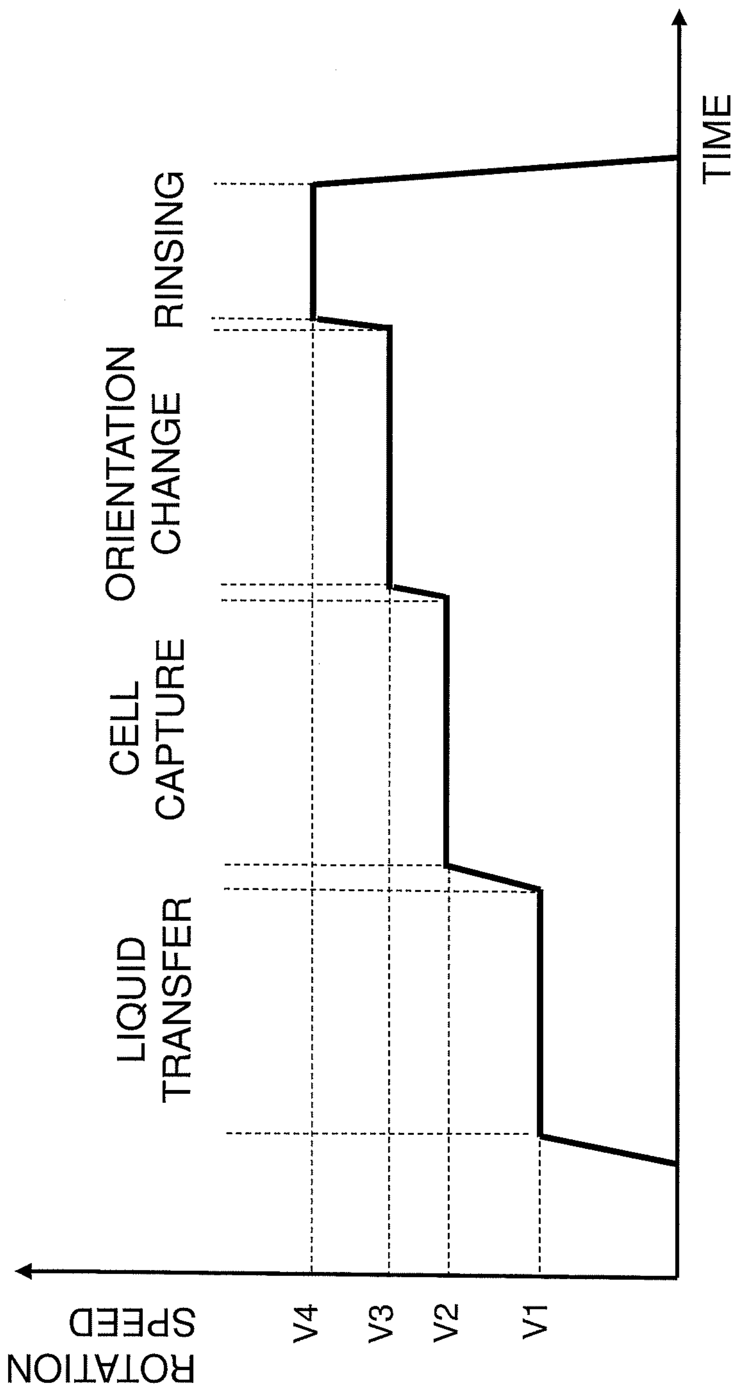
ROTATION SPEED
V4
V3
V2
V1
LIQUID TRANSFER
CELL CAPTURE
ORIENTATION CHANGE
RINSING
TIME

FIG. 55

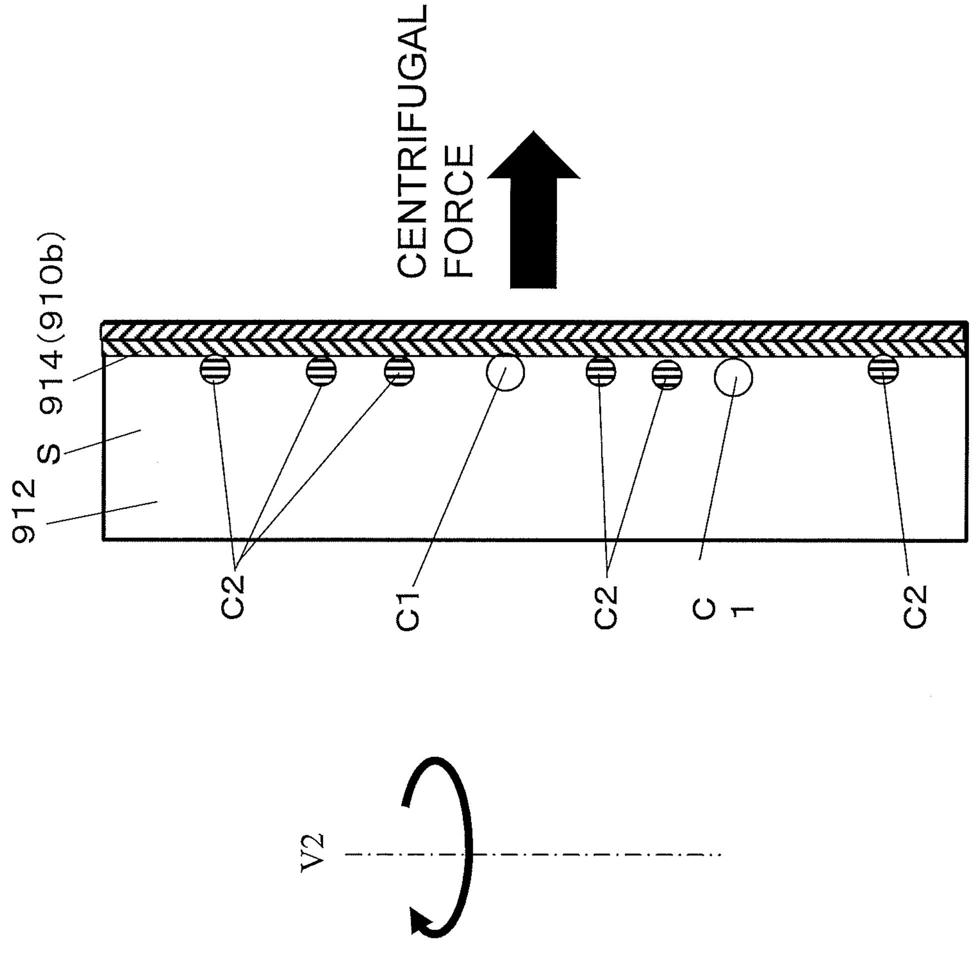


AFTER CENTRIFUGING (CELL CAPTURE)

FIG. 56B

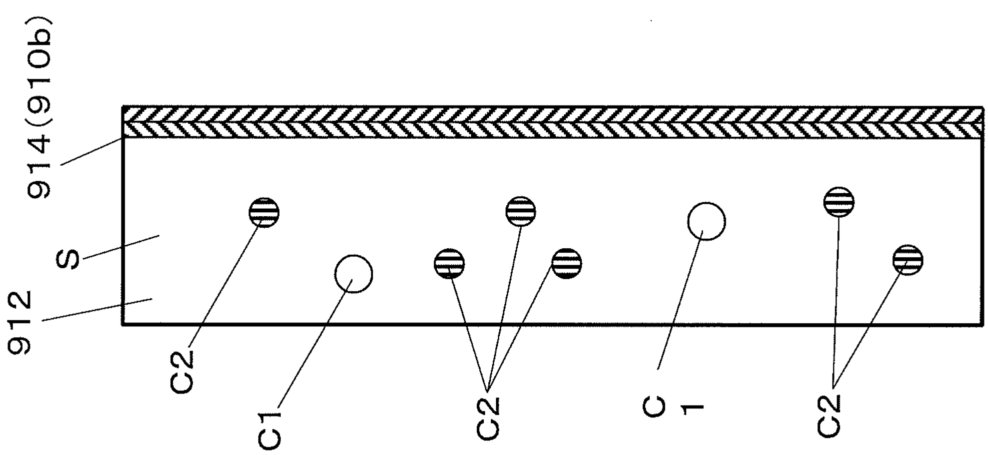


AFTER LIQUID TRANSFER

FIG. 56A

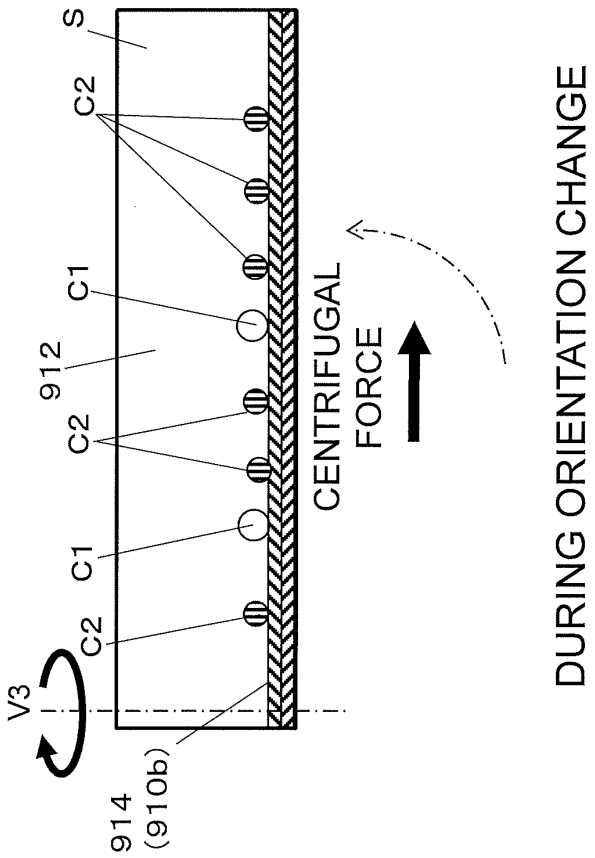


FIG. 57A

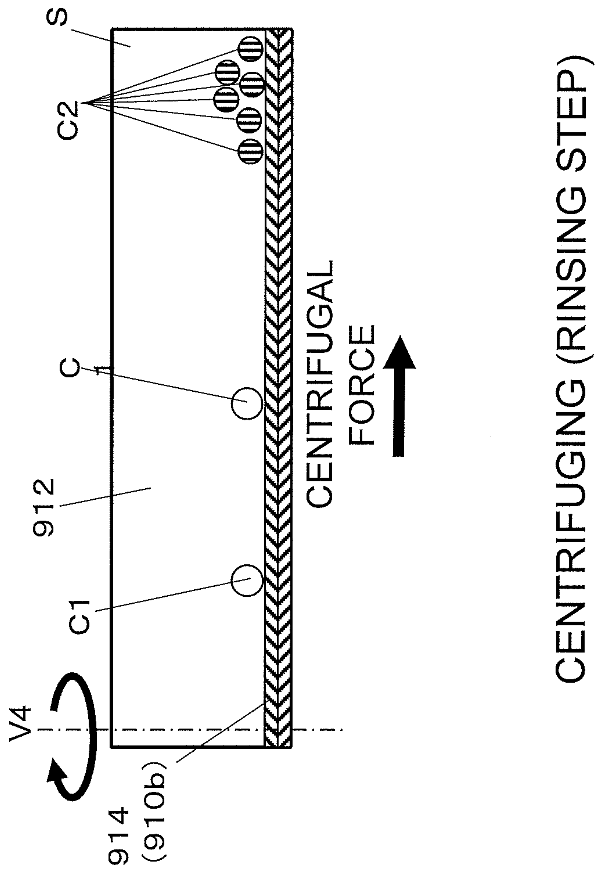


FIG. 57B

FIG. 58A

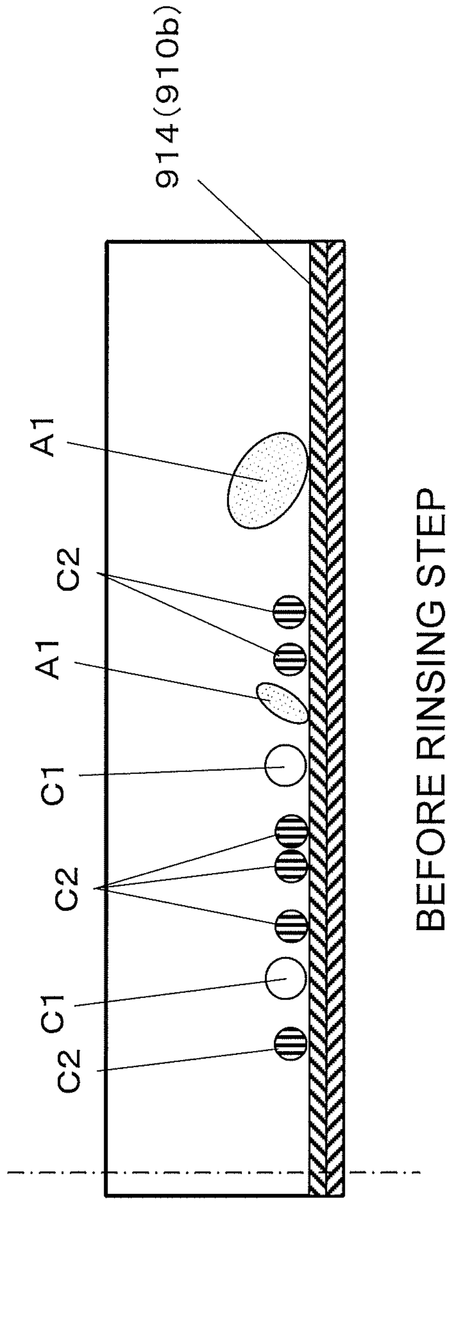


FIG. 58B

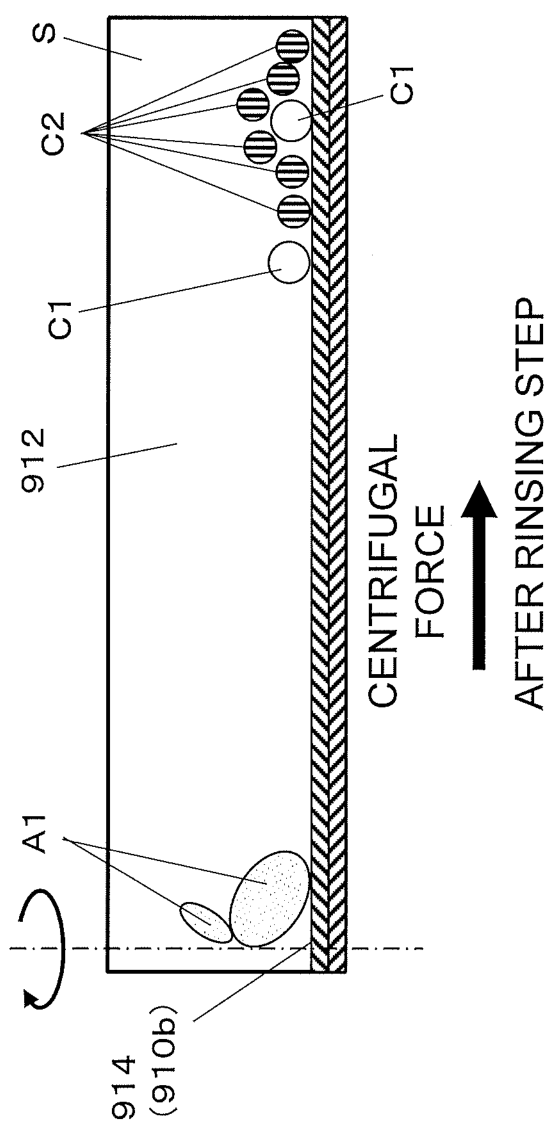

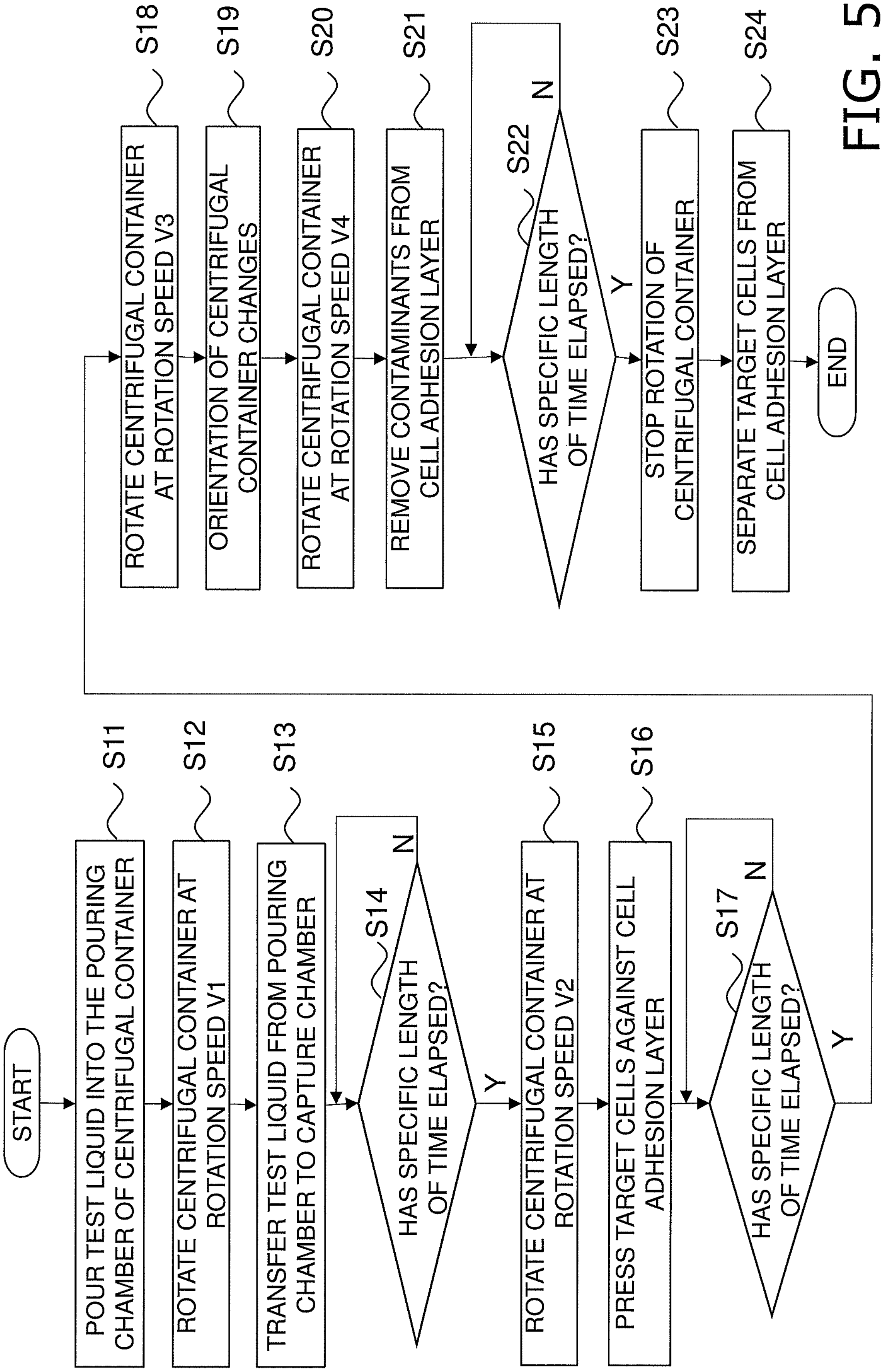
START
POUR TEST LIQUID INTO THE POURING CHAMBER OF CENTRIFUGAL CONTAINER
S11
ROTATE CENTRIFUGAL CONTAINER AT ROTATION SPEED V1
S12
TRANSFER TEST LIQUID FROM POURING CHAMBER TO CAPTURE CHAMBER
S13
HAS SPECIFIC LENGTH OF TIME ELAPSED?
S14
N
Y
ROTATE CENTRIFUGAL CONTAINER AT ROTATION SPEED V2
S15
PRESS TARGET CELLS AGAINST CELL ADHESION LAYER
S16
HAS SPECIFIC LENGTH OF TIME ELAPSED?
S17
N
Y
ROTATE CENTRIFUGAL CONTAINER AT ROTATION SPEED V3
S18
ORIENTATION OF CENTRIFUGAL CONTAINER CHANGES
S19
ROTATE CENTRIFUGAL CONTAINER AT ROTATION SPEED V4
S20
REMOVE CONTAMINANTS FROM CELL ADHESION LAYER
S21
HAS SPECIFIC LENGTH OF TIME ELAPSED?
S22
N
Y
STOP ROTATION OF CENTRIFUGAL CONTAINER
S23
SEPARATE TARGET CELLS FROM CELL ADHESION LAYER
S24
END

FIG. 59

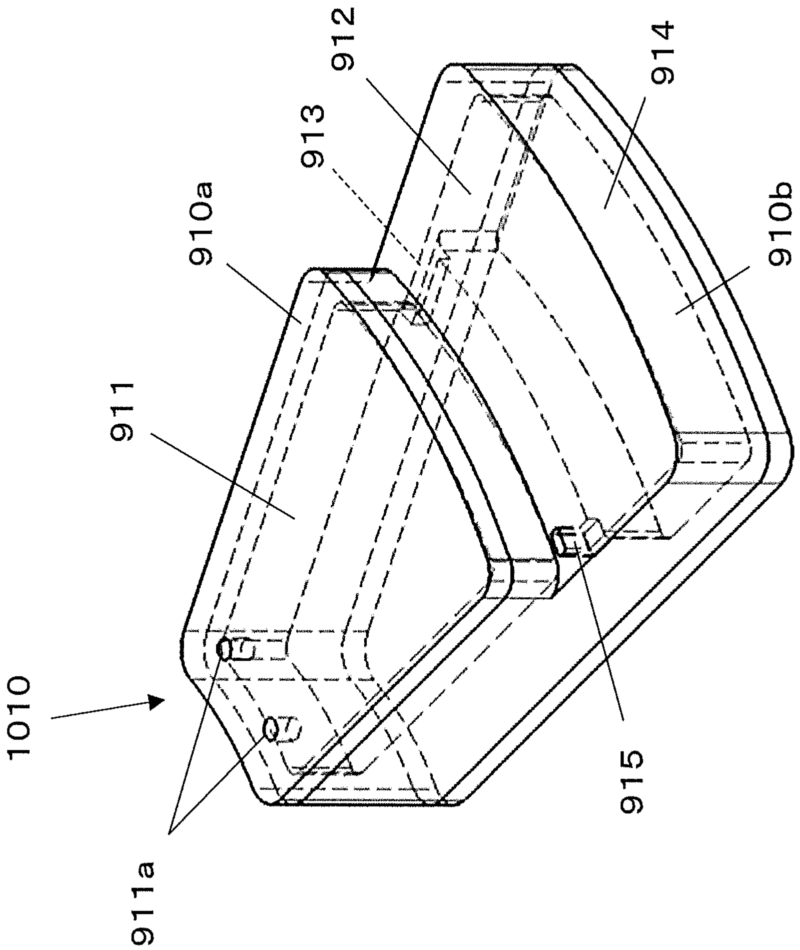


FIG. 60

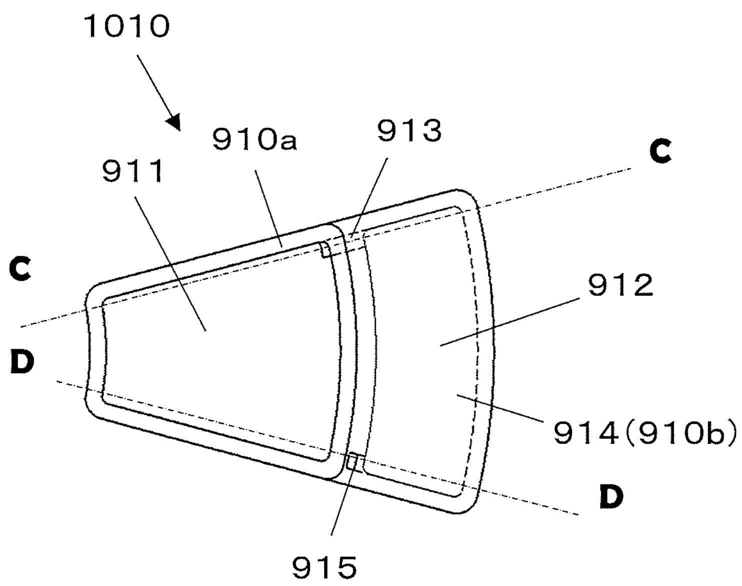


FIG. 61A

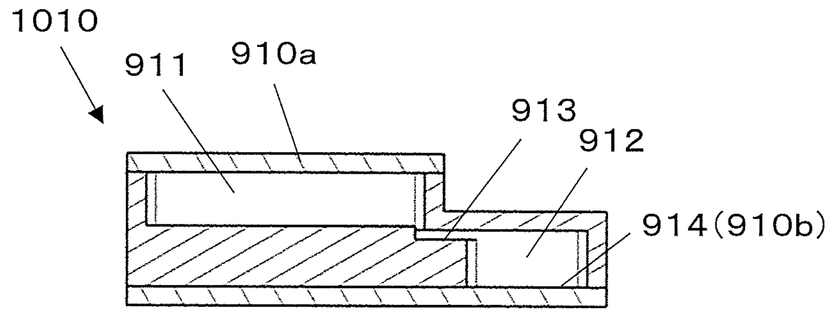


C–C CROSS-SECTIONAL VIEW

FIG. 61B

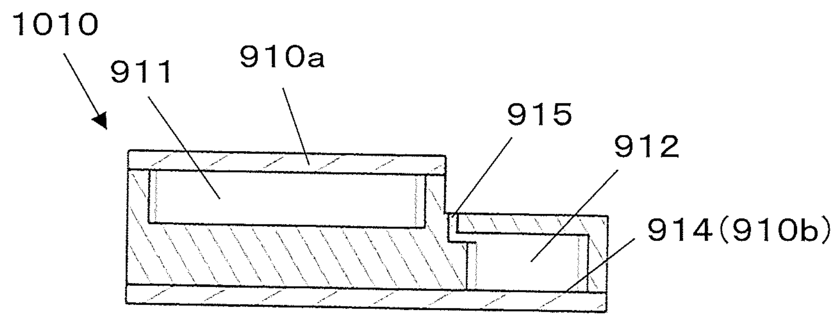


D–D CROSS-SECTIONAL VIEW

FIG. 61C

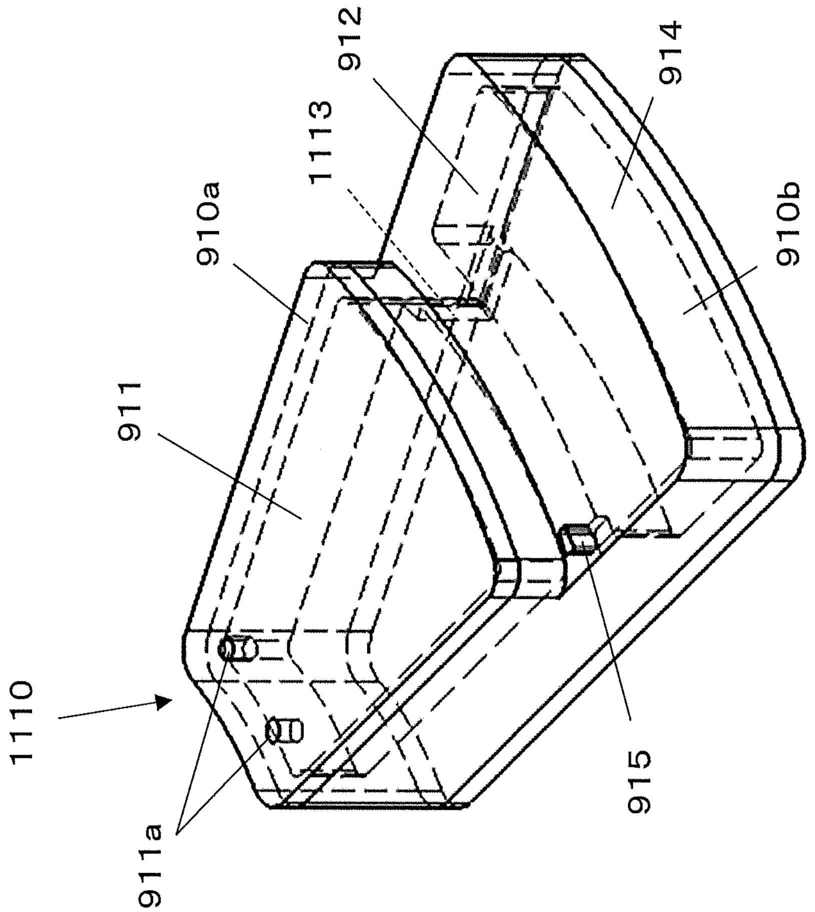


FIG. 62

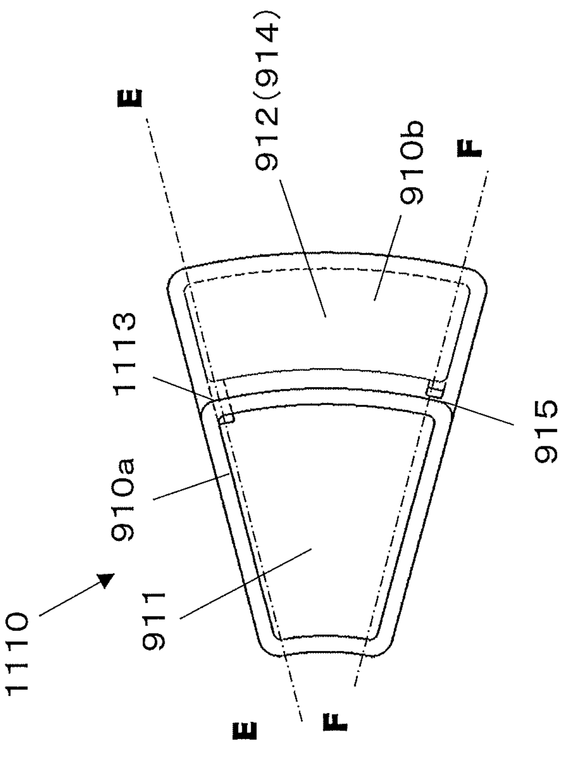


FIG. 63A

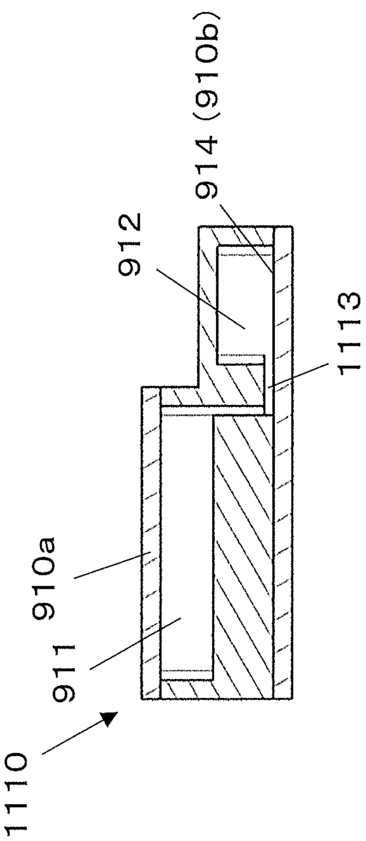


E–E CROSS-SECTIONAL VIEW

FIG. 63B

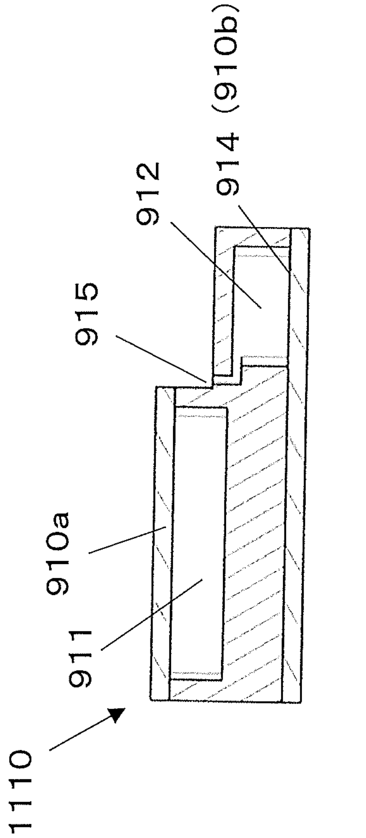


F–F CROSS-SECTIONAL VIEW

FIG. 63C

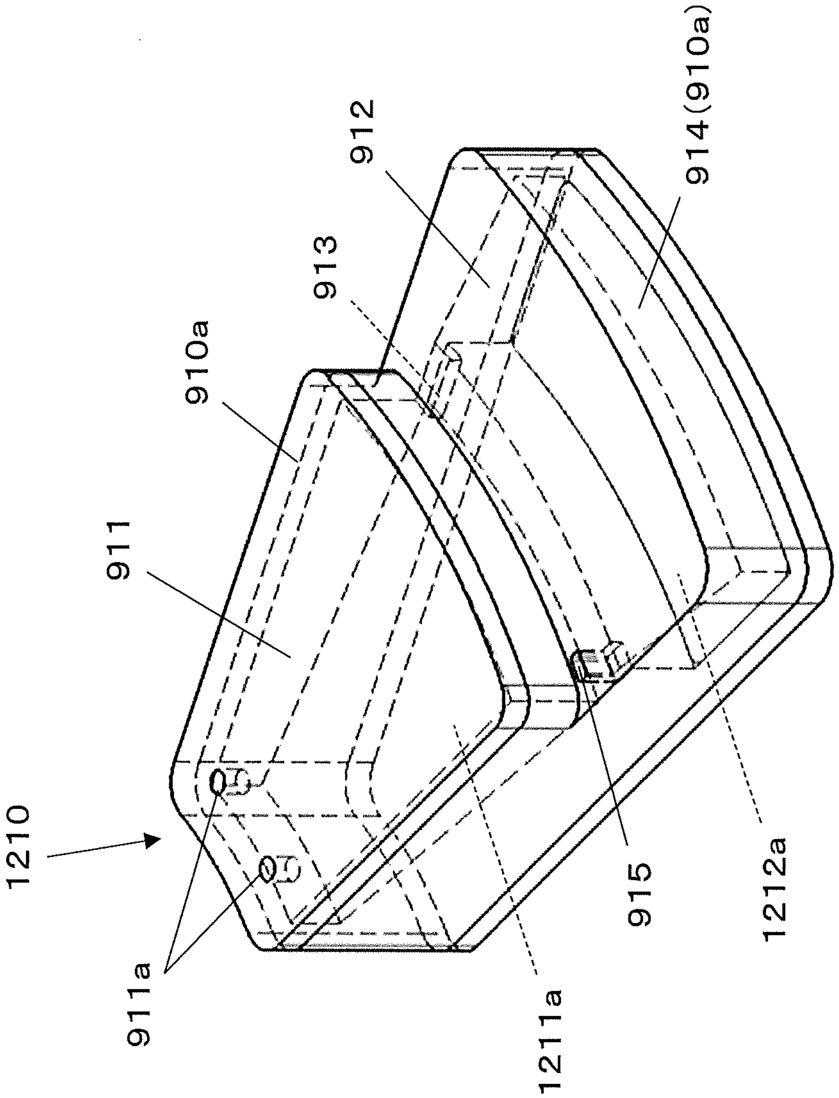


FIG. 64

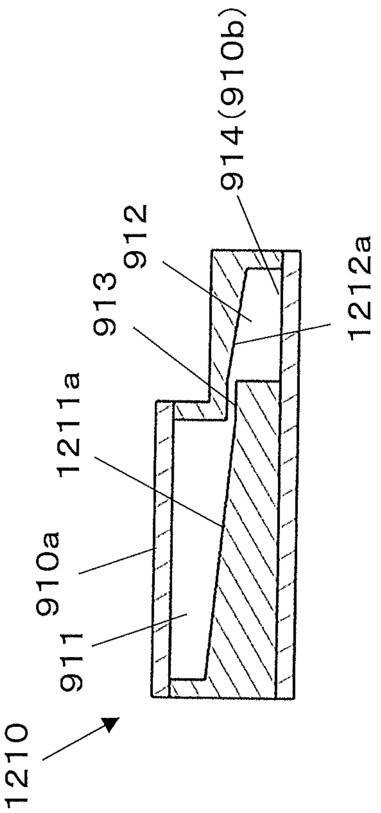


G–G CROSS-SECTIONAL VIEW

FIG. 65B

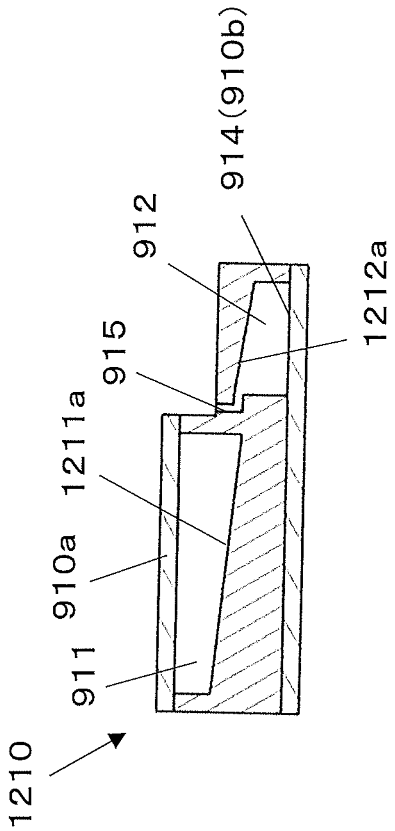


H–H CROSS-SECTIONAL VIEW

FIG. 65C

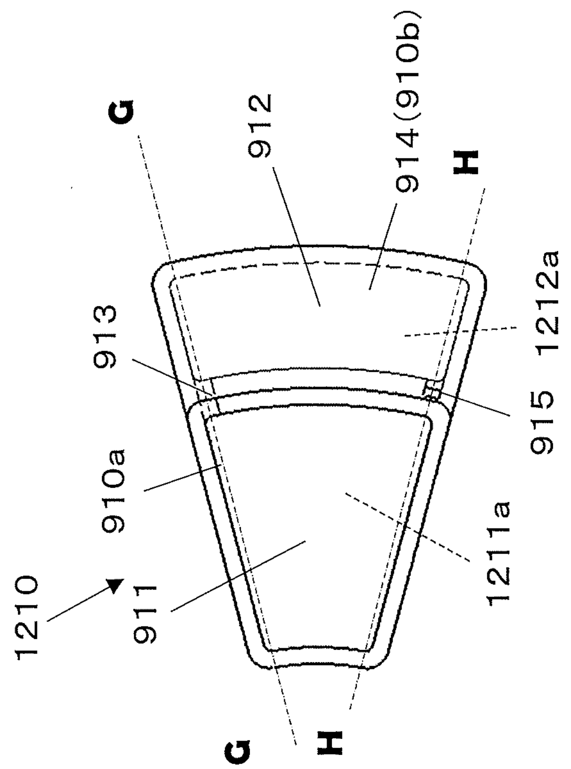


FIG. 65A

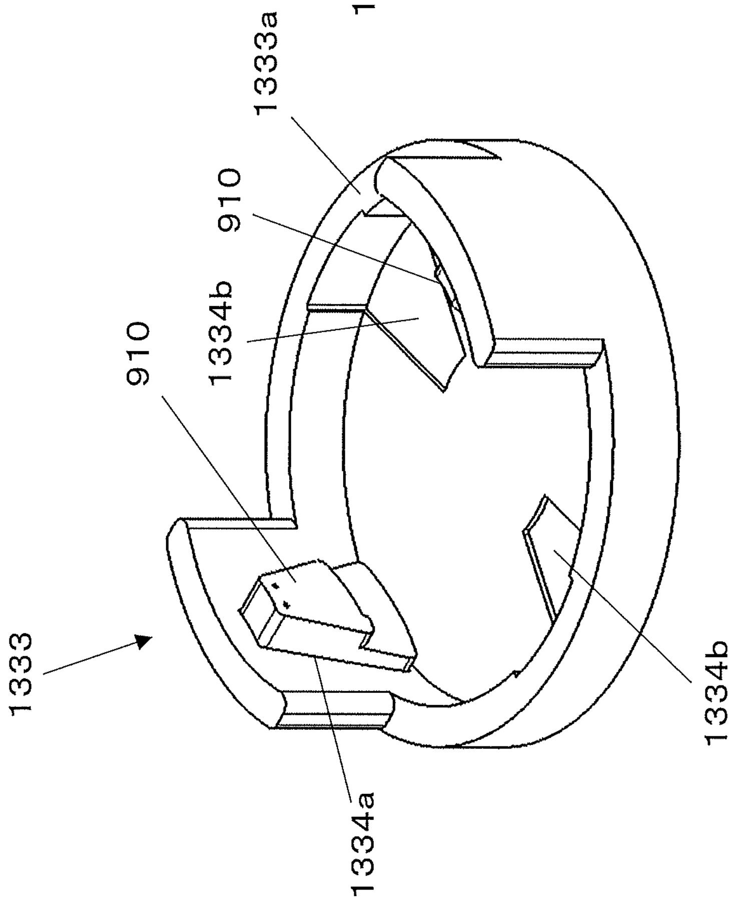


CELL CAPTURE STEP

FIG. 66A

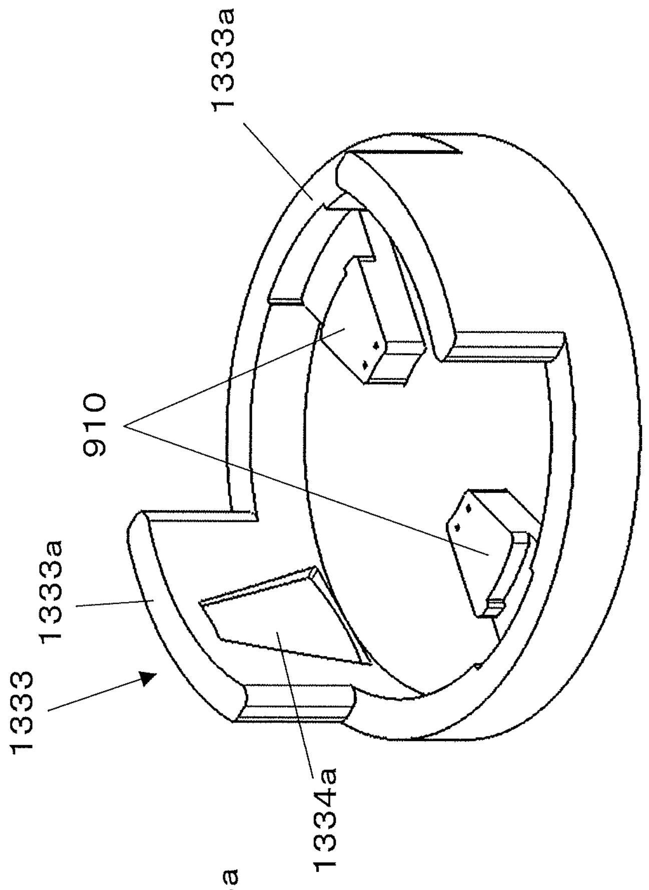


RINSING STEP (CONTAMINANT REMOVAL)

FIG. 66B

**INTERNATIONAL SEARCH REPORT**

International application No.
**PCT/JP2022/025814**

**A. CLASSIFICATION OF SUBJECT MATTER**

***C12M 1/00***(2006.01)i; ***C12M 1/10***(2006.01)i; ***C12M 1/26***(2006.01)i; ***C12N 1/02***(2006.01)i; ***C12N 11/02***(2006.01)i
FI: C12M1/10; C12M1/00 A; C12N1/02; C12N11/02; C12M1/26

According to International Patent Classification (IPC) or to both national classification and IPC

**B. FIELDS SEARCHED**

Minimum documentation searched (classification system followed by classification symbols)

C12M1/00; C12M1/10; C12M1/26; C12N1/02; C12N11/02

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | JP 4-152952 A (TERUMO CORP) 26 May 1992 (1992-05-26) entire text | 1-60 |
| A | WO 2004/087228 A1 (JAPAN SCIENCE AND TECHNOLOGY AGENCY) 14 October 2004 (2004-10-14) entire text, all drawings | 1-60 |
| A | JP 2012-105579 A (YAMAGATA UNIV) 07 June 2012 (2012-06-07) entire text, all drawings | 1-60 |
| A | JP 2017-102038 A (YAMAGATA UNIV) 08 June 2017 (2017-06-08) entire text, all drawings | 1-60 |
| A | JP 2020-118661 A (SUMITOMO RUBBER IND) 06 August 2020 (2020-08-06) entire text, all drawings | 1-60 |
| A | WO 2020/203965 A1 (KYUSYU UNIVERISTY, NATIONAL UNIVERSITY CORPORATION) 08 October 2020 (2020-10-08) entire text, all drawings | 1-60 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 August 2022** | **30 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/025814

C. DOCUMENTS CONSIDERED TO BE RELEVANT

| Category* | Citation of document, with indication, where appropriate, of the relevant passages | Relevant to claim No. |
|---|---|---|
| A | WO 2021/095862 A1 (KYUSYU UNIVERISTY, NATIONAL UNIVERSITY CORPORATION) 20 May 2021 (2021-05-20) entire text, all drawings | 1-60 |
| A | CHEN, C. C. et al. Centrifugal Filter Device for Detection of Rare Cells With Immuno-Binding. IEEE TRANSACTIONS ON NANOBIOSCIENCE. 06 October 2015, vol. 14, no. 8, pp. 864-869, doi: 10.1109/TNB.2015.2485298 entire text, all drawings | 1-60 |

Form PCT/ISA/210 (second sheet) (January 2015)

**INTERNATIONAL SEARCH REPORT**
**Information on patent family members**

International application No.
**PCT/JP2022/025814**

| Patent document cited in search report | Publication date (day/month/year) | Patent family member(s) | Publication date (day/month/year) |
|---|---|---|---|
| JP 4-152952 A | 26 May 1992 | (Family: none) | |
| WO 2004/087228 A1 | 14 October 2004 | (Family: none) | |
| JP 2012-105579 A | 07 June 2012 | (Family: none) | |
| JP 2017-102038 A | 08 June 2017 | (Family: none) | |
| JP 2020-118661 A | 06 August 2020 | US 2020/0238201 A1<br>entire text, all drawings<br>EP 3686598 A1 | |
| WO 2020/203965 A1 | 08 October 2020 | US 2022/0154165 A1<br>entire text, all drawings<br>EP 3929221 A1 | |
| WO 2021/095862 A1 | 20 May 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6474540 B